# EUROPEAN PATENT APPLICATION

(11) **EP 4 755 393 A1**
(43) Date of publication of application: **10.06.2026**
(21) Application number: 24848394.3
(22) Date of filing: 02.08.2024
(51) Int. Cl.: A61K 38/17, A61K 45/00, A61K 48/00, A61P 25/28, C12N 15/11

(54) **USE OF PROTON PUMP REGULATOR IN PREPARATION OF DRUG**

(30) Priority: 03.08.2023 CN 202310969320
(71) Applicant: Shanghai Quietd Biotechnology Co., Ltd., Shanghai 201111 (CN)
(72) Inventor: LI, Kaicheng, Shanghai 201300 (CN); LI, Zhen, Shanghai 201300 (CN); YOU, Pu, Shanghai 201300 (CN)
(74) Representative: Murgitroyd & Company
(86) International application number: PCT/CN2024/109596
(87) International publication number: WO 2025/026441

(57) **Abstract**

Use of a proton pump regulator in the preparation of a reagent for preventing and/or treating diseases, wherein the proton pump regulator regulates ATP6V1B2 and/or a functionally active fragment thereof, and the diseases comprise a stroke.

## Description

### TECHNICAL FIELD

The present application relates to the field of biomedicine, specifically to use of a proton pump regulator in the preparation of a drug for preventing and/or treating stroke.

### BACKGROUND ART

ATP6V1B2 protein encoded by ATP6V1B2 gene is an important structural protein for ATP hydrolysis-driven proton pumps. It is widely distributed in human tissues, but is more abundant in brain tissues, kidneys, osteoclasts and other parts. It plays an important role in synaptic transmission and lysosomal acidification. Mutations in this gene may lead to DOORS (Deafness, Onychodystrophy, Osteodystrophy, Impaired Intellectual Development, and Seizures) syndrome (susceptible autosomal dominant congenital deafness-onychodystrophy syndrome). Decreased expression of the ATP6V1B2 protein can be associated with the pathogenesis of AD.

Stroke, also known as "apoplexy," is characterized by high incidence, high disability rate, high recurrence rate, and high mortality rate. It is one of the most important fatal diseases in the world and the leading cause of disability and death in China. Stroke is mainly divided into two categories: ischemic stroke and hemorrhagic stroke, with about 80% being ischemic stroke.

Therefore, finding effective drugs to treat stroke is an urgent problem to be solved.

### SUMMARY OF THE INVENTION

The present application provides use of a proton pump regulator in the preparation of a reagent for preventing and/or treating diseases, wherein the proton pump regulator regulates ATP6V1B2 and/or a functionally active fragment thereof, and the diseases comprise a stroke.

In another aspect, the present application provides use of a DIR regulator in the preparation of a reagent for preventing and/or treating diseases, wherein the DIR regulator regulates the intron-retained splicing product DIR of DNA damage-inducible transcript 4-like transcripts and/or functional fragments thereof, and the diseases comprise a stroke.

In some embodiments, the stroke includes ischemic stroke and/or hemorrhagic stroke.

In some embodiments, the ischemic stroke includes cerebral infarction.

In some embodiments, the cerebral infarction includes lacunar infarction, ischemic cerebral infarction and/or hemorrhagic infarction.

In some embodiments, the ischemic stroke is caused by factors including: thrombus, embolism and/or hypotension.

In some embodiments, the thrombus is caused by factors including: atherosclerosis, aneurysm, vascular malformation, arteritis and/or vasospasm.

In some embodiments, the ischemic stroke includes large artery atherosclerosis stroke, cardiogenic embolic stroke, small artery occlusion stroke, stroke of other determined etiology and/or stroke of undetermined etiology.

In some embodiments, the hemorrhagic stroke includes brain parenchymal hemorrhage, intraventricular hemorrhage and/or subarachnoid hemorrhage.

In some embodiments, the hemorrhagic stroke includes primary cerebral hemorrhage and/or secondary cerebral hemorrhage.

In some embodiments, the hemorrhagic stroke includes aneurysmal subarachnoid hemorrhage.

In some embodiments, the hemorrhagic stroke is caused by factors including: vascular malformation, aneurysm, blood disease, cerebral amyloid angiopathy, moyamoya disease, cerebral arteritis, anticoagulation or thrombolytic therapy and/or tumor stroke.

In some embodiments, the stroke includes stroke-induced impairments.

In some embodiments, the impairment includes impairments observed through imaging.

In some embodiments, the impairment observed through imaging includes intracerebral hematoma, intraventricular hemorrhage and/or subarachnoid hemorrhage.

In some embodiments, the impairment observed through imaging includes edema, hematoma and/or mass effect.

In some embodiments, the impairment observed through imaging includes dense artery sign, insular ribbon sign, blurred or decreased density of the basal ganglia, occlusion of small penetrating cerebral arteries, edema and/or mass effect.

In some embodiments, the impairment includes impairment of learning ability, and the learning ability comprises cognitive ability, motor ability, memory ability, and/or spatial exploration ability.

In some embodiments, the learning ability is measured by implementing a test selected from the group consisting of: novel object recognition test and water maze test.

In some embodiments, the novel object recognition test is used for evaluating cognitive ability, motor ability and/or spatial exploration ability.

In some embodiments, the water maze test is used for evaluating memory ability, motor ability and/or spatial exploration ability.

In some embodiments, the ATP6V1B2 and/or functionally active fragment thereof is each derived from mammals.

In some embodiments, the ATP6V1B2 and/or functionally active fragment thereof is each derived from humans or mice.

In some embodiments, the ATP6V1B2 comprises the amino acid sequence set forth in SEQ ID NO: 8 or 16.

In some embodiments, the functionally active fragment of the ATP6V1B2 is capable of specifically binding to the amino acid sequence set forth in SEQ ID NO: 5.

In some embodiments, the functionally active fragment of the ATP6V1B2 is capable of specifically binding to the amino acid sequence set forth in SEQ ID NO: 1.

In some embodiments, the functionally active fragment of the ATP6V1B2 comprises at least a portion of the amino acid sequence from position 288 to position 512 of human ATP6V1B2 protein.

In some embodiments, the functionally active fragment of the ATP6V1B2 comprises at least a portion of the amino acid sequence from position 288 to position 512 of mouse ATP6V1B2 protein.

In some embodiments, the functionally active fragment of the ATP6V1B2 comprises the amino acid sequence set forth in any one of SEQ ID NOs: 10-11.

In some embodiments, the ATP6V1B2 comprises the amino acid sequence encoded by the nucleotide sequence set forth in SEQ ID NO: 9 or 17.

In some embodiments, the proton pump regulator is capable of regulating the activity and/or function of the proton pump.

In some embodiments, the proton pump regulator is capable of improving the expression level and/or activity of proton pump-associated proteins in a subject.

In some embodiments, the proton pump regulator is capable of regulating the expression level and/or activity of the ATP6V1B2.

In some embodiments, the proton pump regulator is capable of improving the expression level and/or activity of the ATP6V1B2 in a subject, and the improvement comprises, compared to the original expression level and/or activity of the ATP6V1B2 in the subject, improving the expression level and/or activity of the ATP6V1B2 by at least about 10%.

In some embodiments, the expression level of the ATP6V1B2 includes the expression level of the ATP6V1B2 gene, the transcription level of ATP6V1B2 gene and/or the expression level of the ATP6V1B2 protein.

In some embodiments, the proton pump regulator is capable of increasing the release of neurotransmitters at neuronal synapses, and the increase includes an increase of at least about 10% compared to the original level of neurotransmitter release at neuronal synapses in the subject.

In some embodiments, the proton pump regulator increases the release frequency of excitatory postsynaptic currents, and the increase includes an increase of at least about 10% compared to the original level of release frequency of excitatory postsynaptic currents in the subject.

In some embodiments, the DIR regulator causes a reduction of the expression level and/or biological activity of the DIR and/or functional fragment thereof in the subject.

In some embodiments, the reduction comprises a decrease of at least about 10% in the expression level and/or biological activity of the DIR and/or functional fragment thereof compared to the original expression level and/or biological activity of the DIR and/or functional fragment thereof in the subject.

In some embodiments, the expression level includes the expression level of a gene encoding the DIR/or functional fragment thereof, the transcription level of the gene encoding the DIR/or functional fragment thereof, and/or the expression level of the DIR/or functional fragment thereof.

In some embodiments, the functional fragment of the DIR retains at least a portion of the biological activity of the DIR.

In some embodiments, the biological activity includes influencing the excitability of neurons and/or inhibiting the activity of neurons.

In some embodiments, the biological activity includes the ability to reduce the frequency of excitatory postsynaptic currents (EPSCs), and/or the ability to reduce the amplitude of EPSCs.

In some embodiments, the reduction comprises, by administering the DIR and/or functional fragment thereof and/or a nucleic acid encoding the DIR and/or functional fragment thereof, reducing the frequency of excitatory postsynaptic currents (EPSCs) in the subject, and/or reducing the amplitude of EPSCs in the subject, compared to the original biological activity of the DIR and/or functional fragment thereof in the subject.

In some embodiments, the biological activity includes influencing cognitive abilities.

In some embodiments, the biological activity includes participation in signaling pathways associated with Aβ deposition, and/or participation in signaling pathways associated with Tau tangle formation.

In some embodiments, the biological activity includes inducing Aβ deposition and/or amyloid plaque formation through gelsolin.

In some embodiments, the DIR/or functional fragment thereof induces Aβ deposition and/or amyloid plaque formation by binding to gelsolin.

In some embodiments, the expression levels of the DIR and/or functional fragment thereof are positively correlated with the expression levels of Aβ.

In some embodiments, the reduction comprises, by administering the DIR and/or functional fragment thereof and/or a nucleic acid encoding the DIR and/or functional fragment thereof, reducing the cognitive ability of the subject, compared to the original biological activity of the DIR and/or functional fragment thereof in the subject.

In some embodiments, the DIR/or functional fragment thereof is derived from mammals.

In some embodiments, the DIR/or functional fragment thereof is derived from primates.

In some embodiments, the DIR/or functional fragment thereof is derived from humans.

In some embodiments, the DIR comprises the amino acid sequence set forth in SEQ ID NO: 56.

In some embodiments, the functional fragment of the DIR comprises an amino acid sequence encoded by the retained intron in DDIT4L.

In some embodiments, the functionally active fragment of the DIR comprises the amino acid sequence set forth in any one of SEQ ID NOs: 59-60.

In some embodiments, the proton pump regulator and/or the DIR regulator include proteins and/or polypeptides.

In some embodiments, the proton pump regulator and/or the DIR regulator comprise the amino acid sequence set forth in SEQ ID NO: 33 and/or variants thereof, where X represents any amino acid.

In some embodiments, the proton pump regulator and/or the DIR regulator comprise the amino acid sequence set forth in any one of SEQ ID NOs: 30-32 and/or variants thereof, where X represents any amino acid.

In some embodiments, the proton pump regulator and/or the DIR regulator comprise the amino acid sequence set forth in any one of SEQ ID NOs: 27-29 and/or variants thereof, where X represents any amino acid.

In some embodiments, the proton pump regulator and/or the DIR regulator comprise the amino acid sequences set forth in SEQ ID NOs: 19-26 and/or variants thereof.

In some embodiments, the proton pump regulator and/or the DIR regulator include a multimer.

In some embodiments, the multimer includes a homodimer.

In some embodiments, the cysteine in the amino acid sequence of the proton pump regulator and/or the DIR regulator is not subject to modification for sulfhydryl blocking.

In some embodiments, the serine in the amino acid sequence of the proton pump regulator and/or the DIR regulator is not subject to modification for phosphorylation.

In some embodiments, the proton pump regulator and/or the DIR regulator include fusion proteins and/or fusion polypeptides.

In some embodiments, the fusion protein and/or fusion polypeptide comprise molecules capable of being transported across the blood-brain barrier to the brain.

In some embodiments, the molecule capable of being transported across the blood-brain barrier to the brain comprises a polypeptide.

In some embodiments, the molecule capable of being transported across the blood-brain barrier to the brain comprises a cell-penetrating peptide.

In some embodiments, the cell-penetrating peptide comprises an amino acid sequence as set forth in SEQ ID NO: 34.

In some embodiments, the proton pump regulator and/or the DIR regulator comprise the amino acid sequence set forth in SEQ ID NO: 48, where X represents any amino acid.

In some embodiments, the proton pump regulator and/or the DIR regulator comprise the amino acid sequence set forth in any one of SEQ ID NOs: 45-47, where X represents any amino acid.

In some embodiments, the proton pump regulator and/or the DIR regulator comprise the amino acid sequence set forth in any one of SEQ ID NOs: 42-44 and/or variants thereof, where X represents any amino acid.

In some embodiments, the proton pump regulator and/or the DIR regulator comprise the amino acid sequence set forth in any one of SEQ ID NOs: 35-41 and/or variants thereof.

In some embodiments, the subject includes mammals.

In some embodiments, the subject includes humans.

In some embodiments, the reagent is formulated to be suitable for oral administration and/or injection administration.

In another aspect, the present application provides use of a proton pump regulator in the preparation of a reagent for preventing and/or treating diseases, wherein the proton pump regulator regulates ATP6V1B2 and/or a functionally active fragment thereof, and the disease includes diseases associated with the intron-retained splicing product DIR of DNA damage-inducible transcript 4-like transcripts and/or a functional fragment thereof.

In some embodiments, the ATP6V1B2 and/or functionally active fragment thereof is each from mammals.

In some embodiments, the ATP6V1B2 and/or functionally active fragment thereof is each from humans or mice.

In some embodiments, the ATP6V1B2 comprises the amino acid sequence set forth in SEQ ID NO: 8 or 16.

In some embodiments, the functionally active fragment of the ATP6V1B2 is capable of specifically binding to the amino acid sequence set forth in SEQ ID NO: 5.

In some embodiments, the functionally active fragment of the ATP6V1B2 is capable of specifically binding to the amino acid sequence set forth in SEQ ID NO: 1.

In some embodiments, the functionally active fragment of the ATP6V1B2 comprises at least a portion of the amino acid sequence from position 288 to position 512 of human ATP6V1B2 protein.

In some embodiments, the functionally active fragment of the ATP6V1B2 comprises at least a portion of the amino acid sequence from position 288 to position 512 of mouse ATP6V1B2 protein.

In some embodiments, the functionally active fragment of the ATP6V1B2 comprises the amino acid sequence set forth in any one of SEQ ID NOs: 10-11.

In some embodiments, the ATP6V1B2 comprises the amino acid sequence encoded by the nucleotide sequence set forth in SEQ ID NO: 9 or 17.

In some embodiments, the proton pump regulator is capable of regulating the activity and/or function of the proton pump.

In some embodiments, the proton pump regulator is capable of improving the expression level and/or activity of proton pump-associated proteins in a subject.

In some embodiments, the proton pump regulator is capable of regulating the expression level and/or activity of the ATP6V1B2.

In some embodiments, the proton pump regulator is capable of improving the expression level and/or activity of the ATP6V1B2 in a subject, and the improvement comprises, compared to the original expression level and/or activity of the ATP6V1B2 in the subject, improving the expression level and/or activity of the ATP6V1B2 by at least about 10%.

In some embodiments, the expression level of the ATP6V1B2 includes the expression level of the ATP6V1B2 gene, the transcription level of ATP6V1B2 gene and/or the expression level of the ATP6V1B2 protein.

In some embodiments, the proton pump regulator is capable of increasing the release of neurotransmitters at neuronal synapses, and the increase includes an increase of at least about 10% compared to the original level of neurotransmitter release at neuronal synapses in the subject.

In some embodiments, the proton pump regulator increases the release frequency of excitatory postsynaptic currents, and the increase includes an increase of at least about 10% compared to the original level of release frequency of excitatory postsynaptic currents in the subject.

In some embodiments, the proton pump regulator causes a reduction of the expression level and/or biological activity of the DIR and/or functional fragment thereof in the subject.

In some embodiments, the reduction comprises a decrease of at least about 10% in the expression level and/or biological activity of the DIR and/or functional fragment thereof compared to the original expression level and/or biological activity of the DIR and/or functional fragment thereof in the subject.

In some embodiments, the expression level includes the expression level of a gene encoding the DIR/or functional fragment thereof, the transcription level of the gene encoding the DIR/or functional fragment thereof, and/or the expression level of the DIR/or functional fragment thereof.

In some embodiments, the functional fragment of the DIR retains at least a portion of the biological activity of the DIR.

In some embodiments, the biological activity includes influencing the excitability of neurons and/or inhibiting the activity of neurons.

In some embodiments, the biological activity includes the ability to reduce the frequency of excitatory postsynaptic currents (EPSCs), and/or the ability to reduce the amplitude of EPSCs.

In some embodiments, the reduction comprises, by administering the DIR and/or functional fragment thereof and/or a nucleic acid encoding the DIR and/or functional fragment thereof, reducing the frequency of excitatory postsynaptic currents (EPSCs) in the subject, and/or reducing the amplitude of EPSCs in the subject, compared to the original biological activity of the DIR and/or functional fragment thereof in the subject.

In some embodiments, the biological activity includes influencing cognitive abilities.

In some embodiments, the biological activity includes participation in signaling pathways associated with Aβ deposition, and/or participation in signaling pathways associated with Tau tangle formation.

In some embodiments, the biological activity includes inducing Aβ deposition and/or amyloid plaque formation through gelsolin.

In some embodiments, the DIR/or functional fragment thereof induces Aβ deposition and/or amyloid plaque formation by binding to gelsolin.

In some embodiments, the expression levels of the DIR and/or functional fragment thereof are positively correlated with the expression levels of Aβ.

In some embodiments, the reduction comprises, by administering the DIR and/or functional fragment thereof and/or a nucleic acid encoding the DIR and/or functional fragment thereof, reducing the cognitive ability of the subject, compared to the original biological activity of the DIR and/or functional fragment thereof in the subject.

In some embodiments, the DIR/or functional fragment thereof is derived from mammals.

In some embodiments, the DIR/or functional fragment thereof is derived from primates.

In some embodiments, the DIR/or functional fragment thereof is derived from humans.

In some embodiments, the DIR comprises the amino acid sequence set forth in SEQ ID NO: 56.

In some embodiments, the functional fragment of the DIR comprises an amino acid sequence encoded by the retained intron in DDIT4L.

In some embodiments, the functionally active fragment of the DIR comprises the amino acid sequence set forth in any one of SEQ ID NOs: 59-60.

In some embodiments, the proton pump regulator includes proteins and/or polypeptides.

In some embodiments, the proton pump regulator comprises the amino acid sequence set forth in SEQ ID NO: 33 and/or variants thereof, where X represents any amino acid.

In some embodiments, the proton pump regulator comprises the amino acid sequence set forth in any one of SEQ ID NOs: 30-32 and/or variants thereof, where X represents any amino acid.

In some embodiments, the proton pump regulator comprises the amino acid sequence set forth in any one of SEQ ID NOs: 27-29 and/or variants thereof, where X represents any amino acid.

In some embodiments, the proton pump regulator comprises the amino acid sequences set forth in SEQ ID NOs: 19-26 and/or variants thereof.

In some embodiments, the proton pump regulator includes a multimer.

In some embodiments, the multimer includes a homodimer.

In some embodiments, the cysteine in the amino acid sequence of the proton pump regulator is not subject to modification for sulfhydryl blocking.

In some embodiments, the serine in the amino acid sequence of the proton pump regulator is not subject to modification for phosphorylation.

In some embodiments, the proton pump regulator includes fusion proteins and/or fusion polypeptides.

In some embodiments, the fusion protein and/or fusion polypeptide comprise molecules capable of being transported across the blood-brain barrier to the brain.

In some embodiments, the molecule capable of being transported across the blood-brain barrier to the brain comprises a polypeptide.

In some embodiments, the molecule capable of being transported across the blood-brain barrier to the brain comprises a cell-penetrating peptide.

In some embodiments, the cell-penetrating peptide comprises an amino acid sequence as set forth in SEQ ID NO: 34.

In some embodiments, the proton pump regulator comprises the amino acid sequence set forth in SEQ ID NO: 48, where X represents any amino acid.

In some embodiments, the proton pump regulator comprises the amino acid sequence set forth in any one of SEQ ID NOs: 45-47, where X represents any amino acid.

In some embodiments, the proton pump regulator comprises the amino acid sequence set forth in any one of SEQ ID NOs: 42-44 and/or variants thereof, where X represents any amino acid.

In some embodiments, the proton pump regulator comprises amino acid sequences set forth in any one of SEQ ID NOs: 35-41 and/or variants thereof.

In some embodiments, the subject includes mammals.

In some embodiments, the subject includes humans.

In some embodiments, the reagent is formulated to be suitable for oral administration and/or injection administration.

In some embodiments, the disease associated with the intron-retained splicing product DIR of DNA damage-inducible transcript 4-like transcripts and/or functional fragments thereof includes cognitive impairment, neurodegenerative diseases and/or stroke.

In some embodiments, the cognitive impairment includes cognitive impairment caused by normal aging, Lewy body dementia (LBD), frontotemporal dementia and/or vascular dementia.

In some embodiments, the inducing diseases of the cognitive impairment includes Alzheimer's disease, multi-infarct type, Parkinson's disease, AIDS and/or Creutzfeldt-Jakob disease (CJD).

In some embodiments, the cognitive impairment includes mild cognitive impairment (MCI), moderate-stage cognitive impairment and advanced-stage cognitive impairment.

In some embodiments, the cognitive impairment includes multi-domain amnestic MCI (aMCI-m).

In some embodiments, the neurodegenerative disease includes acute neurodegenerative disease and chronic neurodegenerative disease.

In some embodiments, the neurodegenerative disease includes neurodegenerative disease caused by neuronal death and glial cell homeostasis, neurodegenerative disease caused by aging, neurodegenerative disease caused by influence on the CNS cell function, neurodegenerative disease caused by abnormal communication between cells, and/or neurodegenerative disease caused by impaired cell motility.

In some embodiments, the neurodegenerative disease includes Alzheimer's disease, Parkinson's disease, multiple sclerosis (MS), amyotrophic lateral sclerosis (ALS), and/or Huntington's disease (HD).

In some embodiments, the neurodegenerative disease includes presenile dementia.

In some embodiments, the neurodegenerative disease includes the early stage, middle stage and/or late stage of presenile dementia.

In some embodiments, the stroke includes ischemic stroke and/or hemorrhagic stroke.

In some embodiments, the ischemic stroke includes cerebral infarction.

In some embodiments, the cerebral infarction includes lacunar infarction, ischemic cerebral infarction and/or hemorrhagic infarction.

In some embodiments, the ischemic stroke is caused by factors including: thrombus, embolism and/or hypotension.

In some embodiments, the thrombus is caused by factors including: atherosclerosis, aneurysm, vascular malformation, arteritis and/or vasospasm.

In some embodiments, the ischemic stroke includes large artery atherosclerosis stroke, cardiogenic embolic stroke, small artery occlusion stroke, stroke of other determined etiology and/or stroke of undetermined etiology.

In some embodiments, the hemorrhagic stroke includes brain parenchymal hemorrhage, intraventricular hemorrhage and/or subarachnoid hemorrhage.

In some embodiments, the hemorrhagic stroke includes primary cerebral hemorrhage and/or secondary cerebral hemorrhage.

In some embodiments, the hemorrhagic stroke includes aneurysmal subarachnoid hemorrhage.

In some embodiments, the hemorrhagic stroke is caused by factors including: vascular malformation, aneurysm, blood disease, cerebral amyloid angiopathy, moyamoya disease, cerebral arteritis, anticoagulation or thrombolytic therapy and/or tumor stroke.

In some embodiments, the stroke includes stroke-induced impairments.

In some embodiments, the impairment includes impairments observed through imaging.

In some embodiments, the impairment observed through imaging includes intracerebral hematoma, intraventricular hemorrhage and/or subarachnoid hemorrhage.

In some embodiments, the impairment observed through imaging includes edema, hematoma and/or mass effect.

In some embodiments, the impairment observed through imaging includes dense artery sign, insular ribbon sign, blurred or decreased density of the basal ganglia, occlusion of small penetrating cerebral arteries, edema and/or mass effect.

In some embodiments, the impairment includes impairment of learning ability, and the learning ability comprises cognitive ability, motor ability, memory ability, and/or spatial exploration ability.

In some embodiments, the learning ability is measured by implementing a test selected from the group consisting of: novel object recognition test and water maze test.

In some embodiments, the novel object recognition test is used for evaluating cognitive ability, motor ability and/or spatial exploration ability.

In some embodiments, the water maze test is used for evaluating memory ability, motor ability and/or spatial exploration ability.

Those skilled in the art may easily discern other aspects and advantages of the present application from the following detailed description. Only exemplary embodiments of the present application are shown and described in the following detailed description. As those skilled in the art will realize, the contents of the present application enable those skilled in the art to make modifications to the specific embodiments disclosed without departing from the spirit and scope of the invention to which the present application relates. Accordingly, the drawings and descriptions in the specification of the present application are illustrative only and not limited.

### BRIEF DESCRIPTION OF DRAWINGS

The specific features of the invention to which the present application relates are as shown in the appended claims. The features and advantages of the invention to which the present application relates can be better understood by reference to the exemplary embodiments and the drawings described in detail below. A brief description of the drawings is as follows:
FIG. 1 shows an exemplary gene editing strategy used to prepare the disease model of the present application.
FIGS. 2A-2D show the validation results of the gene editing effect.
FIGS. 3A-3B show the exemplary phenotypic characteristics of DIR-Ki mice.
FIGS. 4A-4C show the exemplary behavioral phenotypic characteristics of DIR-Ki mice.
FIGS. 5A-5C show the exemplary physiological phenotypic characteristics of DIR-Ki mice.
FIGS. 6A-6B show that the growth, metabolism, and motor function of DIR-Ki mice remain unchanged.
FIGS. 7A-7B show that the ATP6V1B2 regulator according to the present application can bind to the ATP6V1B2 protein.
FIGS. 8A-8K show the effects of the ATP6V1B2 regulator according to the present application on excitatory synaptic transmission.
FIG. 9 shows that the ATP6V1B2 regulator according to the present application, when administered intragastrically, can improve the cognitive behavior of AD mice in the novel object recognition test.
FIG. 10 shows that the ATP6V1B2 regulator according to the present application, when administered intragastrically, can improve the learning ability of AD mice in the water maze test.
FIG. 11 shows that the ATP6V1B2 regulator according to the present application, when administered intravenously, can improve the cognitive behavior of AD mice in the novel object recognition test.
FIG. 12 shows that the ATP6V1B2 regulator according to the present application, when administered intravenously, can improve the learning ability of AD mice in the water maze test.
FIG. 13 shows the increased DIR expression in the tissues and serum of MCAO model DIR-Ki mice.
FIG. 14 shows the formation of an immune co-precipitation between the ATP6V1B2 regulator according to the present application and the DIR protein.
FIG. 15 shows that the DIR regulator/ATP6V1B2 regulator according to the present application improves cognitive and memory abilities in DIR-Ki mice.
FIG. 16 shows the bar chart of neurobehavioral scores of each group of animals 24 h after MCAO modeling, indicating that the DIR regulator/ATP6V1B2 regulator according to the present application can reduce the neurobehavioral damage caused by MCAO modeling.
FIG. 17 shows the statistical graph of infarct volume of each group of animals 24 h after MCAO modeling, indicating that the DIR regulator/ATP6V1B2 regulator according to the present application can reduce the infarct volume caused by MCAO modeling.
FIG. 18 shows the results detected under a fluorescence microscope, indicating that the DIR regulator/ATP6V1B2 regulator according to the present application inhibits the increase in ROS caused by OGD.
FIG. 19 shows the statistical results of the inhibition of increased ROS caused by OGD by the DIR regulator/ATP6V1B2 regulator according to the present application.

### DETAILED DESCRIPTION

The embodiments of the invention of the present application will be illustrated below with specific examples. Those skilled in the art may easily understand other advantages and effects of the invention of the present application from the disclosure of the specification.

### DEFINITION OF TERMS

In the present application, the term "DDIT4L" generally refers to DNA damage-inducible transcript 4-like. It can also be called REDD2/RTP801L. Studies have found that DDIT4L can be associated with heart dysfunction. It can also be used to treat gliomas. The accession number of the human DDIT4L gene in GenBank is 115265; the accession number of the human DDIT4L protein in GenBank is NP_660287.1.

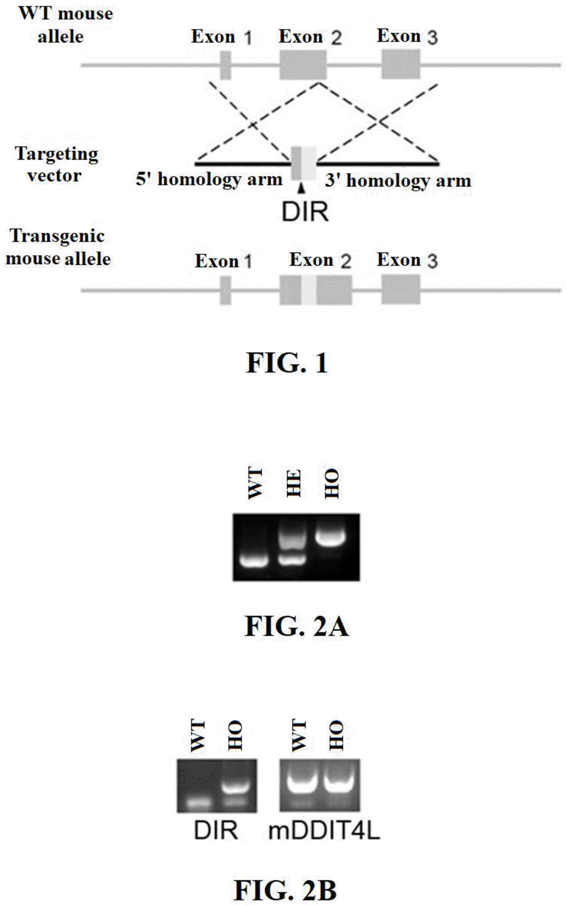

In the present application, the term "DIR" generally refers to the intron-retained splicing product of DDIT4L. The splicing reaction of DDIT4L can be seen in FIG. 1. In the present application, the amino acid sequence of the DIR can be as set forth in SEQ ID NO: 56.

In the present application, the term "ATP6V1B2" generally refers to ATPase H+ transporting V1 subunit B2 protein (or referred to as ATP6B2, DOOD, HO57, VATB, VPP3, Vma2 or ZLS2), and the gene encoding this protein. The ATP6V1B2 can be a multimeric enzyme that mediates intracellular organelle acidification in eukaryotic cells. The ATP6V1B2 may participate in processes such as protein sorting, zymogen activation, receptor-mediated endocytosis, and synaptic vesicle proton gradient generation. The ATP6V1B2 protein may include a cytoplasmic V1 domain and a transmembrane V0 domain. Human ATP6V1B2 has an accession number in GenBank of 526. Human ATP6V1B2 may have an accession number in UniProt of P21281.

In the present application, the term "proton pump-associated protein" generally refers to an associated protein that encodes and/or expresses a proton pump. The proton pump can be a protein that actively transports hydrogen ions on a biological membrane against the electrochemical potential difference of hydrogen ions on both sides of the membrane. The proton pump may include a Na-K pump, a Ca2+ pump, an H+-ATP pump and an H+ pyrophosphoric acid pump.

In the present application, the term "regulator" generally refers to a compound that alters the expression level and/or activity of a molecule. For example, regulators may include compounds that cause a certain activity intensity and/or expression level of a molecule to increase or decrease compared to the activity intensity and/or expression level without the regulator. For example, the regulator may include an inhibitor that reduces the intensity of one or more activities and/or expression level of the molecule.

In the present application, the term "binding agent" generally refers to a natural or non-natural molecule that specifically binds to a target or a portion of a target. Binding agents may include small molecule compounds, polymers and/or biomacromolecules. Binding agents can include proteins, peptides, nucleic acids, sugars, lipids, and small molecule compounds. For example, the binding agent may include a polypeptide. For example, the binding agent may include a fusion polypeptide.

In the present application, the term "expression level" generally refers to the protein, RNA or mRNA level of specific related genes. The expression level of the specific related gene (e.g., human DDIT4L gene) can be determined using any method known in the art. In the present application, the "expression" generally refers to a process of transforming gene-encoded information into a structure that is present in and manipulated in a cell. For example, it may include reverse transcription and amplification analysis (e.g., PCR, connected RT-PCR or quantitative RT-PCT), hybridization analysis, RNA Northern blotting, dot blotting, in-situ hybridization, gel electrophoresis, capillary electrophoresis, column chromatography, western blotting, immunohistochemistry, immunostaining or mass spectrometry. Analysis can be performed directly on biological samples or on proteins/nucleic acids isolated from samples.

In the present application, the term "activity" generally refers to any activity associated with a specific protein. In the present application, the activity may include, for example, any activity associated with the DIR protein. The activity may include protease-associated enzymatic activity. In some cases, the activity may include biological activity. In some cases, the activity may include binding of the protein to a receptor, for example, the binding may produce a measurable downstream effect. In the present application, the activity may include any activity that can be attributed to the protein by those skilled in the art.

In the present application, the term "variant" generally refers to a polypeptide including an amino acid sequence that differs from the amino acid sequence of a parent or reference polypeptide (e.g., wild-type polypeptide) by at least one amino acid residue. In the present application, the variant may have a higher (e.g., at least 80%) homology with the parent or reference polypeptide. The homology may include the similarity or identity of sequences. In the present application, the homology can be determined using standard techniques known in the art (see, for example, Smith and Waterman, Adv. Appl .Math. Advances in Applied Mathematics); and the percentage of identity common to polynucleotide or polypeptide sequences is determined by direct comparison of sequence information between molecules, the comparison being made by sequence alignment and identity determination using methods known in the art. Examples of algorithms suitable for determining sequence similarity are BLAST algorithms (see, Altschure et al., Jay Mohr. Bio. Journal of Molecular Biology, 215:403-410 [1990]). Software for BLAST analysis can be obtained by the National Center of Biotechnology Information (NCBI) in publication.

In the present application, the "variant" and/or "functional variant" can be a protein or polypeptide with substitution, deletion or addition of one or more amino acids in the amino acid sequence of the protein and/or polypeptide (e.g., a binding agent that binds to ATP6V1B2 or a fragment thereof). For example, the variant may include a protein or polypeptide that has amino acid change caused by substitution, deletion, and/or insertion of at least one, e.g., 1-30, 1-20, or 1-10, yet e.g., 1, 2, 3, 4, or 5 amino acids. The functional variant may substantially maintain the biological properties of the protein or polypeptide prior to change (e.g., substitution, deletion, or addition). For example, the functional variant may retain at least 60%, 70%, 80%, 90%, or 100% of the biological activity (e.g., the ability to bind to ATP6V1B2) of the protein or polypeptide prior to the alteration. For example, the substitution can be a conservative substitution. For example, the variant may also be a polypeptide encompassing its functionally active fragment, not limited to polypeptides containing the functionally active fragment of the protein resulting from processing and/or modification that occurs in the cell.

In the present application, the "variant" can refer to a homolog. The homolog can be a protein or polypeptide that shares at least about 85% (e.g., at least about 85%, about 90%, about 91%, about 92%, about 93%, about 94%, about 95%, about 96%, about 97%, about 98%, about 99% or higher) sequence homology with the amino acid sequence of the protein and/or the polypeptide (e.g., a binding agent that specifically binds to ATP6V1B2 or a fragment thereof).

In the present application, the homology generally refers to the similarity, similarity, or association between two or more sequences. "Percent sequence homology" can be calculated by comparing two sequences to be aligned in a comparison window to determine the number of positions with identical nucleic acid bases (e.g., A, T, C, G, U) or identical amino acid residues (e.g., Ala, Pro, Ser, Thr, Gly, Val, Leu, Ile, Phe, Tyr, Trp, Lys, Arg, His, Asp, Glu, Asn, Gln, Cys, and Met) in the two sequences to obtain the number of matching positions, dividing the number of matching positions by the total number of positions in the comparison window (i.e., window size), and multiplying the result by 100 to generate the percent sequence homology. Alignment for the purpose of determining percent sequence homology can be accomplished in a variety of ways known in the art, for example, using publicly available computer software such as BLAST, BLAST-2, ALIGN or Megalign (DNASTAR) software. Those skilled in the art can determine appropriate parameters for aligning sequences, including any algorithm required to achieve maximal alignment within the range of full-length sequences being compared or within the sequence region of interest. The homology can also be determined by the following methods: FASTA and BLAST. A description of the FASTA algorithm can be found in W. R. Pearson and D. J. Lipman, "Improved Tool for Biological Sequence Comparison", (Proc. Natl. Acad. Sci. USA), 85: 2444-2448, 1988; and D. J. Lipman and W. R. Pearson, "Fast and Sensitive Protein Similarity Search", Science, 227: 1435-1441, 1989. A description of the BLAST algorithm can be found in S. Altschul, W. Gish, W. Miller, E. W. Myers and D. Lipman, "A Basic Local Alignment Search Tool", Molecular Biology, 215: 403-410, 1990.

In the present application, the term "amino acid" generally refers to naturally occurring, synthetic, or non-natural amino acids, as well as amino acid analogs and amino acid mimetics that function in a similar manner to naturally occurring amino acids. Exemplary amino acids include naturally occurring amino acids; their analogs, derivatives, and congeners; amino acid analogs with variant side chains; and stereoisomers of any of the aforementioned substances. The 20 commonly used amino acids and their abbreviations used in the present application are used in the conventional way. See Immunology-A Synthesis (2nd edition, edited by E.S.Golub and D.R.Gren, Sinauer Associates, Sunderland, Mass. (1991)), the contents of which are incorporated herein by reference. The present application uses commonly used single-letter and three-letter abbreviations for amino acids (Bruce Alberts et al., Molecular Biology of the Cell, GarlandPublishing, Inc., New York (4th edition, 2002)).

In the present application, the term "conservative replacement" or "conservative substitution" is also called "conservative mutation," which generally refers to the substitution of amino acids in a protein by other amino acids that have similar properties (such as charge, side chain size, hydrophobicity/hydrophilicity, main chain conformation, and rigidity). Examples of amino acid groups with side chains possessing similar chemical properties include: 1) aliphatic side chains: glycine, alanine, valine, leucine, and isoleucine; 2) aliphatic-hydroxy side chains: serine and threonine; 3) amide-containing side chains: asparagine and glutamine; 4) aromatic side chains: phenylalanine, tyrosine, and tryptophan; 5) basic side chains: lysine, arginine, and histidine; 6) acidic side chains: aspartic acid and glutamic acid; and 7) sulfur-containing side chains: cysteine and methionine. Conservative amino acid substitution groups can be, for example, valine-leucine-isoleucine, phenylalanine-tyrosine, lysine-arginine, alanine-valine, glutamic acid-aspartic acid, and asparagine-glutamine.

In the present application, the term "non-conservative replacement or non-conservative substitution" is also referred to as "radical replacement or radical substitution", and it generally refers to the substitution of one amino acid with another amino acid in proteins that has different properties (such as charge, side chain size, hydrophobicity/hydrophilicity, main chain conformation, and rigidity). For example, non-conservative substitutions can also involve the use of non-natural amino acids.

In the present application, the term "polar amino acid" may include polar uncharged amino acids, basic (positively charged) amino acids, and acidic (negatively charged) amino acids. For example, polar amino acids can include threonine, serine, cysteine, tyrosine, asparagine, glutamine, lysine, arginine, histidine, aspartic acid, and glutamic acid.

In the present application, the term "non-polar amino acid" can include phenylalanine, proline, valine, leucine, isoleucine, methionine, tryptophan, alanine, and glycine.

In the present application, the term "fusion polypeptide" generally refers to a polypeptide comprising at least two discrete peptides or polypeptides (which do not exist together in a natural polypeptide in such a manner, i.e., these portions do not naturally exist in the same polypeptide or in the same order), or consisting of at least two discrete peptides or polypeptides (which do not exist together in a natural polypeptide in such a manner, i.e., these portions do not naturally exist in the same polypeptide or in the same order). The discrete peptides or polypeptides can be linked together directly or indirectly to form fusion polypeptides. For example, the discrete peptides or polypeptides can be linked together through peptide bonds to form fusion polypeptides. For example, the discrete peptides or polypeptides can be linked together through linkers to form fusion polypeptides. For example, the fusion polypeptide of the present application may comprise a fusion polypeptide formed by linking a polypeptide capable of binding to ATP6V 1B2 with a polypeptide having a specific function. In the present application, the term "fusion protein" generally refers to a protein composed of two or more polypeptides.

In the present application, the term "blood-brain barrier (BBB)" generally refers to the physiological barrier between the peripheral circulation and the brain and spinal cord, which is composed of the capillary endothelial cells, basement membrane and glial cell terminals of the brain, forming a tight barrier that restricts the transport of molecules, even very small molecules (such as urea (60 Daltons)), to the brain. The BBB in the brain, the blood-spinal cord barrier in the spinal cord, and the blood-retinal barrier in the retina are continuous capillary barriers within the CNS, and are collectively referred to as the blood-brain barrier or BBB herein. The BBB also covers the blood-CSF barrier (choroid plexus), which is composed of ependymal cells rather than capillary endothelial cells.

In the present application, the term "cell-penetrating peptide" generally refers to a class of short peptides that can cross cell membranes or tissue barriers. For example, cell-penetrating peptides can carry biomolecules such as proteins, RNA, and DNA into cells through mechanisms such as endocytosis and direct penetration to exert their effector functions.

In the present application, the term "multimer" generally refers to molecules having two or more polypeptide chains that are covalently, non-covalently, or simultaneously associated by covalent and non-covalent interactions. The multimer may include a dimer.

In the present application, the term "homodimer" generally refers to molecules formed from two identical monomers. The two identical monomers can be aggregated, complexed, or associated with each other by covalent and/or non-covalent interactions.

In the present application, the term "sulfhydryl blocking" generally refers to the process of blocking free sulfhydryl to impede the formation of intramolecular and/or intermolecular disulfide bonds. In the present application, the sulfhydryl blocking may occur on cysteine residues. The sulfhydryl blocking may make it impossible to form disulfide bonds between cysteine residues of a protein to prevent crosslinking or modification of the protein. The sulfhydryl blocking can be achieved by a blocking reagent, which can be a reducing reagent. The blocking reagent may include Dithiothreitol (DTT), β-mercaptoethanol (BME), and tris(2-carboxyethyl) phosphonate (TCEP•HCl).

In the present application, the term "serine phosphorylation" generally refers to phosphorylation modification that occurs on serine residues. The serine phosphorylation can be a process of transferring phosphate groups of a donor (e.g., ATP or GTP) onto serine residues. The serine phosphorylation can be by means of a protein kinase. The serine phosphorylation may result in a change in protein activity.

In the present application, the term "isolated" typically refers to something obtained through artificial means from the natural state. For example, a certain unisolated polynucleotide or polypeptide naturally exists in a living animal, and the same polynucleotide or polypeptide with high purity isolated from the natural state may be called "isolated". The term "isolated" may not exclude the presence of artificial or synthetic substances, nor may it exclude the presence of other impure substances that do not affect the activity of the substance isolated.

In the present application, the term "nucleic acid molecule" typically refers to nucleotides of any length in isolated form, either deoxyribonucleotides or ribonucleotides, or analogs isolated from their natural environment or artificially synthesized.

In the present application, the term "vector" generally refers to a nucleic acid carrying tool into which polynucleotide encoding a certain protein can be inserted and the protein is expressed. A vector can be transformed, transduced, or transfected into a host cell to express a genetic material element it carries within the host cell. A vector may contain multiple elements that control expression. In addition, the vector may also include a replication initiation site. The vector may also include components that help it enter the cell.

In the present application, the term "cell" generally refers to a single cell, cell line, or cell culture that can be, or has been a recipient of a subject plasmid or vector, which includes the nucleic acid molecule of the present application or the vector of the present application. The cell can include the progeny of a single cell. Due to natural, accidental or intentional mutations, the progeny may not necessarily be identical to the original mother cell (in the morphology or genome of the total DNA complement). The cell may include a cell transfected in vitro using the vector according to the present application.

In the present application, the term "immunoconjugate" generally refers to a substance formed by connecting a polypeptide with other active agents, which can be small molecule active agents, such as therapeutic agents, imaging probes, or spectral probes.

In the present application, the term "pharmaceutical composition" typically refers to a composition for use in prevention/treatment of diseases or disorders. The pharmaceutical composition may include the isolated polypetide of the present application, the nucleic acid molecule of the present application, the vector of the present application, and/or the cell of the present application, and optionally a pharmaceutically acceptable adjuvant. In addition, the pharmaceutical composition may contain suitable preparations of one or more (pharmaceutically effective) carriers. The acceptable components of the composition can be non-toxic to the recipient at the doses and concentrations used. The pharmaceutical composition of the present application includes, but is not limited to, a liquid, frozen and lyophilized composition.

In the present application, the term "pharmaceutically acceptable carrier" typically refers to pharmaceutically acceptable carriers, excipients or stabilizers that are non-toxic at the doses and concentrations employed to the cells or mammals to which they are exposed. Physiologically acceptable carriers may include suitable substances. Pharmaceutically acceptable carriers are not usually the same substance as vectors used in genetic engineering to insert nucleic acids.

In the present application, the term "specific binding" or "specific" typically refers to a measurable and reproducible interaction, such as binding between a target and an antibody, that determines the presence of a target in the presence of a heterogeneous population of molecules, including biomolecules. For example, an antibody that specifically binds a target (which can be an epitope) can be an antibody that binds to that target with greater affinity, avidity, more readily, and/or for a greater duration than it binds to other targets. In some embodiments, an antibody specifically binds to an epitope on a protein, and the epitope is conserved across different species of proteins. In some embodiments, specific binding may include, but does not require exclusive binding.

In the present application, the term "neuron" generally refers to nerve cells which are main functional units of the nervous system. The neuron can consist of a cell body, a protuberant axon thereof and one or more dendritic processes. The neuron can transfer information to other neurons or cells by releasing the neurotransmitters at the synapse.

In the present application, the term "transmitter release" generally refers to neurotransmitter release, namely a process that a neuron acts on another neuron by releasing neurotransmitters wrapped in a vesicle to a synaptic space so as to transfer information. In the process of the transmitter release, the basic structure synapse of a neural loop can be involved. In some cases, the transmitter release can be called synaptic transmission. The method of the transmitter release may include synchronous release, asynchronous release and spontaneous release.

In the present application, the term "excitatory postsynaptic current (EPSC)" generally refers to the ion flow that causes the excitatory postsynaptic potential (EPSP). The EPSP stands for postsynaptic potential, which makes it easier for a postsynaptic neuron to trigger an action potential. This temporary depolarization of the postsynaptic membrane potential caused by the influx of positively charged ions into the postsynaptic cell is a result of opening ligand-gated ion channels. The frequency and/or amplitude of the EPSC can be recorded using voltage clamping.

In the present application, the term "release frequency" generally refers to the release frequency of an action potential. The action potential may refer to a process of quickly and reversibly reversing and restoring potentials on two sides of a primary membrane generated on the basis of a resting potential when excitable cells are stimulated. The action potential can consist of a peak potential and a post potential which respectively correspond to a depolarization process and a hyperpolarization process. The release of the action potential may have the characteristics of pulses. In some cases, the release frequency of the pulses is the ratio of the number of the released pulses to the time. For example, the release frequency may include the release frequency of neurotransmitters released from synaptic vesicles. For example, the release frequency may include the release frequency of excitatory postsynaptic currents.

In the present application, the term "learning ability" generally refers to all the abilities related to or required for learning/cognitive processes. The learning ability can include the ability to obtain new information, knowledge and/or skills through processes including experiencing, learning or accepting training, etc.. The learning ability can include imagination ability, attention ability, perceptual observation ability, reading ability, analysis ability, manipulation ability, adaption ability, summarization ability, problem solving ability or combinations thereof.

In the present application, the term "cognitive ability" generally refers to the ability to process information through perception. The cognitive ability can include the ability to grasp the composition of an object, the relationship of performance to other objects, the power of development, the direction of development and the basic rule.

In the present application, the term "motor ability" generally refers to the ability to participate in sports and training. The motor ability may include aerobic motor ability, muscle strength, body flexibility, balance ability and reaction ability. The motor ability can be a comprehensive representation of various factors such as physical form, quality, skills and psychological ability.

In the present application, the term "memory ability" generally refers to the ability to remember, maintain, re-recognize and reproduce the content and experience reflected by an objective object. The memory ability may include sensory memory ability, short-term memory ability and long-term memory ability.

In the present application, the term "spatial exploration ability" generally refers to the ability to explore the shape and/or position of an object. The spatial exploration ability includes observation, thinking, imagination, cognition and/or exploration of the shape and/or position of an object.

In the present application, the term "evaluation score of learning ability" generally refers to a quantitative evaluation score of the learning ability of a subject. The evaluation score of learning ability can be obtained by attention/performance function evaluation (e.g., Wester Memory test), language ability evaluation (e.g., language screening test (LAST)), view space and structural ability evaluation (e.g., visual motor integration test, Hooper visual tissue test, item piecing test, graphic arrangement test, and clock test), operational ability evaluation, daily function evaluation (e.g., disability assessment for dementia (DAD)) and/or neuropsychiatric scale. In some cases, the evaluation score of learning ability can be derived from the results obtained by mini-mental state examination (MMSE), Montreal cognitive assessment (MoCA), Alzheimer's disease assessment scale-cog (ADAS-cog) and clinical dementia rating scale (CDR).

In the present application, the term "novel object recognition test" generally refers to a test to enable an animal (e.g., a mouse) to identify an object in a specific space so as to detect the time for the animal to learn to identify a novel object. In some cases, the novel object recognition test can refer to the test methods described in Ennaceur et al., Behav Brain Res 80 9-25, 1996. For example, the novel object recognition test may include the following steps: placing two identical objects in a fixed-volume container, placing a mouse in the container to identify the two objects, replacing one of the two objects in the same container with a novel object having different shape after a certain period of time, and then measuring the mouse search time for the novel object.

In the present application, the term "water maze test" generally refers to a test to force an animal (e.g., a mouse) to swim, thereby learning to find a platform hidden in water. The water maze test (e.g., Morris water maze) can test the mouse ability to learn and/or memory spatial positional and directional senses. The water maze test may also include acquisition training, probe training, reversal training, or reversal probe training. If the time required for the animal (e.g., the mouse) to enter water to find the platform is shorter, the distance of movement during this process is about shorter, the evaluation score of the animal (e.g., the mouse) learning ability is correspondingly about higher. The water maze evaluation test can be an important test to evaluate learning ability.

In the present application, the term "cognitive impairment" generally refers to diseases and disorders that are deemed or confirmed to be involved in the progressive loss of neuronal structure and/or function (including neuronal death) or associated therewith. For example, the characteristics of the cognitive impairment may include impairment in cognition (e.g., memory, attention, perception, and/or thinking). These impairments can include pathogen-induced cognitive impairments, such as HIV-associated cognitive impairment and Lyme disease-associated cognitive impairment. Examples of cognitive impairment can include Alzheimer's disease, Huntington's disease, Parkinson's disease, amyotrophic lateral sclerosis (ALS), autism, early cognitive impairment (MCI), stroke, traumatic brain injury (TBI), and/or age-related memory impairment (AAMI).

In the present application, the term "neurodegenerative disease" generally refers to cognitive impairment such as dementia caused by gradual loss of neuronal structure and function, including neuronal death and glial cell balance. In some cases, age (e.g., Alzheimer's disease (AD), Parkinsons Disease (PD)) or gene mutations affecting CNS cell function (e.g., Huntington's chorea, premature AD or PD, Amyotrophic Lateral Sclerosis (ALS)) may cause the neurodegenerative disease. The neurodegenerative disease may have a change and/or condition selected from the group consisting of: protein misfolding and aggregation; neuroinflammation (e.g., CNS inflammation occurring under signal stimulation of toxic stimulation (e.g., protein aggregation), infection, traumatic injury or autoimmunity); change in cellular signal transduction; acquired aging/cell death (e.g., interrupted apoptosis signal transduction, mitochondrial dysfunction, autophagy impairment and stress/inflammation activation of necrotic bodies); and motor cell impairment and epigenetic changes.

In the present application, the term "presenile dementia" can be used interchangeably with the term "Alzheimer's disease". The presenile dementia may include the early stage, middle stage and/or late stage of presenile dementia. For example, learning and memory impairments become more pronounced in patients with early-stage presenile dementia, and in some cases, language impairments, executive impairments, cognitive impairments (agnosia), and/or skill executive impairments (apraxia) may occur. For example, patients in the middle stage of Alzheimer's disease will lose the ability to live independently and can be unable to perform most daily activities (in some cases, such patients may present with anomia, paraphasia, and/or anosognosia). For example, patients with advanced presenile dementia can become dependent on caregivers in later stages. For example, they may have completely lost their ability to speak. For example, they can be unable to feed themselves.

In the present application, the term "Alzheimer's disease" generally refers to presenile dementia, and senile dementia, and it is a neurodegenerative disease that is slow in progression and deteriorates over time. The most common early symptoms are loss of short-term memory (difficult to remember the event occurred recently); and when the disease progresses gradually, it may develop gradually at least one of the following symptoms: language disorders, directional disorders (e.g., easy to lose the way), emotional instability, loss of motivation, inability to self-care and behavioral problems. The true cause of Alzheimer's disease is still unknown up to now, and its progression can be related to the deposition of amyloid protein plaques in the brain and the formation of fibrillary tangles caused by excessive phosphorylation of Tau protein. There is no treatment that can prevent or reverse the course of disease, but only methods or permissions to temporarily alleviate or ameliorate the symptoms are available.

In the present application, the term "Aβ" generally refers to any peptide obtained by β-secretase-mediated cleavage of β-amyloid precursor protein (APP). For example, the Aβ may include peptides of 37, 38, 39, 40, 41, 42, and 43 amino acids, and extend from the β-secretase cleavage site to amino acids 37, 38, 39, 40, 41, 42, or 43. The Aβ can also be an N-terminal truncated form of the above peptides, such as pyroglutamic acid forms pE3-40, pE3-42, pE3-43, pE11-42, pE11-43 and similar forms.

In the present application, the term "Tau" generally refers to Tau protein and components of extensive Tau aggregates (e.g., neurofibrillary tangles) that are associated with the stabilization of microtubules in nerve cells. The Tau tangles may include oligomeric and/or fibrous forms of Tau, which are toxic. The Tau may also include all types and forms (e.g., different alternatively spliced forms) of Tau.

In the present application, the terms "mild cognitive impairment (MCI)" and "early cognitive impairment" are used interchangeably, and generally refer to an intermediate clinical state between normal cognition and cognitive impairment. In some cases, the MCI may include cognitive impairment that meets the dementia criteria but to an extent exceeding normal aging. MCI can be diverse in clinical manifestations, etiology, prognosis, and morbidity. In some cases, MCI can be a pathological stage of Alzheimer's disease. Certain forms of cognitive impairment can be considered as an early manifestation of neurodegenerative disease, ultimately causing dementia. In the present application, the terms "moderate-stage cognitive impairment" and "moderate cognitive impairment" are used interchangeably and may include more severe memory impairment that affects the patient's ability to live independently, and/or can be accompanied by sphincter dysfunction. In the present application, the terms "advanced-stage cognitive impairment" and "severe cognitive impairment" can be used interchangeably. They can encompass severe intellectual impairment, inability to take care of oneself, complete reliance on others for assistance, and/or obvious sphincter dysfunction. The severity of cognitive impairment can be determined through clinical manifestations, impairment of daily living abilities, or cognitive assessments. For example, the severity of cognitive impairment can be diagnosed using the activity of daily living scale (ADL), the clinical dementia rating (CDR), or the global deterioration scale (GDS). In some cases, the MCI may include subtypes selected from the group consisting of: aMCI-s: single-domain amnestic MCI; aMCI-m: multi-domain amnestic MCI; naMCI-s: single-domain non-amnestic MCI; and naMCI-m: multi-domain non-amnestic MCI.

In the present application, the term "normal aging-induced cognitive impairment" generally refers to cognitive impairment due to normal aging. For example, the normal aging-induced cognitive impairment may manifest as: memory loss, confusion in the location of familiar places, longer time to complete daily work than usual, or changes in mood and character.

In the present application, the term "LBD" generally refers to Lewy body detmentia or dementia with Lewy bodies. Lewy body detmentia is characterized by abnormal accumulation of proteins into lumps called Lewy bodies. Lewy body dementia results in gradual decline in cognitive ability. Patients with Lewy body detmentia may have a hallucination and change in alertness and attention. Other effects include muscle stiffness, slow movement, difficulty in walking, and trembling. Patients with the Lewy Body in the brain may also have plaque and tangles associated with Alzheimer's disease.

In the present application, the term "frontotemporal dementia" generally refers to pick disease, which is a rare disease that a tau protein only affects the progressiveness of the frontal and temporal lobes of the brain. Frontotemporal dementia patients are difficult in higher levels of reasoning, expression, perception, and language memory formation. The frontal and temporal lobes of the brain of frontotemporal dementia patients may atrophy over time.

In the present application, the term "vascular dementia" generally refers to problems in reasoning, judgment, and memory due to damage to brain blood flow. For example, the vascular dementia may include dementia due to factors at risk of heart disease and stroke, such as hypertension and hypercholesterolemia.

In the present application, the term "multi-infarct type" generally refers to non-cortical small infarcts caused by a single perforating branch occlusion of large cerebral artery. The multi-infarct type can be a particular type of cerebral infarction, also known as ischemic stroke. The multi-infarct type may manifest as: paraesthesia, aphasia, dyskinesia, slowness of action, awkwardness (particularly more difficult in fine actions such as writing).

In the present application, the term "Parkinson's disease" generally refers to a progressive neurodegenerative disease. Clinical features of the Parkinson's Disease (PD) may include motor symptoms (e.g., tremor, slowness of motion, myotonia, and postural instability), as well as neuropsychiatric and other non-motor manifestations. For example, the non-motor manifestations may include cognitive dysfunction and dementia, mood disorders (e.g., depression, anxiety, apathy), and sleep disorders.

In the present application, the term "AIDS" generally refers to acquired immunodeficiency syndrome (AIDS). Clinical manifestations of AIDS include changes in memory, concentration, attention, and motor skills. In some cases, AIDS patients may experience cognitive impairments, for example, about 50% of infected persons may further develop into HIV-associated neurocognitive dysfunction (HAND).

In the present application, the term "Creutzfeldt-Jakob disease (CJD)" generally refers to an infectious spongiform encephalopathy occurring in humans. CJD is a disease caused by prion infection. CJD patients may manifest as: paradoxical behavior, blurred consciousness, loss of appetite and weight, depression, few patients with visual or auditory abnormalities; and during progression, progressive deterioration of the nervous system (e.g., dysesthesia, language disorder and aphasia).

In the present application, the term "multiple sclerosis (MS)" generally refers to a demyelinating neuropathy. The MS patients suffer from damage to the insulating material (i.e. myelin sheath) on the surface of nerve cells in their brain or spinal cord, impaired signal transduction of the nervous system, which may lead to a series of possible symptoms affecting the activity, mind, and even mental state of the patient. These symptoms may include diplopia, unilateral vision impairment, muscle weakness, sensory retardation, or dysordination.

In the present application, the term "amyotrophic lateral sclerosis (ALS)" generally refers to the symptoms of amyotrophic lateral sclerosis, and motor neuron disease, and is a progressive and fatal neurodegenerative disease. A few ALS patients may suffer from frontotemporal dementia. Some ALS patients suffer from degeneration in sense, vision, touch, smell and taste, and a very few patients suffering from amyotrophic lateral sclerosis may suffer from dementia at the same time.

In the present application, the term "Huntington's disease (HD)" is Huntington's chorea, generally a genetic disease that may cause brain cell death. HD patients may suffer from more significant body movement disharmony, with gradual deterioration of ability until movement becomes difficult, unable to speak, as the disease progresses. Cognitive abilities generally decline, leading to dementia

In the present application, the term "aged stage" generally refers to an aging stage of a subject. For example, humans in the aged stage can be more than 60 years old, more than 70 years old or more than 75 years old; and mice in the aged stage can be more than 10 months old, for example more than 13 months old or more than 18 months old. In some cases, the subject of the aged stage may have one or more symptoms of learning deficits, memory disorders, memory deficits and/or brain dysfunction.

In the present application, the term "subject" typically refers to a human or a non-human animal, including, but is not limited to a cat, dog, horse, pig, cow, sheep, rabbit, mouse, rat, or monkey.

In the present application, the terms "stroke" or "cerebral infarction" generally refer to a condition caused by the blockage or bleeding of one or more blood vessels supplying blood to the brain, resulting in cell death, including stroke and the damage caused by stroke. For example, stroke can be an acute cerebrovascular disease. The stroke according to the present application includes acute stroke. The stroke according to the present application includes acute ischemic stroke and/or acute hemorrhagic stroke. In the present application, "ischemic stroke" generally refers to a stroke that occurs when one or more of the blood vessels supplying blood to the brain are blocked. The ischemic stroke according to the present application may include various types of ischemic stroke classified by etiology/pathogenesis. Ischemic stroke can be caused by thrombosis, embolism and/or hypotension. Thrombus can be caused by atherosclerosis, aneurysm, vascular malformation, arteritis and/or vasospasm. The ischemic stroke according to the present application includes cerebral infarction, and the cerebral infarction may include lacunar infarction, ischemic cerebral infarction and/or hemorrhagic infarction. The types of ischemic stroke according to the present application may include, for example, embolic stroke, cardiac embolic stroke, thrombotic stroke, large vessel stroke, lacunar infarction, arterial-arterial stroke, and occult stroke. The types of ischemic stroke according to the present application may include ischemic stroke caused by different reasons. For example, according to the TOAST (Trial of Org 10172 in Acute Stroke Treatment, TOAST) classification, the ischemic stroke according to the present application may include five types of ischemic stroke: large artery atherosclerosis stroke, cardiogenic embolic stroke, small artery occlusion stroke, stroke of other determined etiology and stroke of undetermined etiology. Other determined etiologies may include non-atherosclerotic vascular diseases, hypercoagulable states, and blood disorders, such as: aortic dissection; blood clotting disorders; changes in blood components (polycythemia); vasculitis (leptospirosis, syphilis, etc.); vascular malformations (arteriovenous malformations, moyamoya disease); connective tissue diseases (SLE, pulseless disease); dehydration; trauma; fibromuscular dystrophy; compressive vascular diseases; and drug addiction. In the present application, "hemorrhagic stroke" generally refers to a stroke caused by bleeding in one or more blood vessels that supply blood to the brain. The hemorrhagic stroke may include brain parenchymal hemorrhage, intraventricular hemorrhage and/or subarachnoid hemorrhage. The hemorrhagic stroke may include primary cerebral hemorrhage and/or secondary cerebral hemorrhage. The hemorrhagic stroke may include aneurysmal subarachnoid hemorrhage. The hemorrhagic stroke can be caused by vascular malformation, aneurysm, blood disease, cerebral amyloid angiopathy, moyamoya disease, cerebral arteritis, anticoagulation or thrombolytic therapy and/or tumor stroke.

In the present application, the term "impairment caused by stroke" generally refers to the conditions directly or indirectly induced by stroke. The impairment caused by stroke can include damage that occurs simultaneously with the stroke as well as damage that occurs after the stroke. The impairment caused by stroke may include damage to the brain. The impairment caused by stroke is not limited to the location of the stroke; it can include damage to the entire body of the stroke patient, as long as it is related to or occurs with the stroke. The impairment caused by stroke may manifest as impaired learning ability and/or behavioral ability in patients who have suffered a stroke. The impairment caused by stroke may manifest as impaired cognitive ability, motor ability and/or spatial exploration ability in patients who have suffered a stroke. The impairment caused by stroke can be detected by known means, including but not limited to imaging observation, novel object recognition test and water maze test. The impairment caused by stroke as according to the present application includes neurological symptoms, reduction in activities of daily living, and functional impairment.

In the present application, the term "comprising" generally means to including, generalizing, containing or encompassing. In some cases, it also means "being", "consisting of".

In the present application, the term "about" usually refers to a numerical range of 20% more or less than a specific value. For example, "about X" includes a range of values that are ±20%, ±10%, ±5%, ±2%, ±1%, ±0.5%, ±0.2%, or ±0.1% of X, where X is a numeric value.

### DETAILED DESCRIPTION of THE PRESENT INVENTION

In one aspect, the present application provides the use of a proton pump regulator in the preparation of a reagent for preventing and/or treating diseases, wherein the proton pump regulator regulates ATP6V1B2 and/or a functionally active fragment thereof, and the diseases comprise a stroke.

### ATP6V1B2 and functionally active fragments thereof

In the present application, the ATP6V1B2 can be derived from any organism. For example, the ATP6V1B2 can be derived from humans or mice.

In the present application, the ATP6V1B2 may comprise the amino acid sequence set forth in SEQ ID NO: 8 or 16.

In the present application, the functionally active fragment of the ATP6V1B2 can bind to the binding agent according to the present application. In the present application, the functionally active fragments of the ATP6V1B2 have the capacity of specifically binding to an amino acid sequence shown as SEQ ID NO: 5. For example, the functionally active fragments of the ATP6V1B2 may include a truncation of the ATP6V1B2. For example, the functionally active fragments of the ATP6V1B2 may comprise at least a portion of the amino acid sequence from position 287 to position 512 of human ATP6V1B2 protein. For example, the functionally active fragments of the ATP6V1B2 may comprise at least a portion of the amino acid sequence from position 287 to position 512 of mouse ATP6V1B2 protein.

In the present application, the ATP6V1B2 and/or functionally active fragments thereof may comprise the amino acid sequence set forth in any one of SEQ ID NOs: 8, 10-11, 16.

In the present application, the nucleic acid sequences encoding the ATP6V1B2 and/or functionally active fragments thereof may include the nucleic acid sequence as set forth in SEQ ID NO: 9 or 17.

In the present application, the activity of the ATP6V1B2 may include the biological activity of the ATP6V1B2 protein and/or functionally active fragments thereof (e.g., may include the resulting measurable downstream effect). For example, the activity of the ATP6V1B2 may include improving the expression levels and/or activity of the proton pump-associated protein. In the present application, the increase may include that the activity of the proton pump-related protein is increased by at least about 10% as compared with the activity of the original proton pump-related protein in the subject. For example, it may be increased by at least about 15%, at least about 20%, at least about 25%, at least about 30%, at least about 35%, at least about 40%, at least about 45%, at least about 50%, at least about 55%, at least About 60%, at least about 65%, at least about 70%, at least about 75%, at least about 80%, at least about 85%, at least about 90%, at least about 95%, at least about 100%, at least about 200%, at least about 500% or more.

### ATP6V1B2 regulator

In the present application, the ATP6V1B2 regulator can bind to ATP6V1B2. For example, the ATP6V1B2 regulator can be a binding agent for ATP6V1B2. In the present application, the binding of the ATP6V1B2 regulator to ATP6V1B2 can be specific. For example, the ATP6V1B2 regulator can bind to or associate with ATP6V1B2 with a Ka (i.e., the equilibrium association constant of the binding interaction, numerically 1/M) greater than or equal to about 10⁵M⁻¹ (such as, greater than or equal to about 10⁵M⁻¹, greater than or equal to about 10⁶M⁻¹, greater than or equal to about 10⁷M⁻¹, greater than or equal to about 10⁸M⁻¹, greater than or equal to about 10⁹M⁻¹, greater than or equal to about 10¹⁰M⁻¹, greater than or equal to about 10¹¹M⁻¹, greater than or equal to about 10¹²M⁻¹, greater than or equal to about 10¹³M⁻¹ or higher); or, with a equilibrium dissociation constant Kd less than or equal to about 10⁻⁵M (such as, less than or equal to about 10⁻⁵M, less than or equal to about 10⁻⁶M, less than or equal to about 10⁻⁷M, less than or equal to about 10⁻⁸M, less than or equal to about 10⁻⁹M, less than or equal to about 10⁻¹⁰M, less than or equal to about 10⁻¹¹M, less than or equal to about 10⁻¹²M, less than or equal to about 10⁻¹³M, or lower). For example, the binding of the ATP6V1B2 regulator to ATP6V1B2 and/or functionally active fragments thereof can be in vivo or in vitro.

In the present application, the ATP6V1B2 regulator may include small molecule compounds, polymers and/or biomacromolecules. In the present application, the ATP6V1B2 regulator may include proteins and/or polypeptides.

In the present application, the binding agent may comprise the amino acid sequence set forth in SEQ ID NO: 1 and/or SEQ ID NO: 2 and/or variants thereof. For example, the binding agent may comprise a polypeptide which, compared with the full-length amino acid sequence set forth in SEQ ID NO: 1 or SEQ ID NO: 2, has an amino-terminal deletion, a carboxy-terminal deletion, and/or an internal deletion or substitution, and whose remaining amino acid sequence is generally identical to the corresponding positions of the amino acid sequence set forth in SEQ ID NO: 1 or SEQ ID NO: 2. For example, the binding agent may comprise at least 60%, 70%, 80%, 90%, or 100% of the biological activity of a polypeptide having the amino acid sequence set forth in SEQ ID NO: 1 or SEQ ID NO: 2. For example, the binding agent can comprise a protein or polypeptide that has one or more amino acids substituted, deleted or added to the amino acid sequence set forth in SEQ ID NO: 1 or SEQ ID NO: 2. For example, the binding agent may comprise a polypeptide with amino acid alterations resulting from at least one, such as 1, 2, 3, 4, 5 or more amino acid substitution, deletion and/or insertion in the amino acid sequence set forth in SEQ ID NO: 1 or SEQ ID NO: 2.

For example, the binding agent may comprise homologs of the amino acid sequence set forth in SEQ ID NO: 1 and/or SEQ ID NO: 2. For example, the binding agent can comprise polypeptides that have at least about 40% (for example, at least about 43%, about 50%, about 57%, about 60%, about 62.5%, about 70%, about 71%, about 75%, about 80%, about 85%, about 86%, about 87%, about 88%, about 89%, about 90%, about 92%, about 95%, about 96%, about 97%, about 98%, about 99% or higher) sequence homology with the amino acid sequence set forth in SEQ ID NO: 1 and/or SEQ ID NO: 2.

In the present application, the binding agent may comprise the amino acid sequence set forth in SEQ ID NO: 54 and/or SEQ ID NO: 55 and/or variants thereof. For example, the binding agent may comprise a polypeptide which, compared with the full-length amino acid sequence set forth in SEQ ID NO: 54 or SEQ ID NO: 55, has an amino-terminal deletion, a carboxy-terminal deletion, and/or an internal deletion or substitution, and whose remaining amino acid sequence is generally identical to the corresponding positions of the amino acid sequence set forth in SEQ ID NO: 54 or SEQ ID NO: 55. For example, the binding agent may comprise at least 60%, 70%, 80%, 90%, or 100% of the biological activity of a polypeptide having the amino acid sequence set forth in SEQ ID NO: 54 or SEQ ID NO: 55. For example, the binding agent can comprise a protein or polypeptide that has one or more amino acids substituted, deleted or added to the amino acid sequence set forth in SEQ ID NO: 54 or SEQ ID NO: 55. For example, the binding agent may comprise a polypeptide with amino acid alterations resulting from at least one, such as 1, 2, 3, 4, 5 or more amino acid substitution, deletion and/or insertion in the amino acid sequence set forth in SEQ ID NO: 54 or SEQ ID NO: 55.

For example, the binding agent may comprise homologs of the amino acid sequence set forth in SEQ ID NO: 54 and/or SEQ ID NO: 55. For example, the binding agent can comprise polypeptides that have at least about 40% (for example, at least about 43%, about 50%, about 57%, about 60%, about 62.5%, about 70%, about 71%, about 75%, about 80%, about 85%, about 86%, about 87%, about 88%, about 89%, about 90%, about 92%, about 95%, about 96%, about 97%, about 98%, about 99% or higher) sequence homology with the amino acid sequence set forth in SEQ ID NO: 54 and/or SEQ ID NO: 55.

In the present application, the binding agent may comprise the amino acid sequence set forth in SEQ ID NO: 33 and/or variants thereof, where X can be any amino acid. For example, the binding agent may comprise the amino acid sequence set forth in any one of SEQ ID NOs: 30-32 and/or variants thereof, where X can be any amino acid. For example, the binding agent may comprise the amino acid sequence set forth in any one of SEQ ID NOs: 27-29 and/or variants thereof, where X can be any amino acid. For example, the binding agent may comprise the amino acid sequence set forth in any one of SEQ ID NOs: 19-26 and/or variants thereof, where X can be any amino acid.

In the present application, the ATP6V1B2 regulator may include a polypeptide, which may have at least 7 amino acids. For example, the polypeptide may have at least 8 amino acids. In the present application, the ATP6V1B2 regulator can comprise a polypeptide containing the polypeptide sequence according to the present application, having any number of amino acids.

In the present application, the ATP6V1B2 regulator can include a polypeptide, and the amino acids from the N-terminus to the C-terminus of the polypeptide are represented as X1, X2, X3, X4, X5, X6, X7, X8, and so on.

In an embodiment, X1 of the polypeptide can be serine, X2-X4 of the polypeptide can be any amino acid, X5 of the polypeptide can be valine, X6 of the polypeptide can be valine, and X7 of the polypeptide can be cysteine.

For example, X2 of the polypeptide can be proline. For example, X2 of the polypeptide can be a conservatively substituted amino acid of proline. For example, X2 of the polypeptide can be a non-conservatively substituted amino acid of proline. For example, X2 of the polypeptide can be alanine. For example, X2 of the polypeptide can be a conservatively substituted amino acid of alanine. For example, X2 of the polypeptide can be a non-conservatively substituted amino acid of alanine. For example, X2 of the polypeptide can be a non-polar amino acid. For example, X2 of the polypeptide can be a polar amino acid.

For example, X3 of the polypeptide can be valine. For example, X3 of the polypeptide can be a conservatively substituted amino acid of valine. For example, X3 of the polypeptide can be a non-conservatively substituted amino acid of valine. For example, X3 of the polypeptide can be alanine. For example, X3 of the polypeptide can be a conservatively substituted amino acid of alanine. For example, X3 of the polypeptide can be a non-conservatively substituted amino acid of alanine. For example, X3 of the polypeptide can be a non-polar amino acid. For example, X3 of the polypeptide can be a polar amino acid.

For example, X4 of the polypeptide can be aspartic acid. For example, X4 of the polypeptide can be a conservatively substituted amino acid of aspartic acid. For example, X4 of the polypeptide can be a non-conservatively substituted amino acid of aspartic acid. For example, X4 of the polypeptide can be alanine. For example, X4 of the polypeptide can be a conservatively substituted amino acid of alanine. For example, X4 of the polypeptide can be a non-conservatively substituted amino acid of alanine. For example, X4 of the polypeptide can be a non-polar amino acid. For example, X4 of the polypeptide can be a polar amino acid.

For example, the polypeptide may have X8, and X8 of the polypeptide can be any amino acid, for example, X8 of the polypeptide can be serine. For example, X8 of the polypeptide can be a conservatively substituted amino acid of serine. For example, X8 of the polypeptide can be a non-conservatively substituted amino acid of serine. For example, X8 of the polypeptide can be a non-polar amino acid. For example, X8 of the polypeptide can be a polar amino acid. For example, the polypeptide may not have X8.

In an embodiment, X1 of the polypeptide can be serine, X2 of the polypeptide can be any amino acid, X3 of the polypeptide can be valine or alanine, X4 of the polypeptide can be aspartic acid or alanine, X5 of the polypeptide can be valine, X6 of the polypeptide can be valine, X7 of the polypeptide can be cysteine, and X8 of the polypeptide can be serine or the polypeptide does not have X8.

In an embodiment, X1 of the polypeptide can be serine, X2 of the polypeptide can be proline or alanine, X3 of the polypeptide can be any amino acid, X4 of the polypeptide can be aspartic acid or alanine, X5 of the polypeptide can be valine, X6 of the polypeptide can be valine, X7 of the polypeptide can be cysteine, and X8 of the polypeptide can be serine or the polypeptide does not have X8.

In an embodiment, X1 of the polypeptide can be serine, X2 of the polypeptide can be proline or alanine, X3 of the polypeptide can be valine or alanine, X4 of the polypeptide can be any amino acid, X5 of the polypeptide can be valine, X6 of the polypeptide can be valine, X7 of the polypeptide can be cysteine, and X8 of the polypeptide can be serine or the polypeptide does not have X8.

In an embodiment, X1 of the polypeptide can be serine, X2 of the polypeptide can be any amino acid, X3 of the polypeptide can be any amino acid, X4 of the polypeptide can be aspartic acid or alanine, X5 of the polypeptide can be valine, X6 of the polypeptide can be valine, X7 of the polypeptide can be cysteine, and X8 of the polypeptide can be serine or the polypeptide does not have X8.

In an embodiment, X1 of the polypeptide can be serine, X2 of the polypeptide can be proline or alanine, X3 of the polypeptide can be any amino acid, X4 of the polypeptide can be any amino acid, X5 of the polypeptide can be valine, X6 of the polypeptide can be valine, X7 of the polypeptide can be cysteine, and X8 of the polypeptide can be serine or the polypeptide does not have X8.

In an embodiment, X1 of the polypeptide can be serine, X2 of the polypeptide can be any amino acid, X3 of the polypeptide can be valine or alanine, X4 of the polypeptide can be any amino acid, X5 of the polypeptide can be valine, X6 of the polypeptide can be valine, X7 of the polypeptide can be cysteine, and X8 of the polypeptide can be serine or the polypeptide does not have X8.

In an embodiment, X1 of the polypeptide can be serine, X2 of the polypeptide can be any amino acid, X3 of the polypeptide can be any amino acid, X4 of the polypeptide can be any amino acid except glycine, X5 of the polypeptide can be valine, X6 of the polypeptide can be valine, X7 of the polypeptide can be cysteine, and X8 of the polypeptide can be serine or the polypeptide does not have X8.

In an embodiment, X1 of the polypeptide can be serine, X2 of the polypeptide can be serine, X3 of the polypeptide can be any amino acid except cysteine, X4 of the polypeptide can be any amino acid, X5 of the polypeptide can be valine, X6 of the polypeptide can be valine, X7 of the polypeptide can be cysteine, and X8 of the polypeptide can be serine or the polypeptide does not have X8.

In an embodiment, X1 of the polypeptide can be serine, X2 of the polypeptide can be any amino acid except serine, X3 of the polypeptide can be any amino acid, X4 of the polypeptide can be any amino acid, X5 of the polypeptide can be valine, X6 of the polypeptide can be valine, X7 of the polypeptide can be cysteine, and X8 of the polypeptide can be serine or the polypeptide does not have X8.

In an embodiment, X1 of the polypeptide can be serine, X2 of the polypeptide can be serine, X3 of the polypeptide can be cysteine, X4 of the polypeptide can be any amino acid except glycine, X5 of the polypeptide can be valine, X6 of the polypeptide can be valine, X7 of the polypeptide can be cysteine, and X8 of the polypeptide can be serine or the polypeptide does not have X8.

In an embodiment, X1 of the polypeptide can be serine, X2 of the polypeptide can be serine, X3 of the polypeptide can be any amino acid except cysteine, X4 of the polypeptide can be glycine, X5 of the polypeptide can be valine, X6 of the polypeptide can be valine, X7 of the polypeptide can be cysteine, and X8 of the polypeptide can be serine or the polypeptide does not have X8.

In an embodiment, X1 of the polypeptide can be serine, X2 of the polypeptide can be any amino acid except serine, X3 of the polypeptide can be cysteine, X4 of the polypeptide can be glycine, X5 of the polypeptide can be valine, X6 of the polypeptide can be valine, X7 of the polypeptide can be cysteine, and X8 of the polypeptide can be serine or the polypeptide does not have X8.

In the present application, the ATP6V1B2 regulator may include a multimer. For example, the multimer may include a dimer. For example, the multimer may include a homodimer.

In the present application, the ATP6V1B2 regulator can be a polypeptide, the polypeptide may comprise cysteine, and the cysteine in the amino acid sequence of the polypeptide may not be subject to modification for sulfhydryl blocking. For example, if the cysteine residue in the amino acid sequence of the polypeptide is blocked by a sulfhydryl group, it may lose its ability to bind to the ATP6V1B2 protein.

In the present application, the ATP6V1B2 regulator can be a polypeptide, the polypeptide may comprise serine, and the serine in the amino acid sequence of the polypeptide may not be subject to modification for phosphorylation. For example, if the serine residue in the amino acid sequence of the polypeptide is phosphorylated, it may lose its ability to bind to the ATP6V1B2 protein.

In the present application, the ATP6V1B2 regulator may be fusion proteins and/or fusion polypeptides. For example, the fusion protein and/or fusion polypeptide may include a first moiety and a second moiety. For example, the fusion protein and/or polypeptide may include the ATP6V1B2 regulator of ATP6V1B2 as according to the present application and/or the polypeptide as according to the present application as a first moiety. For example, the fusion protein and/or polypeptide can include a second moiety that is the same as, different from, or not exactly the same as the first moiety.

For example, the first moiety and the second moiety are directly or indirectly linked. For example, the first moiety and the second moiety can be linked via a peptide bond. For example, the first moiety and the second moiety can be linked via a linker. For example, the first moiety and the second moiety can be linked via a non-peptide.

For example, the second moiety may have specific functions. For example, the second moiety can be transported across the blood-brain barrier to the brain. For example, the second moiety can comprise molecules capable of being transported across the blood-brain barrier to the brain. For example, the molecules capable of being transported across the blood-brain barrier to the brain comprise polypeptides. For example, the second moiety can comprise a cell-penetrating peptide. For example, the cell-penetrating peptide can be transported across the blood-brain barrier to the brain. For example, the second moiety comprises an amino acid sequence as set forth in SEQ ID NO: 34.

For example, the second moiety can bind to ATP6V1B2. For example, the second moiety can fully, incompletely or not at all bind to ATP6V1B2. For example, the second moiety can affect the binding of the first moiety to ATP6V1B2. For example, the second moiety can enhance the ability of the first moiety to bind to ATP6V1B2. For example, a fusion polypeptide or fusion protein comprising both the first and second moieties has a stronger ability to bind to ATP6V1B2 than the first moiety does to ATP6V1B2. For example, compared with the ability of the first moiety to bind to ATP6V1B2, the ability of the fusion polypeptide or fusion protein comprising both the first moiety and the second moiety to bind to ATP6V1B2 is at least about 1%, about 5%, about 10%, about 15%, about 20%, about 25%, about 30%, about 35%, about 40%, about 45%, about 50%, about 60%, about 70%, about 80%, about 90%, about 100%, or more stronger. For example, the second moiety can weaken the ability of the first moiety to bind to ATP6V1B2. For example, a fusion polypeptide or fusion protein comprising both the first and second moieties has a weaker ability to bind to ATP6V1B2 than the first moiety does to ATP6V1B2. For example, compared with the ability of the first moiety to bind to ATP6V1B2, the ability of the fusion polypeptide or fusion protein comprising both the first moiety and the second moiety to bind to ATP6V1B2 is at least about 1%, about 5%, about 10%, about 15%, about 20%, about 25%, about 30%, about 35%, about 40%, about 45%, about 50%, about 60%, about 70%, about 80%, about 90%, about 100%, or more weaker. For example, the bonding ability can be measured using techniques commonly used in the art. For example, the binding capacity can be represented by the equilibrium dissociation constant Kd. For example, the binding capacity can be determined by immunoprecipitation.

For example, the fusion protein and/or polypeptide may include the ATP6V1B2 regulator as according to the present application and/or the polypeptide as according to the present application as the first moiety and a cell-penetrating peptide as the second moiety. For example, the ATP6V1B2 regulator and the cell-penetrating peptide can be linked by peptide bonds to form a fusion polypeptide and/or a fusion protein.

For example, the fusion protein and/or polypeptide may comprise the amino acid sequence set forth in any one of SEQ ID NO: 48 and/or variants thereof, where X is any amino acid. For example, the fusion protein and/or polypeptide may comprise the amino acid sequence set forth in any one of SEQ ID NOs: 45-47 and/or variants thereof, where X is any amino acid. For example, the fusion protein and/or polypeptide may comprise an amino acid sequence as set forth in any one of SEQ ID NOs: 42-44 and/or variants thereof, wherein X is any amino acid. For example, the fusion protein and/or polypeptide may comprise an amino acid sequence as set forth in any one of SEQ ID NOs: 35-41 and/or variants thereof.

In the present application, the ATP6V1B2 regulator can regulate the expression level and/or activity of the proton pump-associated protein. For example, the ATP6V1B2 regulator can increase the expression level and/or activity of the proton pump-associated protein. For example, the expression level of the proton pump-associated protein includes the expression level of a gene encoding the proton pump-associated protein, the transcription level of the gene encoding the proton pump-associated protein, and/or the expression level of the proton pump-associated protein. For example, the improvement includes that the expression level and/or activity of the proton pump-associated protein is increased by at least about 10% as compared with the expression level and/or activity of original proton pump-associated protein in the subject. In the present application, the improvement includes that the expression level of the proton pump-associated protein is increased by at least about 5% as compared with the expression level of original proton pump-associated protein in the subject. For example, it can be increased by at least about 10%, at least about 15%, at least about 20%, at least about 25%, at least about 30%, at least about 35%, at least about 40%, at least about 45%, at least about 50%, at least about 55%, at least About 60%, at least about 65%, at least about 70%, at least about 75%, at least about 80%, at least about 85%, at least about 90%, at least about 95%, at least about 100%, at least about 200%, at least about 500% or more.

For example, the expression levels of proton pump-related proteins can be measured using conventional techniques in the art. For example, the expression level of the proton pump-associated protein is measured by implementing a test selected from the group consisting of: qPCR, qRT-PCR, hybridization analysis, RNA Northern blotting, dot blotting, in-situ hybridization, gel electrophoresis, capillary electrophoresis, column chromatography, western blotting, immunohistochemistry, immunostaining and mass spectrometry. For example, the expression level of the proton pump-associated protein can be measured, but not limited to, by using a substance selected from the group consisting of: a primer capable of specifically amplifying the gene encoding the proton pump-associated protein, nucleic acid molecules specifically bound to the gene encoding the proton pump-associated protein, nucleic acid molecules specifically bound to the proton pump-associated protein, small molecules specifically bound to the proton pump-associated protein, a probe specifically bound to the proton pump-associated protein, and polypeptide specifically bound to the proton pump-associated protein.

In the present application, the proton pump-associated proteins may include NADH dehydrogenase, coenzyme Q, succinate-coenzyme Q reductase, cytochrome c, and/or coenzyme Q-cytochrome c reductase.

In the present application, the activity of the proton pump-associated protein can include the biological activity of the proton pump-associated protein.

For example, the activity of the proton pump-associated protein can be measured through the activity level of a hydrogen/potassium adenosine triphosphate enzyme system (also known as hydrogen/potassium ion ATPase, i.e., H+/K+ ATPase); and/or activity level of an H2 receptor. In the present application, the increase of the activity of the proton pump-related protein may include that the activity of the proton pump-related protein is increased by at least about 5% as compared with the activity of the original proton pump-related protein in the subject. For example, it can be increased by at least about 10%, at least about 15%, at least about 20%, at least about 25%, at least about 30%, at least about 35%, at least about 40%, at least about 45%, at least about 50%, at least about 55%, at least About 60%, at least about 65%, at least about 70%, at least about 75%, at least about 80%, at least about 85%, at least about 90%, at least about 95%, at least about 100%, at least about 200%, at least about 500% or more.

In the present application, the ATP6V1B2 regulator can regulate the expression level and/or biological activity of ATP6V1B2 and/or functionally active fragments thereof. For example, the ATP6V1B2 regulator can increase the expression level and/or activity of the ATP6V1B2. In the present application, the increase may include that the expression level of ATP6V1B2 is increased by at least about 10% as compared with the expression level and/or activity of original ATP6V1B2 in the subject. For example, it may be increased by at least about 15%, at least about 20%, at least about 25%, at least about 30%, at least about 35%, at least about 40%, at least about 45%, at least about 50%, at least about 55%, at least About 60%, at least about 65%, at least about 70%, at least about 75%, at least about 80%, at least about 85%, at least about 90%, at least about 95%, at least about 100%, at least about 200%, at least about 500% or more.

For example, the expression level of the ATP6V1B2 comprises the expression level of the ATP6V1B2 gene, the transcription level of ATP6V1B2 gene and/or the expression level of the ATP6V1B2 protein. For example, the expression level may include the levels of polynucleotide, mRNA or amino acid product or protein of a specific gene (for example, human ATP6V1B2 gene). The expression level may include the levels of polynucleotides transcribed from, proteins translated from, or fragments of post-translationally modified proteins derived from the specific gene (e.g., human ATP6V1B2 gene).

For example, the expression levels of ATP6V1B2 can be measured using conventional techniques in the art. For example, the expression level of ATP6V1B2 is measured by implementing a test selected from the group consisting of: qPCR, qRT-PCR, hybridization analysis, RNA Northern blotting, dot blotting, in-situ hybridization, gel electrophoresis, capillary electrophoresis, column chromatography, western blotting, immunohistochemistry, immunostaining and mass spectrometry. For example, the expression level of ATP6V1B2 can be measured, but not limited to, by utilizing a substance selected from the group consisting of: a primer capable of specifically amplifying the ATP6V1B2 gene, nucleic acid molecules specifically bound to the ATP6V1B2 gene, nucleic acid molecules specifically bound to the ATP6V1B2 protein, small molecules specifically bound to the ATP6V1B2 protein, a probe specifically bound to the ATP6V1B2 protein, and polypeptides specifically bound to the ATP6V1B2 protein.

In the present application, the ATP6V1B2 regulator can improve cognitive abilities. For example, the cognitive ability may include cognitive abilities that can be measured through the novel object recognition behavioral test. For example, the cognitive ability may include cognitive abilities that can be measured through the water maze behavioral test. For example, the increase may include an improvement in the cognitive abilities of the subject after administration of the ATP6V1B2 regulator to the subject. For example, the improvement in the cognitive ability of the subject may be manifested by an increase in the time spent by the subject in the quadrant where the platform is located, a reduction in the interval between entries into the quadrant, and/or an increase in the number of crossings through the quadrant in the water maze behavioral test.

In the present application, the ATP6V1B2 regulator can increase the synaptic neurotransmitter release. For example, the increase includes that at least about 10% is increased as compared with the original synaptic neurotransmitter release level in the subject. For example, it can be increased by at least about 20%, at least about 50%, at least about 60%, at least about 65%, at least about 70%, at least about 75%, at least about 80%, at least about 85%, at least about 90%, at least about 95%, at least about 100%, at least about 200%, at least about 500% or more.

In the present application, the ATP6V1B2 regulator can increase the release frequency of excitatory postsynaptic current. In the present application, the increase includes that at least about 10% is increased as compared with the release frequency level of the excitatory postsynaptic current in the subject. For example, it can be increased by at least about 20%, at least about 50%, at least about 60%, at least about 65%, at least about 70%, at least about 75%, at least about 80%, at least about 85%, at least about 90%, at least about 95%, at least about 100%, at least about 200%, at least about 500% or more.

In another aspect, the present application provides use of a DIR regulator in the preparation of a reagent for preventing and/or treating diseases, wherein the DIR regulator regulates the intron-retained splicing product DIR of DNA damage-inducible transcript 4-like transcripts and/or functional fragments thereof, and the diseases comprise a stroke.

### DIR and functional fragments thereof

Abnormal splicing of the human DDIT4L gene results in the formation of an intron retention form (DIR). Sequence alignment revealed that the nucleic acid sequence is conserved in primates, with high conservation in chimpanzees, which are closely related to humans, and relatively poor conservation in macaques. However, it is not conserved in commonly used laboratory animals such as rats, mice, dogs, and pigs.

In the present application, the functional fragment generally refers to a polypeptide including an amino acid sequence that differs from the amino acid sequence of a parent or reference polypeptide (e.g., DIR) by at least one amino acid residue. In the present application, the functional fragment may have a higher (e.g., at least 80%) homology with the parent or reference polypeptide. The homology may include the similarity or identity of sequences. In the present application, the homology can be determined using standard techniques known in the art (see, for example, Smith and Waterman, Adv. Appl .Math. Advances in Applied Mathematics); and the percentage of identity common to polynucleotide or polypeptide sequences is determined by direct comparison of sequence information between molecules, the comparison being made by sequence alignment and identity determination using methods known in the art. Examples of algorithms suitable for determining sequence similarity are BLAST algorithms (see, Altschure et al., Jay Mohr. Bio. Journal of Molecular Biology, 215:403-410 [1990]). Software for BLAST analysis can be obtained by the National Center of Biotechnology Information (NCBI) in publication.

In the present application, the expression level of the DIR can include the expression level of the DIR gene, the transcription level of the DIR gene, and/or the expression level of the DIR protein. For example, the expression level may include the levels of polynucleotides, mRNA, amino acid products or proteins derived from specific genes (for example, the human *DDIT4L* gene; and/or the genes encoding human DIR and/or functional fragments thereof (e.g., DIR-I and/or DIR-II) (e.g., human DIR gene)). The expression level may include the levels of transcribed polynucleotides, translated proteins, or fragments of post-translational modified proteins of specific genes. In the present application, QDLIR can be used interchangeably with DIR, which can be an intron retention form produced by abnormal splicing during human *DDIT4L* gene expression. In the present application, the gene encoding the DIR can be referred to as the DIR gene.

In the present application, the expression levels of the functional fragments of the DIR (for example, DIR-I, and/or, DIR-II) can include the expression levels of the genes encoding the functional fragments of the DIR, the transcription levels of the genes encoding the functional fragments of the DIR, and/or the expression levels of the functional fragment proteins of the DIR. For example, the expression level may include the levels of polynucleotides, mRNA, amino acid products or proteins derived from specific genes (for example, genes encoding human DIR and/or functional fragments thereof (e.g., DIR-I and/or DIR-II)). The expression level may include the levels of polynucleotides transcribed from, proteins translated from, or fragments of post-translationally modified proteins derived from specific genes (for example, genes encoding the functional fragments of human DIR (e.g., DIR-I and/or DIR-II)).

In the present application, the DIR-I may be an amino acid sequence consisting of the first 27 amino acids from the N-terminus of IR (i.e., the amino acid sequence encoded by the retained intron, as set forth in SEQ ID NO: 58). The amino acid sequence of DIR-I is as set forth in SEQ ID NO: 59. The DIR-II can be an amino acid sequence consisting of the last 27 amino acids from the C-terminus of IR. The amino acid sequence of DIR-II is as set forth in SEQ ID NO: 60.

In the present application, the reduction may include a decrease of at least about 10% in the expression level of the DIR compared with the original expression level of the DIR in the subject. For example, it can be reduced by at least about 15%, at least about 20%, at least about 25%, at least about 30%, at least about 35%, at least about 40%, at least about 45%, at least about 50%, at least about 55%, at least About 60%, at least about 65%, at least about 70%, at least about 75%, at least about 80%, at least about 85%, at least about 90%, at least about 95%, at least about 100%, at least about 200%, at least about 500% or more.

In the present application, the expression level of the DIR may be determined by using substances selected from the group consisting of: primers that specifically amplify the DIR gene, nucleic acid molecules that specifically bind to the DIR gene, nucleic acid molecules that specifically bind to the DIR protein, small molecules that specifically bind to the DIR protein, probes that specifically bind to the DIR protein, and polypeptides that specifically bind to the DIR protein.

In the present application, the expression level of the functional fragments of the DIR (e.g., DIR-I and/or DIR-II) may be determined by using substances selected from the group consisting of: primers that specifically amplify the genes encoding the functional fragments of DIR, nucleic acid molecules that specifically bind to the genes encoding the functional fragments of DIR, nucleic acid molecules that specifically bind to the functional fragments (e.g., DIR-I and/or DIR-II), small molecules that specifically bind to the functional fragments (e.g., DIR-I and/or DIR-II), probes that specifically bind to the functional fragments (e.g., DIR-I and/or DIR-II), and polypeptides that specifically bind to the functional fragments (e.g., DIR-I and/or DIR-II).

In the present application, the expression level of the DIR and/or functional fragment thereof can be measured by implementing a test selected from the group consisting of: reverse transcription and amplification analysis (e.g., PCR, connected RT-PCR or quantitative RT-PCT), hybridization analysis, RNA Northern blotting, dot blotting, in-situ hybridization, gel electrophoresis, capillary electrophoresis, column chromatography, western blotting, immunohistochemistry, immunostaining or mass spectrometry. For example, the expression level of the DIR according to the present application can be measured through qPCR, qRT-PCR, northern hybridization, western hybridization and/or ELISA detection. The expression level of the DIR can also be measured by directly analyzing a biological sample or analyzing protein/nucleic acid separated from the sample.

In the present application, the activity of the DIR and/or functional fragment thereof can include the biological activity of the DIR protein. For example, the biological activity can include influencing the excitability of neurons and/or inhibiting the activity of neurons. For example, the biological activity can include inhibiting cognitive abilities by suppressing the excitability of neurons and/or the activity of neurons.

In the present application, the biological activity may include the ability to reduce the frequency of excitatory postsynaptic currents (EPSCs), and/or the ability to reduce the amplitude of EPSCs. For example, the reduction may comprise, by administering the DIR and/or functional fragment thereof and/or a nucleic acid encoding the DIR and/or functional fragment thereof, reducing the frequency of excitatory postsynaptic currents (EPSCs) in the subject, and/or reducing the amplitude of EPSCs in the subject, compared to the original biological activity of the DIR and/or functional fragment thereof in the subject.

For example, the functional fragment DIR-I of the DIR can reduce the frequency of EPSCs. For example, the reduction may include a decrease of at least about 10% in the frequency of EPSCs after the administration of the DIR-I, compared with the original frequency of EPSCs in the subject. For example, it can be reduced by at least about 15%, at least about 20%, at least about 25%, at least about 30%, at least about 35%, at least about 40%, at least about 45%, at least about 50%, at least about 55%, at least About 60%, at least about 65%, at least about 70%, at least about 75%, at least about 80%, at least about 85%, at least about 90%, at least about 95%, at least about 100%, at least about 200%, at least about 500% or more. For example, the functional fragment DIR-II of the DIR can reduce the frequency of EPSCs. For example, the reduction may include a decrease of at least about 10% in the amplitude of EPSCs after the administration of the DIR-II, compared with the original amplitude of EPSCs in the subject. For example, it can be reduced by at least about 15%, at least about 20%, at least about 25%, at least about 30%, at least about 35%, at least about 40%, at least about 45%, at least about 50%, at least about 55%, at least About 60%, at least about 65%, at least about 70%, at least about 75%, at least about 80%, at least about 85%, at least about 90%, at least about 95%, at least about 100%, at least about 200%, at least about 500% or more.

In the present application, the biological activity can include influencing cognitive abilities. In the present application, the biological activity may include participation in signaling pathways associated with Aβ deposition, and/or participation in signaling pathways associated with Tau tangle formation. For example, the DIR and/or functional fragments thereof can inhibit cognitive abilities. For example, the DIR and/or functional fragments thereof can suppress cognitive abilities by inhibiting signaling pathways associated with Aβ deposition, and/or, by inhibiting signaling pathways associated with the generation of Tau tangles.

In the present application, the reduction in the activity of the DIR and/or functional fragment thereof may comprise, by administering the DIR and/or functional fragment thereof and/or a nucleic acid encoding the DIR and/or functional fragment thereof, reducing the cognitive ability of the subject, compared to the original biological activity of the DIR and/or functional fragment thereof in the subject.

In the present application, the reduction may include a decrease of at least about 10% in the biological activity of the DIR compared with the original biological activity of the DIR in the subject. For example, it can be reduced by at least about 15%, at least about 20%, at least about 25%, at least about 30%, at least about 35%, at least about 40%, at least about 45%, at least about 50%, at least about 55%, at least About 60%, at least about 65%, at least about 70%, at least about 75%, at least about 80%, at least about 85%, at least about 90%, at least about 95%, at least about 100%, at least about 200%, at least about 500% or more.

In the present application, the expression level of the DIR and/or functional fragment thereof (e.g., the expression level in plasma) can be positively correlated with the Aβ level (e.g., the expression level in plasma). For example, the expression levels of the DIR and/or functional fragment thereof may be positively correlated with the expression levels of Aβ40. For example, the expression levels of the DIR and/or functional fragment thereof may be positively correlated with the expression levels of Aβ42.

In the present application, the expression levels of the DIR and/or functional fragment thereof (for example, the expression levels in the plasma) can be positively correlated with the degree of cognitive decline in the subject. For example, the expression levels of the DIR and/or functional fragments thereof can increase with the progression of cognitive impairment (e.g., disease progression of MCI and/or AD).

In the present application, the expression level of the DIR and/or functional fragment thereof (e.g., the expression level in plasma) can be positively correlated with the Aβ level (e.g., the expression level of Aβ-PET in the cortex). In the present application, the expression levels of the DIR and/or functional fragment thereof may be correlated with the formation of amyloid plaques in patients with Alzheimer's disease.

In the present application, the expression levels of the DIR and/or functional fragment thereof may be correlated with the storage of declarative memory. For example, the higher the expression level of the DIR and/or functional fragments thereof is, the lower the ability to store declarative memory becomes. In the present application, the expression levels of the DIR and/or functional fragment thereof may be correlated with the storage of associative learning. For example, the higher the expression level of the DIR and/or functional fragments thereof is, the lower the ability to store associative learning becomes.

In the present application, the reduction in the expression level of the DIR and/or functional fragment thereof may improve cognitive ability. In the present application, the expression level of the DIR and/or functional fragment thereof may be reduced by means of gene editing. For example, the gene editing method may include knock-down (e.g., using a CRISPR/Cas system; e.g., using antisense oligonucleotides). In the present application, the reduction of expression level may include a decrease of at least about 10% in the expression level of the DIR compared with the original expression level of the DIR in the subject. For example, it can be reduced by at least about 15%, at least about 20%, at least about 25%, at least about 30%, at least about 35%, at least about 40%, at least about 45%, at least about 50%, at least about 55%, at least About 60%, at least about 65%, at least about 70%, at least about 75%, at least about 80%, at least about 85%, at least about 90%, at least about 95%, at least about 100%, at least about 200%, at least about 500% or more.

In the present application, the lower the expression level of the DIR and/or functional fragment thereof, the higher the cognitive ability (measured, for example, through the novel object recognition behavioral test). For example, when the reduction in the expression level is a decrease of at least about 10% in the expression level of the DIR compared with the original expression level of DIR in the subject, the cognitive ability may be improved by at least about 10%. For example, it may be increased by at least about 15%, at least about 20%, at least about 25%, at least about 30%, at least about 35%, at least about 40%, at least about 45%, at least about 50%, at least about 55%, at least About 60%, at least about 65%, at least about 70%, at least about 75%, at least about 80%, at least about 85%, at least about 90%, at least about 95%, at least about 100%, at least about 200%, at least about 500% or more.

In the present application, the DIR/or functional fragment thereof may be derived from mammals. For example, it can be derived from primates. For example, it can be derived from humans.

In the present application, the DIR may comprise the amino acid sequence set forth in SEQ ID NO: 56.

In the present application, the functional fragment of the DIR may comprise an amino acid sequence encoded by the retained intron in DDIT4L. In the present application, the functional fragment of the DIR may comprise the amino acid sequence set forth in SEQ ID NO: 58.

In the present application, the functional fragment of the DIR may comprise the amino acid sequence set forth in any one of SEQ ID NOs: 59-60.

In the present application, the DIR/or functional fragment thereof may be involved in Aβ deposition. For example, the DIR/or functional fragment thereof may induce Aβ deposition through gelsolin.

The intron retained in DDIT4L (i.e. DIR-intron (IR), whose amino acid sequence is set forth in SEQ ID NO: 58) can be the main region for the interaction between DIR and Aβ. Aβ can facilitate the interaction between DIR and gelsolin. In the present application, the DIR-intron may be involved in Aβ deposition.

In the present application, the DIR/or functional fragment thereof can, under pathological conditions, bind to gelsolin, thereby causing Aβ deposition and the formation of amyloid plaques.

### DIR regulator

In the present application, the DIR regulator can bind to the DIR and/or functional fragments thereof. For example, the DIR regulator may bind to DIR-II.

For example, the DIR regulator may be a binding agent for the DIR and/or functional fragments thereof. In the present application, the binding of the DIR regulator to the DIR and/or functional fragments thereof may be specific. For example, the DIR regulator can bind to or associate with the DIR and/or functional fragments thereof with a Ka (i.e., the equilibrium association constant of the binding interaction, numerically 1/M) greater than or equal to about 10⁵M⁻¹ (such as, greater than or equal to about 10⁵M⁻¹, greater than or equal to about 10⁶M⁻¹, greater than or equal to about 10⁷M⁻¹, greater than or equal to about 10⁸M⁻¹, greater than or equal to about 10⁹M⁻¹, greater than or equal to about 10¹⁰M⁻¹, greater than or equal to about 10¹¹M⁻¹, greater than or equal to about 10¹²M⁻¹, greater than or equal to about 10¹³M⁻¹ or higher); or, with a equilibrium dissociation constant Kd less than or equal to about 10⁻⁵M (such as, less than or equal to about 10⁻⁵M, less than or equal to about 10⁻⁶M, less than or equal to about 10⁻⁷M, less than or equal to about 10⁻⁸M, less than or equal to about 10⁻⁹M, less than or equal to about 10⁻¹⁰M, less than or equal to about 10⁻¹¹M, less than or equal to about 10⁻¹²M, less than or equal to about 10⁻¹³M, or lower). For example, the binding of the DIR regulator to the DIR and/or functionally active fragments thereof can be in vivo or in vitro.

In the present application, the DIR regulator may include small molecule compounds, polymers and/or biomacromolecules. In the present application, the DIR regulator may include proteins and/or polypeptides.

In the present application, the DIR regulator can reduce the expression level and/or biological activity of the DIR and/or functional fragment thereof, and/or the nucleic acid encoding the DIR and/or functional fragment thereof.

In the present application, the DIR regulator can reduce the expression level of the DIR gene, the transcription level of the DIR gene, and/or the expression level of the DIR protein. For example, the regulator may reduce the levels of polynucleotides, mRNA, amino acid products or proteins derived from, for example, the human DDIT4L gene; and/or the genes encoding human DIR and/or functional fragments thereof (e.g., DIR-I and/or DIR-II) (e.g., human DIR gene). For example, the regulator may reduce the levels of polynucleotides transcribed from, proteins translated from, or fragments of post-translationally modified proteins derived from genes such as the human DDIT4L gene; and/or the genes encoding human DIR and/or functional fragments thereof (e.g., DIR-I and/or DIR-II) (e.g., human DIR gene).

In the present application, the DIR regulator may reduce the expression level of the genes encoding the functional fragments of DIR, the transcription level of the genes encoding the functional fragments of DIR, and/or the expression level of the protein of the functional fragments of DIR. For example, the regulator may reduce the levels of polynucleotides, mRNA, amino acid products or proteins derived from the genes encoding the functional fragments of human DIR (e.g., DIR-I and/or DIR-II). For example, the regulator may reduce the levels of polynucleotides transcribed from, proteins translated from, or fragments of post-translationally modified proteins derived from genes encoding the functional fragments of human DIR (e.g., DIR-I and/or DIR-II).

In the present application, the reduction may include a decrease of at least about 10% in the expression level of the DIR compared with the original expression level of the DIR in the subject. For example, it can be reduced by at least about 15%, at least about 20%, at least about 25%, at least about 30%, at least about 35%, at least about 40%, at least about 45%, at least about 50%, at least about 55%, at least About 60%, at least about 65%, at least about 70%, at least about 75%, at least about 80%, at least about 85%, at least about 90%, at least about 95%, at least about 100%, at least about 200%, at least about 500% or more.

In the present application, the reduction may include a decrease of at least about 10% in the expression level of the functional fragment of the DIR compared with the original expression level of the DIR in the subject. For example, it can be reduced by at least about 15%, at least about 20%, at least about 25%, at least about 30%, at least about 35%, at least about 40%, at least about 45%, at least about 50%, at least about 55%, at least About 60%, at least about 65%, at least about 70%, at least about 75%, at least about 80%, at least about 85%, at least about 90%, at least about 95%, at least about 100%, at least about 200%, at least about 500% or more.

In the present application, the DIR regulator can reduce the biological activity of the DIR and/or functional fragment thereof. In the present application, the reduction may include a decrease of at least about 10% in the biological activity of the DIR or functional fragments thereof compared with the original biological activity of the DIR in the subject. For example, it can be reduced by at least about 15%, at least about 20%, at least about 25%, at least about 30%, at least about 35%, at least about 40%, at least about 45%, at least about 50%, at least about 55%, at least About 60%, at least about 65%, at least about 70%, at least about 75%, at least about 80%, at least about 85%, at least about 90%, at least about 95%, at least about 100%, at least about 200%, at least about 500% or more.

In the present application, the DIR regulator can suppress signaling pathways associated with Aβ deposition. In the present application, the regulator can suppress signaling pathways associated with the generation of Tau tangles. In the present application, the inhibition may include a reduction of at least about 10% in the expression levels and/or biological activity of molecules involved in signaling pathways related to Aβ deposition and/or Tau tangle generation in the subject after administration of the regulator compared to the original expression levels and/or biological activity of these molecules. For example, it can be reduced by at least about 15%, at least about 20%, at least about 25%, at least about 30%, at least about 35%, at least about 40%, at least about 45%, at least about 50%, at least about 55%, at least About 60%, at least about 65%, at least about 70%, at least about 75%, at least about 80%, at least about 85%, at least about 90%, at least about 95%, at least about 100%, at least about 200%, at least about 500% or more. In the present application, the regulator can inhibit Aβ deposition and/or amyloid plaque formation via gelsolin. For example, the regulator can inhibit the binding of the DIR/ or functional fragments thereof to gelsolin, thereby suppressing the deposition of Aβ and/or the formation of amyloid plaques.

In the present application, the DIR regulator can reduce the binding of DIR-II to gelsolin. For example, after the administration of the regulator, the binding level between DIR-II and gelsolin may be reduced by at least about 15%, at least about 20%, at least about 25%, at least about 30%, at least about 35%, at least about 40%, at least about 45%, at least about 50%, at least about 55%, at least About 60%, at least about 65%, at least about 70%, at least about 75%, at least about 80%, at least about 85%, at least about 90%, at least about 95%, at least about 100%, at least about 200%, at least about 500% or more.

In another aspect, the present application provides use of a proton pump regulator in the preparation of a reagent for preventing and/or treating diseases, wherein the proton pump regulator regulates ATP6V1B2 and/or a functional active fragment thereof, and the disease includes diseases associated with the intron-retained splicing product DIR of DNA damage-inducible transcript 4-like transcripts and/or a functional fragment thereof.

### ATP6VIB2 regulator as DIR regulator

In the present application, the ATP6V1B2 regulator can bind to the DIR and/or functional fragments thereof. In the present application, the ATP6V1B2 regulator can regulate the DIR and/or functional fragments thereof.

In the present application, the ATP6V1B2 regulator can bind to the DIR and/or functional fragments thereof. For example, the ATP6V1B2 regulator may bind to DIR-II. For example, the ATP6V1B2 regulator may be a binding agent for the DIR and/or functional fragments thereof. In the present application, the binding of the ATP6V1B2 regulator to the DIR and/or functional fragments thereof may be specific. For example, the ATP6V1B2 regulator can bind to or associate with the DIR and/or functional fragments thereof with a Ka (i.e., the equilibrium association constant of the binding interaction, numerically 1/M) greater than or equal to about 10⁵M⁻¹ (such as, greater than or equal to about 10⁵M⁻¹, greater than or equal to about 10⁶M⁻¹, greater than or equal to about 10⁷M⁻¹, greater than or equal to about 10⁸M⁻¹, greater than or equal to about 10⁹M⁻¹, greater than or equal to about 10¹⁰M⁻¹, greater than or equal to about 10¹¹M⁻¹, greater than or equal to about 10¹²M⁻¹, greater than or equal to about 10¹³M⁻¹ or higher); or, with a equilibrium dissociation constant Kd less than or equal to about 10⁻⁵M (such as, less than or equal to about 10⁻⁵M, less than or equal to about 10⁻⁶M, less than or equal to about 10⁻⁷M, less than or equal to about 10⁻⁸M, less than or equal to about 10⁻⁹M, less than or equal to about 10⁻¹⁰M, less than or equal to about 10⁻¹¹M, less than or equal to about 10⁻¹²M, less than or equal to about 10⁻¹³M, or lower). For example, the binding of the ATP6V1B2 regulator to the DIR and/or functional active fragments thereof can be in vivo or in vitro.

In the present application, the DIR regulator may include an ATP6V1B2 regulator. In the present application, the ATP6V1B2 regulator can reduce the expression level and/or biological activity of the DIR and/or functional fragment thereof, and/or the nucleic acid encoding the DIR and/or functional fragment thereof.

In the present application, the ATP6V1B2 regulator can reduce the expression level of the DIR gene, the transcription level of the DIR gene, and/or the expression level of the DIR protein. For example, the ATP6V1B2 regulator may reduce the levels of polynucleotides, mRNA, amino acid products or proteins derived from, for example, the human DDIT4L gene; and/or the genes encoding human DIR and/or functional fragments thereof (e.g., DIR-I and/or DIR-II) (e.g., human DIR gene). For example, the ATP6V1B2 regulator may reduce the levels of polynucleotides transcribed from, proteins translated from, or fragments of post-translationally modified proteins derived from genes such as the human DDIT4L gene; and/or the genes encoding human DIR and/or functional fragments thereof (e.g., DIR-I and/or DIR-II) (e.g., human DIR gene).

In the present application, the ATP6V1B2 regulator may reduce the expression level of the genes encoding the functional fragments of DIR, the transcription level of the genes encoding the functional fragments of DIR, and/or the expression level of the protein of the functional fragments of DIR. For example, the ATP6V1B2 regulator may reduce the levels of polynucleotides, mRNA, amino acid products or proteins derived from the genes encoding the functional fragments of human DIR (e.g., DIR-I and/or DIR-II). For example, the ATP6V1B2 regulator may reduce the levels of polynucleotides transcribed from, proteins translated from, or fragments of post-translationally modified proteins derived from genes encoding the functional fragments of human DIR (e.g., DIR-I and/or DIR-II).

In the present application, the reduction may include a decrease of at least about 10% in the expression level of the DIR compared with the original expression level of the DIR in the subject. For example, it can be reduced by at least about 15%, at least about 20%, at least about 25%, at least about 30%, at least about 35%, at least about 40%, at least about 45%, at least about 50%, at least about 55%, at least About 60%, at least about 65%, at least about 70%, at least about 75%, at least about 80%, at least about 85%, at least about 90%, at least about 95%, at least about 100%, at least about 200%, at least about 500% or more.

In the present application, the reduction may include a decrease of at least about 10% in the expression level of the functional fragment of the DIR compared with the original expression level of the DIR in the subject. For example, it can be reduced by at least about 15%, at least about 20%, at least about 25%, at least about 30%, at least about 35%, at least about 40%, at least about 45%, at least about 50%, at least about 55%, at least About 60%, at least about 65%, at least about 70%, at least about 75%, at least about 80%, at least about 85%, at least about 90%, at least about 95%, at least about 100%, at least about 200%, at least about 500% or more.

In the present application, the ATP6V1B2 regulator can reduce the biological activity of the DIR and/or functional fragment thereof. In the present application, the reduction may include a decrease of at least about 10% in the biological activity of the DIR or functional fragments thereof compared with the original biological activity of the DIR in the subject. For example, it can be reduced by at least about 15%, at least about 20%, at least about 25%, at least about 30%, at least about 35%, at least about 40%, at least about 45%, at least about 50%, at least about 55%, at least About 60%, at least about 65%, at least about 70%, at least about 75%, at least about 80%, at least about 85%, at least about 90%, at least about 95%, at least about 100%, at least about 200%, at least about 500% or more.

### Indications

### Diseases associated with the intron-retained splicing product DIR of DNA damage-inducible transcript 4-like transcripts and/or functional fragments thereof

In the present application, the disease associated with the intron-retained splicing product DIR of DNA damage-inducible transcript 4-like transcripts and/or functional fragments thereof may include diseases associated with the expression level and/or biological activity of the DIR. For example, the diseases associated with the DIR and/or functional fragment thereof can include those caused by an increase in the expression level and/or biological activity of the DIR. For example, in patients suffering from diseases associated with the DIR and/or functional fragment thereof, an increase in the expression level and/or biological activity of the DIR may be detected.

For example, compared with the expression level of the original DIR in the subjects or the expression level of the DIR in normal individuals, the expression level of the functional fragment of the DIR in the subjects with diseases associated with the DIR and/or functional fragments thereof is increased by at least about 10%. For example, it may be increased by at least about 15%, at least about 20%, at least about 25%, at least about 30%, at least about 35%, at least about 40%, at least about 45%, at least about 50%, at least about 55%, at least About 60%, at least about 65%, at least about 70%, at least about 75%, at least about 80%, at least about 85%, at least about 90%, at least about 95%, at least about 100%, at least about 200%, at least about 500% or more.

For example, compared with the biological activity of the original DIR in the subjects or the biological activity of the DIR in normal individuals, the expression level of the functional fragment of the DIR in the subjects with diseases associated with the DIR and/or functional fragments thereof is increased by at least about 10%. For example, it may be increased by at least about 15%, at least about 20%, at least about 25%, at least about 30%, at least about 35%, at least about 40%, at least about 45%, at least about 50%, at least about 55%, at least About 60%, at least about 65%, at least about 70%, at least about 75%, at least about 80%, at least about 85%, at least about 90%, at least about 95%, at least about 100%, at least about 200%, at least about 500% or more.

For example, the disease associated with the intron-retained splicing product DIR of DNA damage-inducible transcript 4-like transcripts and/or functional fragments thereof may include cognitive impairment, neurodegenerative diseases and/or stroke.

In the present application, the cognitive impairment may include mild cognitive impairment (MCI), moderate cognitive impairment, and severe cognitive impairment. For example, the cognitive impairments may include cognitive impairments caused by normal aging, Lewy body dementia (LBD), frontotemporal dementia and/or vascular dementia. For example, the inducing diseases of the cognitive impairment may include Alzheimer's disease, multi-infarct type, Parkinson's disease, AIDS and/or Creutzfeldt-Jakob disease (CJD). In the present application, the cognitive impairment may include multi-domain amnestic MCI (aMCI-m).

In the present application, the neurodegenerative disease includes acute neurodegenerative disease and chronic neurodegenerative disease. For example, the neurodegenerative disease includes neurodegenerative disease caused by neuronal death and glial cell homeostasis, neurodegenerative disease caused by aging, neurodegenerative disease caused by influence on the CNS cell function, neurodegenerative disease caused by abnormal communication between cells, and/or neurodegenerative disease caused by impaired cell motility.

### Stroke

In the present application, the ATP6V1B2 regulator and/or the DIR regulator can prevent and/or treat stroke. The ATP6V1B2 regulator provided in the present application and/or the DIR regulator provided in the present application can be used to ameliorate neurological symptoms, activities of daily living ability, and dysfunctions caused by stroke.

In the present application, the stroke may include acute stroke. For example, the stroke may include acute ischemic stroke and acute hemorrhagic stroke.

In the present application, the stroke may include acute ischemic stroke and/or hemorrhagic stroke.

In the present application, the ischemic stroke may include cerebral infarction. For example, the cerebral infarction may include lacunar infarction, ischemic cerebral infarction and/or hemorrhagic infarction. In the present application, the ischemic stroke may include ischemic stroke caused by any etiology. For example, the ischemic stroke may include large artery atherosclerotic ischemic stroke, cardioembolic ischemic stroke, small artery occlusion ischemic stroke, ischemic stroke of other determined etiologies, and ischemic stroke of undetermined etiologies. For example, the ischemic stroke may be caused by factors including: thrombus, embolism and/or hypotension. For example, the thrombus may be caused by factors including: atherosclerosis, aneurysm, vascular malformation, arteritis and/or vasospasm.

In the present application, the hemorrhagic stroke may include brain parenchymal hemorrhage, intraventricular hemorrhage and/or subarachnoid hemorrhage. For example, the hemorrhagic stroke may include primary cerebral hemorrhage and/or secondary cerebral hemorrhage. For example, the hemorrhagic stroke may include aneurysmal subarachnoid hemorrhage. For example, the hemorrhagic stroke may be caused by factors including: vascular malformation, aneurysm, blood disease, cerebral amyloid angiopathy, moyamoya disease, cerebral arteritis, anticoagulation or thrombolytic therapy and/or tumor stroke.

In the present application, the stroke may include impairments caused by stroke.

For example, the impairment may include impairments observed through imaging. For example, the impairment observed through imaging may include intracerebral hematoma, intraventricular hemorrhage and/or subarachnoid hemorrhage. For example, the impairment observed through imaging may include edema, hematoma and/or mass effect. For example, the impairment observed through imaging may include dense artery sign, insular ribbon sign, blurred or decreased density of the basal ganglia, occlusion of small penetrating cerebral arteries, edema and/or mass effect. In the present application, the imaging observation may include conventional imaging observation methods in the art. For example, the imaging observations may include, but are not limited to, ultrasound, CT, computed tomography angiography (CTA), computed tomography perfusion (CTP), magnetic resonance imaging (MRI), and digital subtraction angiography (DSA).

For example, the impairment may include impairment of learning ability, and the learning ability comprises cognitive ability, motor ability, memory ability, and/or spatial exploration ability.

In the present application, the learning ability may include all abilities that are related to or required for learning/cognitive processes. For example, the learning ability may include cognitive ability, motor ability, memory ability, and/or spatial exploration ability.

In the present application, the impairment of learning ability may mean that the assessment score of the subject's learning ability after the damage caused by stroke is decreased by about 10% compared with that obtained before the occurrence of stroke. For example, it may be reduced by at least about 15%, at least about 20%, at least about 30%, at least about 40%, at least about 50%, at least about 60%, at least about 65%, at least about 70%, at least about 75%, at least about 80%, at least about 85%, at least about 90%, at least about 95%, at least about 100%, at least about 200%, at least about 500% or more.

In the present application, the evaluation score of learning ability can be obtained by attention/performance function evaluation (e.g., Wester Memory test), language ability evaluation (e.g., language screening test (LAST)), view space and structural ability evaluation (e.g., visual motor integration test, Hooper visual tissue test, item piecing test, graphic arrangement test, and clock test), operational ability evaluation, daily function evaluation (e.g., disability assessment for dementia (DAD)) and/or neuropsychiatric scale. For example, the evaluation score of learning ability can be measured by a test selected from the group consisting of: novel object recognition test and water maze test.

In the present application, the novel object recognition test can evaluate the cognitive ability, motor ability and/or spatial exploration ability. In the present application, the novel object recognition test can enable a subject (such as a mouse) to explore and learn an object with a specific shape in a fixed container, and distinguish a novel object which appears in the fixed container and is different from the previous object in shape after several days according to the memory obtained by learning. If a mouse takes a shorter time to discriminate the novel object with a different shape several days later, its learning ability will be correspondingly higher. The water maze evaluation test can be an important experiment for evaluating the learning ability of the subject.

In the present application, the water maze test can evaluate the memory ability, motor ability and/or spatial exploration ability. In the present application, the water maze test (e.g., the Morris water maze) can force the subject (e.g., a mouse) to swim, thereby learning to locate the hidden platform in the water, and further assessing the mouse's spatial exploration ability and/or memory ability related to spatial localization and orientation. The water maze test may also include acquisition training, probe training, reversal training, or reversal probe training. The shorter the time taken by a mouse to locate the platform from the moment it enters the water, and the shorter the distance it travels during this period, the correspondingly higher its learning ability will be. The water maze evaluation test can be an important test to evaluate the learning ability of the subject.

### Subjects

In the present application, the subject may include mammals. For example, the subject may include a rodent and/or a primate, for example, the subject may include human.

In the present application, the subject may be of any age group. For example, the subject can be in the aged stage.

In the present application, the subjects may include non-stroke patients, non-cognitive impairment patients, and/or non-neurodegenerative disease patients. For example, the subject can be normal and/or healthy humans. For example, the subject may have needs and/or desires to further improve their ability to learn.

In the present application, the subjects may include stroke patients and/or patients with damage caused by stroke.

For example, the subjects may include patients with ischemic stroke and/or hemorrhagic stroke. For example, the subject may include patients with cerebral infarction. For example, the patient may include those with lacunar infarction, ischemic cerebral infarction and/or hemorrhagic infarction. For example, the patient may include those suffering from thrombus, embolism and/or hypotension. For example, the patient may include those suffering from atherosclerosis, aneurysm, vascular malformation, arteritis and/or vasospasm.

In the present application, the subject may include patients suffering from brain parenchymal hemorrhage, intraventricular hemorrhage and/or subarachnoid hemorrhage. For example, the subject may include patients suffering from primary cerebral hemorrhage and/or secondary cerebral hemorrhage. For example, the subject may include patients suffering from aneurysmal subarachnoid hemorrhage. For example, the patient may include those suffering from vascular malformation, aneurysm, blood disease, cerebral amyloid angiopathy, moyamoya disease, cerebral arteritis, anticoagulation or thrombolytic therapy and/or tumor stroke.

In the present application, the subject may further include patients suffering from impairments caused by stroke.

For example, the patient may include those with observed intracerebral hematoma, intraventricular hemorrhage and/or subarachnoid hemorrhage. For example, the patient may include those with observed edema, hematoma and/or mass effect. For example, the patient may include those with observed dense artery sign, insular ribbon sign, blurred or decreased density of the basal ganglia, occlusion of small penetrating cerebral arteries, edema and/or mass effect.

In the present application, the subject may include a patient with neurodegenerative disease. For example, the subject may include a patient with Alzheimer's disease. For example, patient with Alzheimer's disease can be in the pre, mild, middle, or late stages of Alzheimer's disease.

In the present application, the subject may include a patient with cognitive impairment. For example, the subject may suffer from mild cognitive impairment (MCI) (e.g., loss of short-term memory, difficult to express or understand abstract things, precarious emotion or behavior, difficult to learn new things and follow complex instructions, hypojudgment and/or need of other people to remind in basic self-care), moderate cognitive impairment (e.g., confusion of long-term memory and real-time memory, word inadequacy, behavioral character transition or instable emotion and/or need of other people to assist in self-care) or severe cognitive impairment (e.g., memory impairment, decline in physical activity and mental condition, unable to effectively express or communicate, unable to self-care and/or biological clock confusion). In the present invention, the subject may suffer from a disease which can induce the cognitive impairment. For example, the subject may suffer from Alzheimer's disease, multi-infarct type, Parkinsons disease, AIDS and/or Creutzfeldt-Jakob disease (CJD).

### prevention and/or treatment of a disease

The present application provides use of the proton pump regulator according to the present application and/or the DIR regulator according to the present application in the preparation of a reagent for preventing and/or treating diseases, wherein the disease includes a stroke.

The present application provides a method for preventing and/or treating a disease, which comprises administering the proton pump regulator according to the present application and/or the DIR regulator according to the present application to a subject in need thereof, wherein the disease includes a stroke.

The present application provides use of the proton pump regulator according to the present application and/or the DIR regulator according to the present application in the prevention and/or treatment of a disease, wherein the disease includes a stroke.

The present application provides use of the proton pump regulator according to the present application in the preparation of a reagent for preventing and/or treating diseases, wherein the disease includes diseases associated with the intron-retained splicing product DIR of DNA damage-inducible transcript 4-like transcripts and/or a functional fragment thereof.

The present application provides a method for preventing and/or treating a disease, which comprises administering the proton pump regulator according to the present application to a subject in need thereof, wherein the disease includes diseases associated with the intron-retained splicing product DIR of DNA damage-inducible transcript 4-like transcripts and/or a functional fragment thereof.

The present application provides use of the proton pump regulator according to the present application in prevention and/or treatment of a disease, wherein the disease includes diseases associated with the intron-retained splicing product DIR of DNA damage-inducible transcript 4-like transcripts and/or a functional fragment thereof.

The reagents according to the present application can be administered in any manner. For example, the reagent according to the present application may be administered orally and/or via injection. For example, the reagents according to the present application can be formulated in a form suitable for their administration routes. For example, the reagent according to the present application may be formulated for oral administration and/or injection administration.

The proton pump regulator according to the present application, the DIR regulator according to the present application, and/or the reagent for preventing and/or treating diseases according to the present application can be administered at a therapeutically effective dose. For example, a therapeutically effective dose may include an amount that, when given to a subject, effectively prevents or improves the symptoms of one or more diseases or conditions or the development of such diseases or conditions. For example, a therapeutically effective amount may include an amount of a binding compound sufficient to cause symptom improvement, such as an amount that treats, cures, prevents, or improves a medical condition of interest or increases the speed at which such a condition is treated, cured, prevented, or improved. For example, when an individual is given a single active ingredient, the therapeutically effective dose refers only to that ingredient. For example, when used in combination, the therapeutically effective dose refers to the total amount of active ingredients that produce a therapeutic effect, regardless of whether they are combined, administered sequentially, or administered simultaneously.

The proton pump regulator according to the present application and/or the DIR regulator according to the present application can prevent and/or treat stroke. The proton pump regulator according to the present application, the DIR regulator according to the present application can prevent and/or treat impairment caused by stroke.

The ATP6V1B2 according to the present application and/or the DIR regulator according to the present application can prevent and/or treat diseases associated with the intron-retained splicing product DIR of DNA damage-inducible transcript 4-like transcripts and/or functional fragments thereof For example, the ATP6V1B2 regulator can prevent and/or treat cognitive impairment, neurodegenerative diseases, and/or stroke associated with the DIR and/or functional fragments thereof.

In the present application, the preventive and/or therapeutic effect of the ATP6V1B2 regulator and/or the DIR regulator according to the present application on the damage caused by stroke may be observed by means of imaging. For example, the proton pump regulator according to the present application and/or the DIR regulator according to the present application can reduce the extent of cerebral infarction. For example, the cerebral infarction area of the subject is reduced by at least about 5% compared with that before the administration of the ATP6V1B2 regulator according to the present application and/or the DIR regulator according to the present application. For example, it may be reduced by at least about 10%, at least about 15%, at least about 20%, at least about 30%, at least about 40%, at least about 50%, at least about 60%, at least about 65%, at least about 70%, at least about 75%, at least about 80%, at least about 85%, at least about 90%, at least about 95%, at least about 100%, at least about 200%, at least about 500% or more.

In the present application, the prevention and/or treatment includes not only the prevention and/or treatment of a disease, but also generally includes preventing the onset of the disease, slowing or reversing the progression of the disease, preventing or slowing the onset of one or more symptoms associated with the disease, reducing and/or alleviating one or more symptoms associated with the disease, reducing the severity and/or duration of the disease and/or any symptoms associated with it and/or preventing a further increase in the severity of the disease and/or any symptoms associated with it, preventing, reducing or reversing any physiological damage caused by the disease, and any pharmacological effects that are generally beneficial to the patient being treated. For example, the binding agent according to the present application, the polypeptide according to the present application, the fusion polypeptide according to the present application, the immunoconjugate according to the present application, the nucleic acid molecule according to the present application, the vector according to the present application, the cell according to the present application, the pharmaceutical composition according to the present application, and/or the reagent for preventing and/or treating diseases according to the present application are not required to achieve a complete cure or eradicate any symptom or manifestation of the disease. As is recognized in the relevant art, a drug used as a therapeutic agent may reduce the severity of a given disease state, without the need to eliminate every manifestation of the disease to be considered a useful therapeutic agent. Similarly, a therapy administered prophylactically may constitute an effective prophylactic agent without the need to completely prevent the onset of the condition. Simply reducing the impact of the disease on the subject (e.g., by reducing the number or severity of their symptoms, or by increasing the effectiveness of another treatment, or by producing another beneficial effect), or reducing the likelihood of the disease occurring or worsening, is sufficient.

Without intending to be bound by any theory, the following examples are merely intended to illustrate the fusion proteins, preparation methods, uses, and the like of the present application, and are not intended to limit the scope of the present invention.

### EXAMPLES

### Example 1: Construction and characterization of a DIR-KI mouse model

### 1.1 Construction of a DIR-KI mouse model

Construction strategy: Since the first 20 amino acids of mouse DDIT4L are identical to those of DIR, CRISPR/Cas9 technology was used to insert the nucleic acid sequence encoding the remaining 64 amino acids of DIR directly into the nucleic acid sequence encoding the 20th amino acid (aspartic acid) in the mouse DDIT4L genome via homologous recombination (FIG. 1), so that the mouse can normally express human DIR (SEQ ID NO: 56).

In brief: First, Cas9 mRNA and sgRNA for knock-in (SEQ ID NO: 64) were obtained by in vitro transcription; then, a homologous recombinant vector was constructed as the donor, which contained a 5' homology arm of about 3 kb (SEQ ID NO: 65), a nucleic acid sequence encoding the last 64 amino acids of human DIR (SEQ ID NO: 66), and a 3' homology arm of about 3 kb (SEQ ID NO: 67). Next, the Cas9-encoding mRNA, sgRNA and donor vector were microinjected into the fertilized eggs of C57BL/6J mice to obtain F0-generation mice. Finally, F0-generation mice that were identified as positive by PCR were mated with C57BL/6J mice to obtain positive F1-generation mice. Mating F1-generation mice could provide F2-generation wild-type, heterozygous, and homozygous mice.

### 1.2 Identification of DIR-KI mice

Subsequently, the gene knock-in mice in Example 1 were verified to express human DIR normally through various methods such as genome identification, mRNA identification, immunohistochemical staining, and protein detection.

Genome identification: The tails of DIR-KI mice from Example 1 were collected, and genomic DNA was extracted from the mice using the TIANamp Genomic DNA Kit (TIANGEN), identification primers P1 (SEQ ID NO: 68) and P2 (SEQ ID NO: 69) were designed, and the genotypes of the mice were identified by PCR reaction combined with gel electrophoresis. A 476 bp PCR product can be obtained from wild-type (WT) mice, two PCR products of 476 bp and 671 bp can be obtained from DIR heterozygous (HE) mice, and a 671 bp PCR product can be obtained from DIR homozygous (HO) mice (FIG. 2A).

mRNA identification: Brain tissue from DIR-KI mice in Example 1 was collected and dispersed. Trizol (Sigma) reagent and isopropanol were added to extract mRNA. After washing with 70% ethanol solution and drying, it was dissolved in ultrapure water. One microgram of the mRNA was taken, and the mRNA was reverse-transcribed into cDNA using the Hifair^{®} II 1st Strand cDNA Synthesis Kit (Yeasen Biotechnology). Primers P3 (SEQ ID NO: 70), P4 (SEQ ID NO: 71), and P5 (SEQ ID NO: 72) were designed to identify the expression of DDIT4L and DIR by PCR. P3 and P4 were used to identify DDIT4L, and it was found that both wild-type mice and DIR-KI mice could normally express DDIT4L mRNA. P3 and P5 were used to identify DIR, and only DIR-KI mice were able to express DIR mRNA (FIG. 2B).

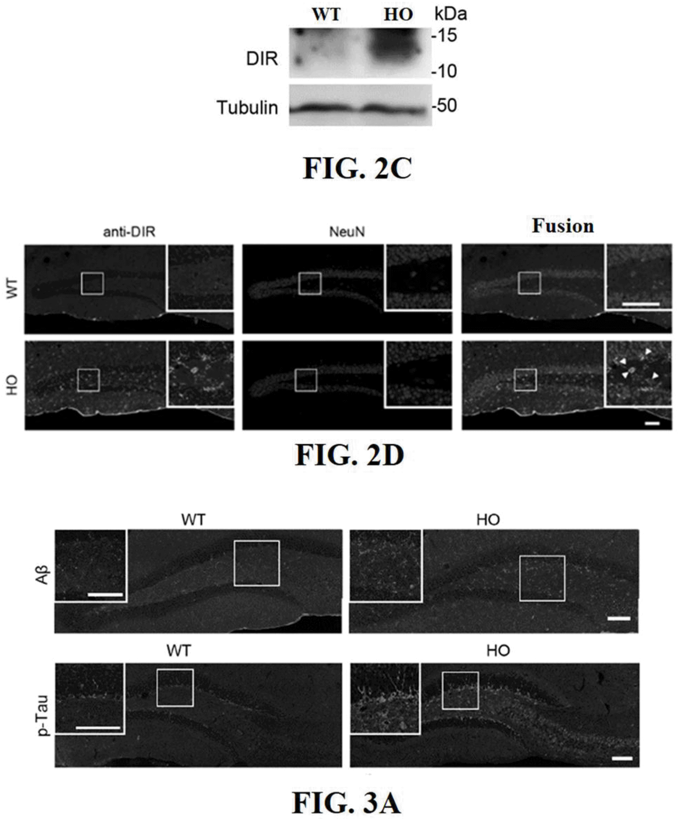

Protein detection: Brain tissue from DIR-KI mice and wild-type mice was lysed with lysis buffer (50mM Tris, 150mM NaCl, 1% Triton X-100, 1% sodium deoxycholate, 0.1% SDS, pH 7.4), centrifuged, and the supernatant was mixed with an appropriate amount of 4X loading buffer and denatured at 100°C for 10 min. Finally, an appropriate amount of sample was taken for a Western blot experiment. The results showed that DIR protein could be detected in the brain tissue of DIR-KI mice (FIG. 2C).

Immunohistochemical staining: Adult DIR-KI mice were anesthetized and perfused with PBS, and then perfused with Lana's fixative (45 mM NaH2PO4·2H2O, 115 mM Na2HPO4·12H2O, 4% PFA, 14% saturated picric acid). Immediately thereafter, the brain tissues of the mice were harvested and sectioned into tissue sections with a thickness of about 50 µm using a Leica cryostat. An appropriate amount of the sections were taken and mounted onto gelatin-precoated glass slides. After blocking with donkey serum, the sections were treated with the DIR antibody (GL Biochem) for labeling, followed by treatment with a fluorescent secondary antibody to label the DIR antibody. Finally, the labeled tissue sections were imaged using a Leica SP8 two-photon confocal microscope, which revealed that DIR was highly enriched in neurons. Immunostaining of brain sections from homozygous (HO) DIR-KI mice showed that DIR was expressed in many neurons of the dentate gyrus (DG) of the hippocampus, some neurons in the CA1 and CA3 regions, and a few cortical and thalamic neurons (FIG. 2D, scale bar = 100 µm.).

### 1.3 Physiological characteristics of DIR-KI mice

The activity of neurons in DIR-KI mice was detected by electrophysiological recording, and the presence of p-Tau elevation and Aβ accumulation was determined by immunohistochemical staining.

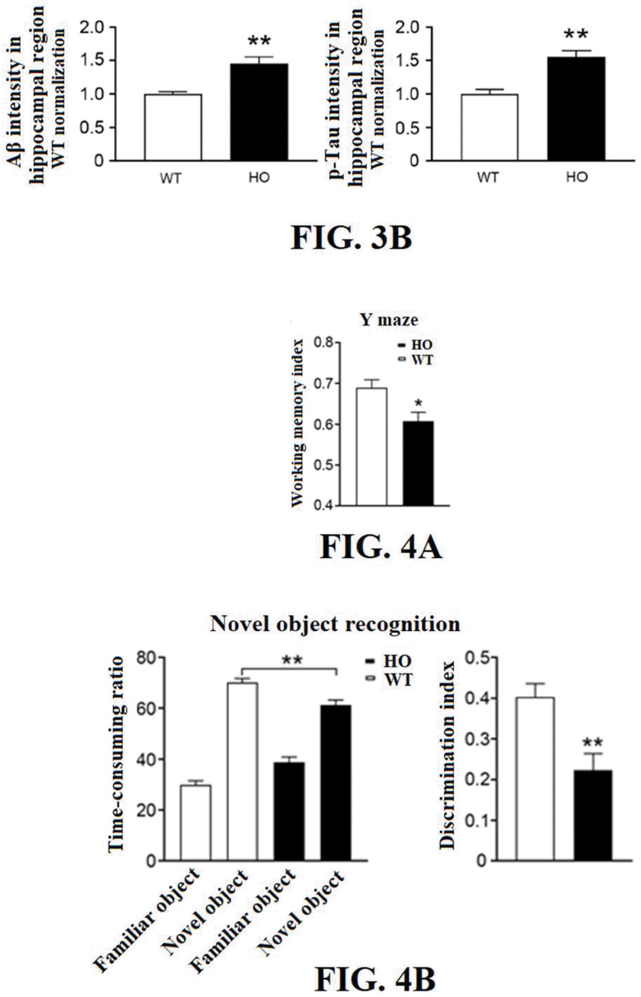

Immunohistochemical staining: Adult DIR-KI mice were anesthetized and perfused with PBS, and then fixed with 4% paraformaldehyde solution. Immediately thereafter, the brain tissues of the mice were harvested and sectioned into tissue sections with a thickness of about 50 µm using a Leica cryostat. An appropriate amount of the sections were taken and mounted onto gelatin-precoated glass slides. After blocking with donkey serum, the sections were incubated with p-Tau (Abcam, Cat. No. ab32057) and Aβ (Agrisera, Cat. No. AS0932) antibodies for labeling, followed by incubation with fluorescent secondary antibodies to label the p-Tau and Aβ antibodies. Finally, the labeled tissue sections were imaged using a Leica SP8 two-photon confocal microscope. The results showed that the contents of p-Tau and Aβ in the brain tissue of DIR-KI mice were significantly increased (FIG. 3), and the expression levels of Aβ and p-Tau in the dentate gyrus of the hippocampus of DIR-KI mice were increased (FIG. 3A, scale bar = 100 µm; FIG. 3B, **, p < 0.01).

### 1.4 Behavioral characteristics of DIR-KI mice

Various behavioral tests were used to verify whether DIR-KI mice exhibited memory impairment.

Y-maze test: Mice were allowed to acclimatize to their living environment for one week before conducting the behavioral test. On the day of the experiment, the mice were allowed to acclimatize to the experimental room for 30 min in advance. After the experiment began, the mice were placed facing the box wall in the starting arm A of the Y-shaped maze and allowed to explore the maze freely for 10 min. When the mouse is in the center of the maze, it can choose to enter from any direction. Cognitive-normal mice tend to enter unexplored arms during the experiment. The entire process was recorded using a camera. After the experiment, the mice were returned to their cages. The experimental apparatus was cleaned with 75% alcohol during the experimental intervals. After the experiment, the order in which the mice explored the maze arms was manually recorded to calculate the total number of arm entries and the alternation ratio (alternation ratio = actual number of alternating arm entries / (total number of arm entries - 2)). The experimenters and data analysts were kept double-blind. The results showed that the DIR-KI mice performed worse than wild-type mice, indicating that the working memory of DIR-KI mice was significantly reduced (FIG. 4A).

Novel object recognition test: On the day of the experiment, the mice were allowed to acclimatize to the experimental room for 30 min in advance. The test was divided into an acclimation period, a training period and a testing period. Acclimation period: The mice were removed from their cages and placed in the center of a plastic box to allow them to freely explore the experimental area for 10 min. The training period began 12-24 h after the acclimation period. During the training period, two identical batteries were placed 5 cm away from the box wall. The mice were placed in the center of the area and allowed to explore freely for 10 min. The testing period was conducted within 3-4 h after the end of the training period. During the testing period, 1 battery was replaced with a toy randomly, and the toy was placed in the same position as the original object. The mice were placed in the center of the area and allowed to explore freely for 10 min. After the experiment, the mice were returned to their cages. The entire process was recorded using a camera. The experimental apparatus was cleaned with 75% alcohol during the experimental intervals. After the experiment, the time taken for the mice to explore novel and familiar objects in the video was counted manually, and the time recognition index of the novel objects was calculated. When a mouse's nose is within 2 cm of an object, smelling or touching the object is considered exploratory behavior; biting or climbing on the object is not considered exploratory behavior. The recognition index of mice for novel objects = (time spent exploring novel objects - time spent exploring familiar objects) / (time spent exploring novel objects + time spent exploring familiar objects). Data from mice whose total exploration time was less than 15 s will be excluded. The experimenters and data analysts were kept double-blind. The results showed that DIR-KI mice had a weaker ability to recognize novel objects than wild-type mice (FIG. 4B).

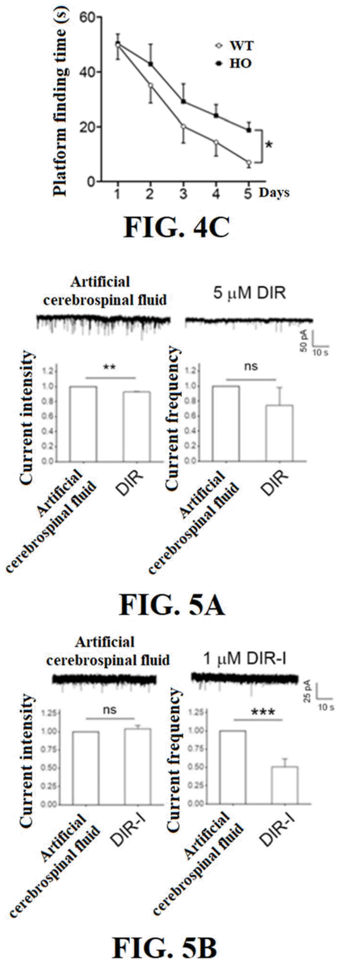

Water maze test: On the day of the experiment, the mice were allowed to acclimatize to the experimental room for 30 min in advance. The test was divided into training period and testing period. The training period consisted of 5-6 days, and the testing period was 1 day. Before the experiment, the water tank was filled with water to a depth of 30 cm, the water temperature was maintained at 19-22°C, and talcum powder was added to the water to make it cloudy. The water tank was divided into four directions: N, S, E, and W, the platform was placed in the NE quadrant, and the daily water entry points for the mice were selected as non-repeating combinations of S, W, NW and SE. The room setting remained unchanged during the experiment, with specific patterns affixed around the water maze as distant clues. The room lighting was not direct light. The entire experiment was recorded using a camera, and the recordings from the testing period were analyzed using Etho Vision XT 14 software. Training period: The platform was positioned 0.5 cm below the water surface. The mouse was placed into the water from the designated entry point, facing the tank wall, and a 1-min timer was started simultaneously. The timing was stopped when the mice reached the platform, and if the mice did not reach the platform within 1 min, the mice were placed on the platform or guided to the platform. After the mice had stayed on the platform for 30 s, they were taken out, dried off, and returned to their cages. A 30-min interval was required before the next entry into the water. The mice were placed at new entry points, and the above experiment was repeated three times. The researchers recorded the time it took for the mice to climb onto the platform in each experiment. The experiment was repeated the next day. The mice were trained 4 times per day for a total of 5-6 days. Testing period: test was performed 24 h after the end of the training day. The platform was removed. The mice entered the water from the SW quadrant, facing the tank wall. The mice were taken out after 1 min, dried off, and returned to their cages. After the experiment, the behavioral videos of the mice were analyzed using Etho Vision XT 14 software, and the collected data included the escape latency to enter the target quadrant, the number of times crossing the platform location, and the residence time in the target and non-target quadrants. The experimenters and data analysts were kept double-blind. The results showed that the number of times DIR-KI mice crossed the quadrant containing the platform was significantly reduced, and the time spent entering the quadrant containing the platform was significantly increased, indicating that the spatial memory ability of DIR-KI mice was weakened (FIG. 4C).

### 1.5 Disease models containing functionally active fragments of DIR

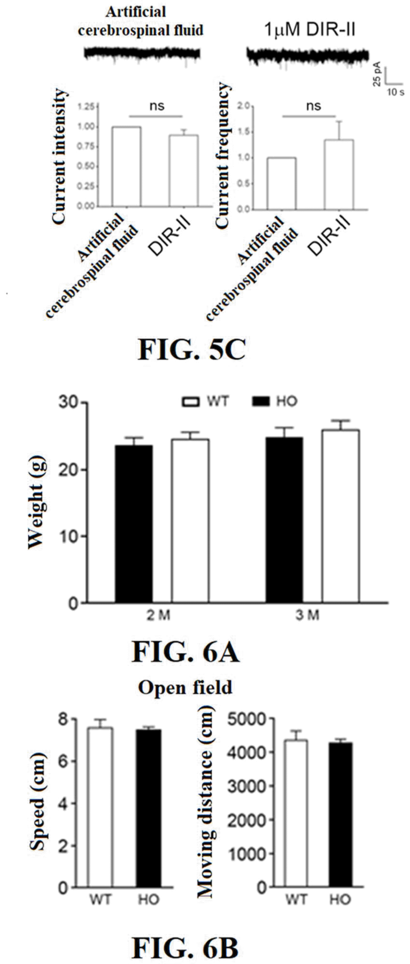

Electrophysiological recording of brain sections: Mouse brain tissue was placed in pre-cooled artificial cerebrospinal fluid (ACSF), continuously bubbled with 95% O2 and 5% CO2, and then sliced into sections with a thickness of approximately 350 µm using a Leica vibratome. After selecting the appropriate brain sections, they were placed in a recording chamber filled with artificial cerebrospinal fluid (ACSF). The chamber was fixed under the field of view of a microscope, and fresh artificial cerebrospinal fluid (ACSF) was continuously perfused to maintain the viability of the brain tissue. Finally, electrodes were used to record the postsynaptic currents evoked in the hippocampus. The results showed that after administration of the hippocampal section DIR-IR (SEQ ID NO: 58) fragment, the amplitude of the postsynaptic current was significantly reduced, and the frequency of the current also tended to decrease (FIG. 5A). The DIR-IR fragment was further divided into two parts, DIR-I (SEQ ID NO: 59) and DIR-II (SEQ ID NO: 60), and their effects on postsynaptic current were compared. After administering the DIR-I fragment to the hippocampal brain slices, there was no significant change in the amplitude of the postsynaptic current, but the frequency of the current decreased significantly (FIG. 5B). After administering the DIR-II fragment to the hippocampal brain sections, there was a tendency for the amplitude of the postsynaptic current to decrease, but the frequency of the current did not show any significant change (FIG. 5C). Therefore, it can be inferred that the fragments of DIR can at least partially or fully realize the functions of DIR.

### 1.6 The growth, metabolism and motor functions of the disease model remained unchanged.

By weighing the mice, no significant difference was found between wild-type mice (WT) and homozygous mice (HO), indicating that DIR knock-in does not affect the growth and metabolism of mice (FIG. 6A).

Open field test: On the test day, the mice were allowed to acclimate to the experimental room 30 min in advance. During the experiment, the mice were removed from their cages and placed in the center of a plastic box to allow them to freely explore the experimental area for 10 min. After the experiment, the mice were returned to their cages. The entire process was recorded using a camera. The experimental apparatus was cleaned with 75% alcohol during the experimental intervals. After the experiment, the experimental video was analyzed using Etho Vision XT 14 software to determine the time the mice spent in the central and peripheral regions. According to the results, no significant difference was found between wild-type mice (WT) and homozygous mice (HO), indicating that the motor ability of DIR knock-in mice did not change (FIG. 6B).

### Example 2: Binding of ATP6VIB2 regulators to ATP6V1B2 and their biological activity

### 2.1 Binding of ATP6V1B2 regulators to ATP6VIB2

Expression plasmids of hATP6V1B2, QD202, site-mutated QD202(D15A), site-mutated QD202(V16A), site-mutated QD202(P13A), and site-mutated QD202(V14A) were constructed respectively. The specific sequences are shown in Table 1. The hATP6V1B2 was co-transfected into HEK 293 cells with the expression plasmids of QD202, site-mutated QD202(D15A), site-mutated QD202(V16A), site-mutated QD202(P13A) and site-mutated QD202(V14A), respectively, and cellular proteins were extracted 48 h after transfection.

**Table 1: Plasmid sequences**

| Plasmid name | Vector | CDS sequence |
|---|---|---|
| hATP6V1B2 | pcDNA3.1(+)-myc-his | atggcgctgcgggcgatgcgggggattgtcaacggggccgcacccgag ctacccgtgcccaccggtgggccggcggtgggagctcgggagcaggc gctggcagtcagtcggaactacctctcccagcctcgcctcacatacaaga cagtatctggagtcaatggtccactagtgatcttagatcatgttaagtttccca ggtatgctgaaattgtccatttgaccttaccggatggcacaaagagaagtg ggcaagttctggaagttagtggttccaaggcagtagttcaggtatttgaagg gacttcaggtatagatgctaagaaaacgtcctgtgagtttactggggatatt ctccgaacaccggtgtctgaggatatgcttggtcgggtattcaatggatcg ggaaaacccattgacagaggtcctgttgtactggccgaagacttccttgat atcatgggtcagccaatcaaccctcaatgtcgaatctacccagaggaaatg attcagactggcatttcggccatcgatgggatgaacagtattgctagggggg cagaaaattcctatcttctctgctgctgggctaccacacaatgagattgcag ctcagatctgtcgccaggctggtttggtaaagaaatccaaagatgtagtag actacagtgaggaaaattttgcaattgtatttgctgctatgggtgtaaacatg gaaactgcccggttcttcaaatctgactttgaagaaaatggctcaatggaca atgtctgcctctttttgaacttggctaatgacccaaccattgagcgaattatca ctcctcgcctggctctaaccacagctgaatttctggcgtaccaaTGtgag aaacatgtattggttattctaacagacatgagttcttatgctgaagcacttcga gaggtttcagcagccagggaagaggtacctggtcgacgaggttttccagg ttacatgtatacagatttagccacgatatatgaacgcgctgggcgagtgga agggagaaacggctcgattactcaaatccctattctaaccatgcctaatgat gatatcactcaccccatcccagacttgactggctacattacagaggggcag atctatgtggacagacagctgcacaacagacagatttatccacctatcaatg tgctgccctcactatcacggttaatgaagtctgctattggagaagggatgac caggaaggatcatgccgatgtatctaaccagctatatgcgtgctatgctatt ggaaaggatgtgcaagccatgaaagctgtcgttggagaagaagcccttac ctcagatgatcttctctacttggaatttctgcagaagtttgagaggaacttcat tgctcagggtccttacgaaaatcgcactgtctttgagactttggacattggct ggcagctactccgaatcttccccaaagaaatgctgaagagaatccctcag agcaccctcagcgaattttaccctcgagactctgcaaagcat (SEQ ID NO: 49) |
| QD202 | pEGFP-N3 | atgtacggcaggaagaagaggaggcagaggaggaggagccccgtgga cgtggtgtgcagc (SEQ ID NO: 73) |
| QD202(D15A) | pEGFP-N3 | atgtacggcaggaagaagaggaggcagaggaggaggagccccgtggc cgtggtgtgcagc (SEQ ID NO: 50) |
| QD202(V16A) | pEGFP-N3 | atgtacggcaggaagaagaggaggcagaggaggaggagccccgtgga cgccgtgtgcagc (SEQ ID NO: 51) |
| QD202(P13A) | pEGFP-N3 | atgtacggcaggaagaagaggaggcagaggaggaggagcgccgtgg acgtggtgtgcagc (SEQ ID NO: 52) |
| QD202(V14A) | pEGFP-N3 | atgtacggcaggaagaagaggaggcagaggaggaggagccccgccg acgtggtgtgcagc (SEQ ID NO: 53) |

Protein extraction was performed using Pierce RIPA lysis buffer. Taking cells in a 60 mm culture dish as an example, 500 µL of Pierce RIPA lysis buffer containing protease inhibitors was added. Cells were collected and transferred to a clean 1.5 mL EP tube using a cell scraper. After mixing for 1 h on a rotary mixer at 4°C, the cells were centrifuged at 13,000 rpm at 4°C for 10 min to remove cell debris. The hATP6V1B2 was equally divided into four aliquots, which were then mixed separately with the cell lysates transfected with QD202, site-mutated QD202(P13A), site-mutated QD202(V14A), site-mutated QD202(D15A), and site-mutated QD202(V16A), 30 µL of the supernatant was taken and mixed with 10 µL of 4× loading buffer, followed by boiling at 100°C for 5 min and storage at -20°C; 2 µL of the antibody (Anti-GFP, Roche, 11814460001) was added to the remaining 470 µL of the protein lysate, and the mixture was incubated overnight (about 16-18 h) on a rotary shaker at 4°C. 30 µL of Protein G Sepharose beads (Roche, 11243233001) were placed in an EP tube and washed three times with Pierce RIPA lysis buffer to eliminate interference from the original stock solution. The protein that had been incubated with the antibody overnight was added to the Sepharose beads, followed by mixing and incubation at 4°C for 2 h to ensure sufficient binding between the antibody and the protein. The mixture was centrifuged at 4000 rpm for 1 min. The supernatant was aspirated off, followed by the addition of 600 µL of Pierce RIPA lysis buffer. The tube was gently inverted, followed by centrifugation to collect the supernatant. The above steps were repeated three times to wash away non-specifically bound proteins. Finally, 30 µL of 1×loading buffer was added, the mixture was mixed well, heated at 60°C for 20 min to denature the protein, and then stored at -20°C. The protein was examined by western blot (Myc-Tag (9B11) Mouse mAb, CST, 2276S; Anti-GFP, Roche, 11814460001).

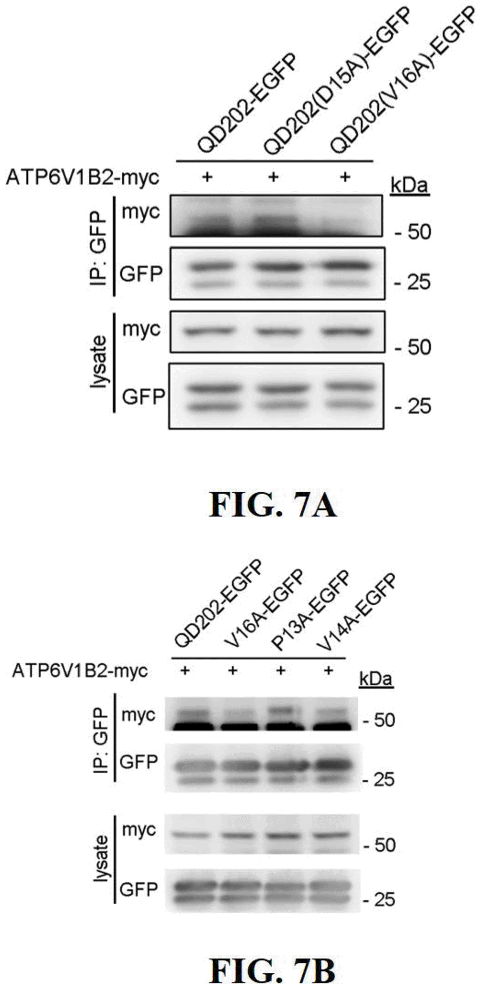

The hATP6V1B2 was co-transfected into the host cell line HEK 293 with the expression plasmids of QD202, site-mutated QD202(P13A), site-mutated QD202(V14A), site-mutated QD202(D15A) and site-mutated QD202(V16A), respectively. Co-immunoprecipitation assay demonstrated that the Sepharose beads could make Myc-tagged hATP6V1B2 bind to QD202; also bind to QD202(D15A) in which aspartic acid at position 15 was substituted with alanine, QD202(P13A) in which proline at position 13 was substituted with alanine and QD202(V14A) in which valine at position 14 was substituted with alanine. However, the binding to QD202(V16A) in which valine at position 16 was substituted with alanine was significantly reduced. It is therefore hypothesized that valine at position 16 of QD202 is one of the key amino acids for the binding between hATP6V1B2 and QD202, whereas proline at position 13, valine at position 14 and aspartic acid at position 15 can be replaced with alanine, as illustrated in FIG. 7A and FIG. 7B.

### 2.2 Biological activity of ATP6V1B2 regulators

This example demonstrates that the ATP6V1B2 regulators according to the present application can enhance the spontaneous release of excitatory synaptic transmitters by neurons and increase the release frequency of excitatory postsynaptic currents.

### Test steps:

Polypeptide QD202 was synthesized according to the amino acid sequence set forth in SEQ ID NO: 1.
Polypeptide QD202 N'-1 was synthesized according to the amino acid sequence set forth in SEQ ID NO: 2.
Polypeptide QD202 C'-1 was synthesized according to the amino acid sequence set forth in SEQ ID NO: 3.
Polypeptide QD202 C'-2 was synthesized according to the amino acid sequence set forth in SEQ ID NO: 4.
Polypeptide SS8 was synthesized according to the amino acid sequence set forth in SEQ ID NO: 54.

0.1-0.15 ml of 20% ethyl carbamate was injected into the abdominal cavity of a C57 mouse of 2 to 3 months old for anesthesia; a head was quickly cut, and brain tissues were stripped and put into artificial cerebrospinal fluid (ACSF) ice-water mixed liquid into which mixed gas (95%O₂ and 5%CO₂) was introduced in advance, and stood for 2 min; the ACSF included the following components: 11.7 mM of NaCl, 0.36 mM of KCl, 0.12 mM of NaH₂PO₄, 0.25 mM of CaCl₂, 0.12 mM of MgCl₂, 25 mM of NaHCO₃ and 11 mM of glucose. A hippocampus coronal brain section of 350 mm was cut by a vibration slicer. The hippocampus coronal brain section was placed into the ACSF at room temperature for more than 30 min for recovery, and then the brain section was put into a record slot; continuous perfusing was performed at a speed of 2 to 2.5 ml/min by the ACSF into which the mixed gas was introduced; blind whole-cell patch-clamp recording was performed on hippocampal CA1 pyramidal neurons under an Olympic BX51 upright microscope; whole-cell patch-clamp was performed by an Axon700B amplifier and a 1550B digital-to-analog converter; spontaneous excitatory postsynaptic current (sEPSC) of the pyramidal neuron was recorded by a voltage clamp under the clamp voltage of -70 mV; after recording for 5 min, synthetic polypeptides such as 5 µM QD202 or QD202 N'-1, C'-1, C'-2 and SS8 were applied for 5 min; and then perfusing and flushing were performed for 10 min by the ACSF. The recorded data were analyzed by Minianalysis software.

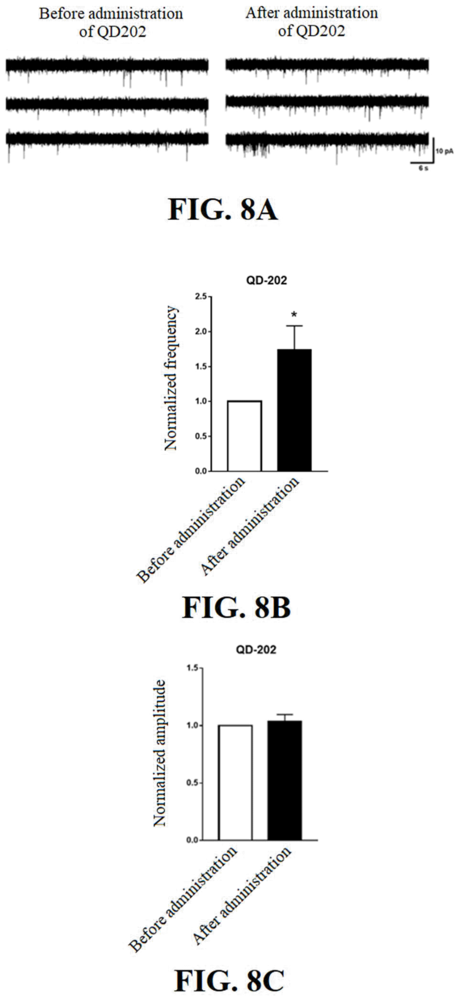

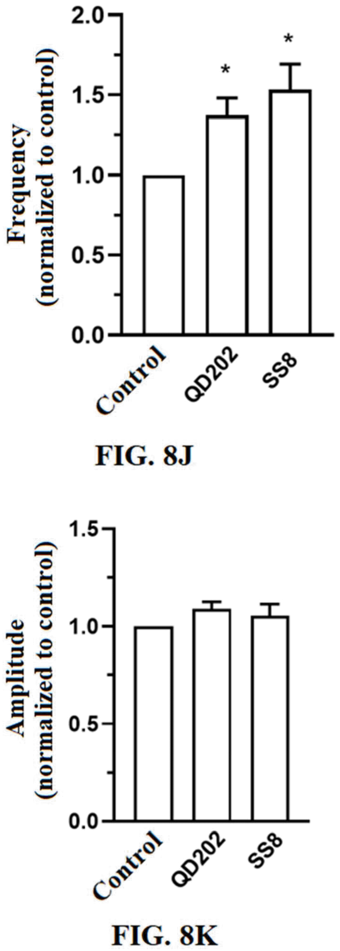

The results show that both QD202 and SS8 can increase the spontaneous excitatory synaptic transmitter release of hippocampal CA1 neurons, which is mainly reflected in increasing the release frequency of excitatory postsynaptic current, but has no influence on the excitatory postsynaptic current amplitude (see FIGS. 8A, 8B, 8C); furthermore, the effect of SS8 in increasing the frequency of sEPSC is stronger than that of QD202 (as shown in FIG. 8J and FIG. 8K).

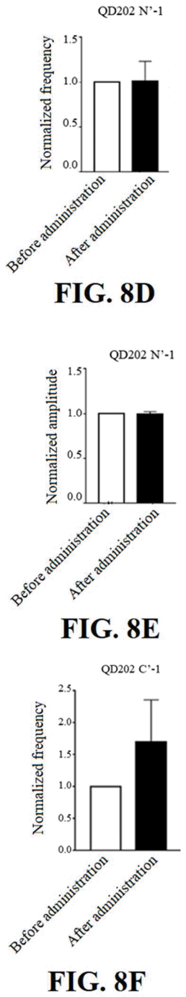

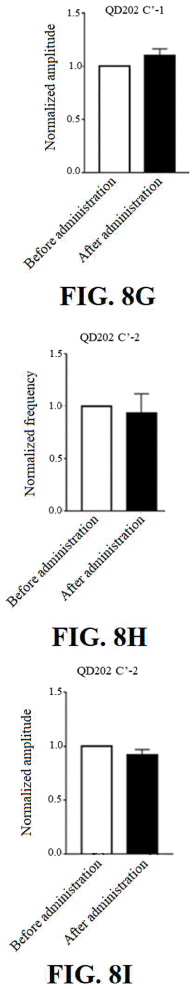

Compared with the effect of QD202 on excitatory synaptic transmission, the effect similar to that of QD202 cannot be observed by removing one amino acid at the N-terminus (see FIGS. 8D, 8E); the effect similar to that of QD202 can still be observed by removing one amino acid at the C-terminus (see FIGS. 8F, 8G); and the effect similar to that of QD202 cannot be observed by removing two amino acids at the C-terminus (see FIGS. 8H, 8I).

### 2.3 The improvement effect of ATP6V1B2 regulator on learning ability

### Novel object recognition test

For the novel object recognition, refer to the behavioral experiment protocol recorded in Leger, M., et al. Object recognition test in mice. Nat Protoc. 8, 2531-2537 (2013), where an open field box (40×40×35 cm) made of blue opaque plastic was used to conduct the novel object recognition test. On the first day, mice were put into the open field box to adapt for 10 min. On the second day, each mouse was lightly put in the center of the box, two similar objects (a battery I) were put in the central area and freely explore for 10 min, and then the mice were sent back to feeding cages. After 3 h, the mice were put back to the box again (one battery was replaced with a doll toy which was 10 cm high) for 10 min to carry out a memory retention test. A video was analyzed by Etho Vision XT 14 software, and the time for exploring novel/familiar objects by the mice was recorded. The discrimination index was computed as (Tnovel-Tfamliar)/(Tnovel+Tfamiliar).

### Morris water maze test

Morris water maze referred to the test solutions recorded in Qing-Feng Wu et al., Fibroblast growth factor 13 is a microtubule-stabilizing protein modulating neuronal polarization and migration. Cell. 149, 1549-1564 (2012). The test was carried out in a circular pool filled with water (120 cm in diameter, 30 cm in depth, titanium dioxide was added to make opaque, kept temperature at 21±1°C) in a room with a fixed environment. The test was divided into an acclimation period (1 day), a training period (5-6 days) and a testing period (1 day). Acclimation period: the platform was placed 0.5 cm above the water surface, and the mice were guided to the platform. Training period: the platform was placed 0.5 cm underwater, and the mice were trained to find the platform. The timing was stopped when the mice reached the platform, and if the mice did not reach the platform within 1 min, the mice were guided to the platform and allowed to stay on the platform for 30 s. Training were performed 4 times a day(8:00-16:00), the mice were dropped from different water points each time, each training was performed at interval of at least 30 min, a total of 6 days, and the order of water points was different every day. Testing period: test was performed 24 h after the end of the training day. The platform was removed, the mice entered the water from untrained water entry points, and videoed for 1 min. The footage was analyzed with Etho Vision XT 14 software, and the recording parameters include the escape latency of entering the target quadrant, the number of platform crossings, and the dwell time in the target quadrant.

### 2.3.1 Oral administration of polypeptide

Ten-month-old APP/PS1 transgenic mice (purchased from MODEL ORGANISMS) were selected and equally divided into three groups; the mice were administered intragastrically with PBS buffer (group 2), QD202 (group 3, QD202 dosage: 6.25 mg/kg, with the concentration of QD202 being 500 µM) and memantine (group 4, memantine dosage: 1.25 mg/kg, with the concentration of memantine being 1 mM), respectively. Ten-month-old C57BL/6 mice without any intragastric administration served as the control (group 1).

Behavioral test was performed on the four groups of mice.

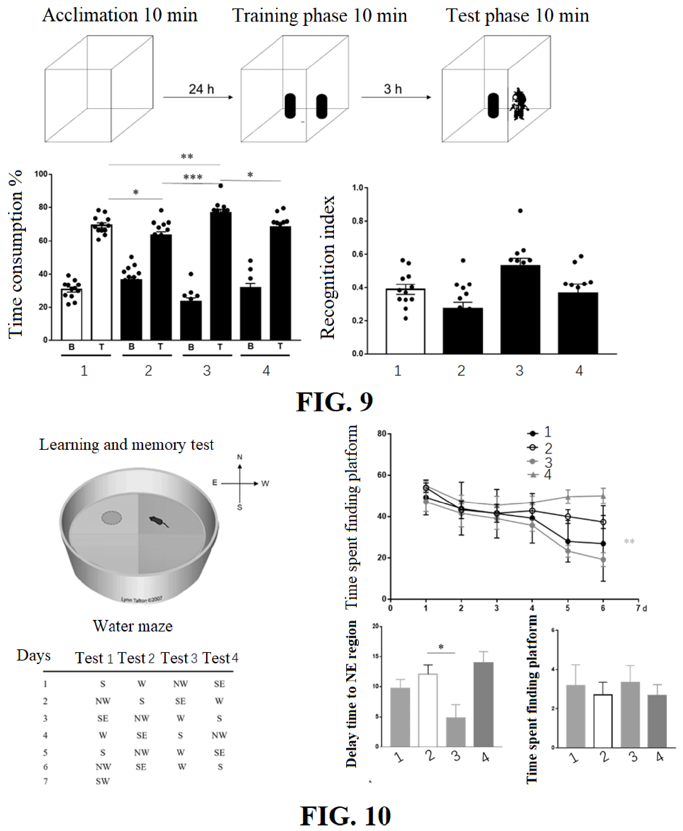

The results show that after QD202 was continuously administered in an intragastric manner for 1 week, the APP/PS1 transgenic AD mice show obvious improvement of learning and cognitive functions in the novel object recognition test (shown in FIG. 9) and the water maze behavior (shown in FIG. 10).

### 2.3.2 Intravenous injection of polypeptide

APP/PS1 transgenic mice of 10 months old (purchased from MODEL ORGANISMS) were selected and equally divided into two groups; the mice were administered with a PBS buffer solution (group 2), and QD202 (group 3, QD202 dosage 1.25 mg/kg, the concentration of QD202 being 500 µM) through intravenous injection; and C57 BL/6 mice of 10 months old without taking any reagent through intravenous injection served as the control (group 1).

Behavioral test was performed on the four groups of mice.

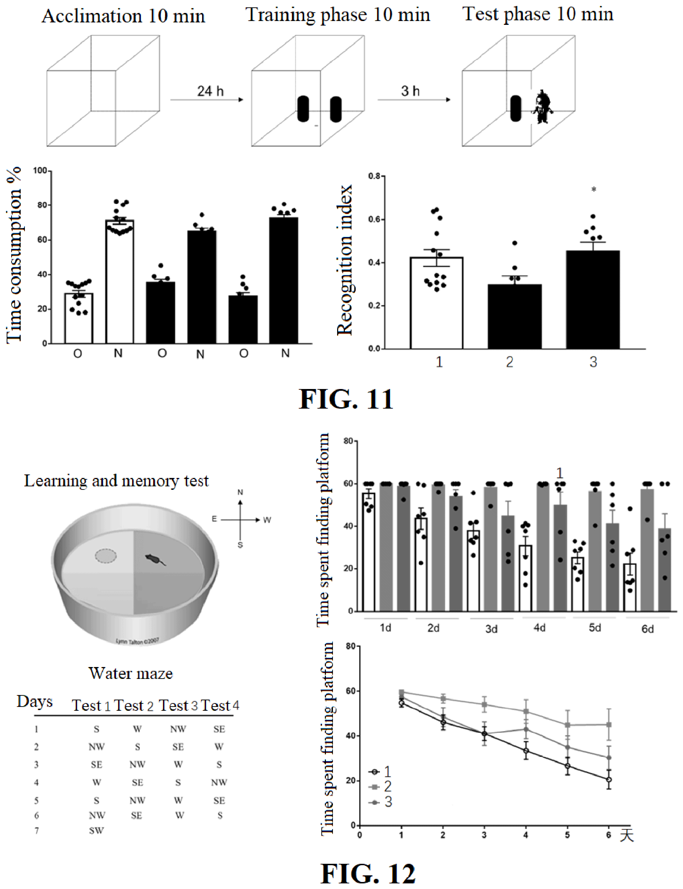

The results show that after QD202 is continuously injected in an intravenous manner for 1 week, the APP/PS1 transgenic AD mice show obvious improvement on learning and cognitive functions in novel object recognition (shown in FIG. 11) and water maze behavior (shown in FIG. 12) detection.

Therefore, QD202 has an obvious effect of improving the learning ability, particularly the memory ability and cognitive ability.

### Example 3: Increased DIR expression after MCAO (Middle Cerebral Artery Occlusion) surgery in DIR-Ki mice

### (1) Materials and methods:

MCAO surgery was performed on the left side of DIR-Ki mice. Blood was collected from the cardiac apex of both DIR-Ki mice that did not undergo surgery and those that underwent surgery. Cerebral cortex and hippocampal tissues were collected from both the ipsilateral and contralateral sides. Cerebral cortex and hippocampal tissues were also collected from WT mice that did not undergo surgery. 10 mg of tissue was added to 100 µL of tissue RIPA lysis buffer containing protease inhibitors, and a steel ball was added. The tissue was ground at 60 Hz for 1 min in a tissue homogenizer, centrifuged at 13,000 rpm at 4°C for 10 min, the supernatant was collected, 4× loading buffer was added and the mixture was denatured at 100°C for 5 min. Blood samples taken from the apex of the heart were placed in centrifuge tubes treated with the anticoagulant EDTA·2Na, centrifuged at 1,200×g for 10 min at 4°C, the supernatant plasma was collected, 4×loading buffer was added, the mixture was denatured at 100°C for 5 min and then diluted 10 times with 1×loading buffer. The sample was stored at -20°C. Proteins were detected by western blot (Anti-DIR, GL Biochem custom; HIF1A, Sigma, SAB2702132; β-Tubulin, Sigma, T5293).

### (2) Experimental results:

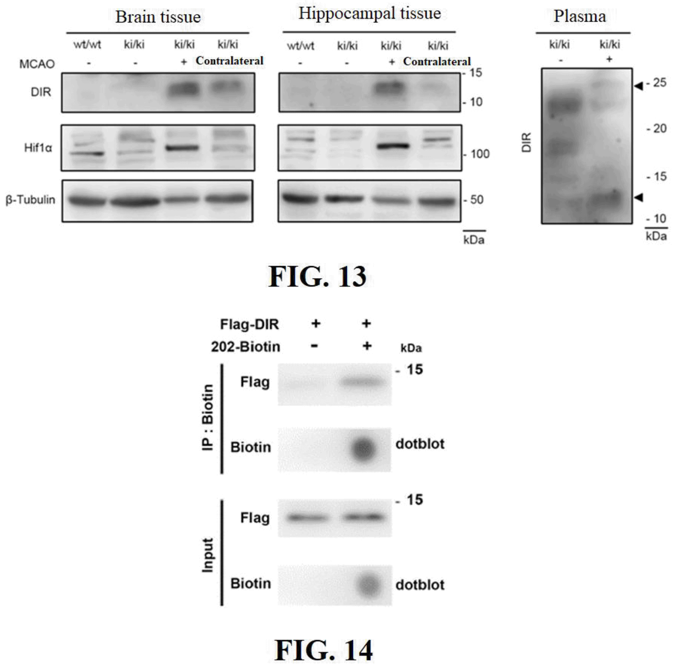

Western blot experiments on tissues and serum (FIG. 13) showed that the expression level of DIR in the brain tissue and hippocampus of DIR-Ki mice was significantly increased on the surgical side of MCAO model mice, while the expression level of DIR on the contralateral side of MCAO model mice was slightly increased, and the expression level of DIR in plasma was significantly increased in MCAO model mice. In summary, MCAO increases the expression of DIR in brain tissue and plasma.

### Example 4: Binding of DIR to DIR regulators and/or ATP6V1B2 regulators

This example demonstrates that the DIR regulators according to the present application and/or the ATP6V1B2 regulators according to the present application can bind to DIR.

### (1) Materials and methods:

The DIR expression plasmid was constructed, and the specific sequence is shown in Table 1. The Flag-His-DIR expression plasmid was transfected into HEK 293, and cell proteins were extracted 48 h after transfection.

**Table 2: Plasmid sequences**

| Plasmid name | Vector | CDS sequence |
|---|---|---|
| FH-DIR | pCMV-Flag-His | ATGGTTGCAACTGGCAGTTTGAGCAG CAAGAACCCGGCCAGCATTTCAGAAT TGCTGGACTGTGGCTATCACCCAGAG AGCCTGCTAAGTGGTGAGTGTCCCCG CTGTCCACCCTTCTCCCCTCCCAAGG TCGGTGCCTCTGGATCTATTGGGTGG GCGGGCTTTCGACTTCCTCCCCTACC TCTATGTAGGAGGTGCCACAAAATCC ATCTGAGGAGACTCCTTTCCAAATTT TCTAACATCTTCTCTCCATAA |

Protein extraction was performed using Pierce RIPA lysis buffer, 1000 µL of Pierce RIPA lysis buffer containing protease inhibitors was added. Cells were collected and transferred to a clean 1.5 mL EP tube using a cell scraper. After mixing for 1 h on a rotary mixer at 4°C, the cells were centrifuged at 13,000 rpm at 4°C for 10 min to remove cell debris. The supernatant of the cell lysate was divided into two aliquots, each 500 µL, and 10 µL of QD202-Biotin peptide at a concentration of 1 mM was added to one aliquot. From each of the two aliquots, 30 µL of the supernatant was taken and mixed with 10 µL of 4× loading buffer. The mixture was boiled at 100°C for 5 min and then stored frozen at -20°C. For the remaining 470 µL of protein lysate, 2 µL of the antibody (Anti-Biotin, Vector, sp-3000) was added; the mixture was incubated overnight (about 16-18 h) on a rotary shaker at 4°C. 30 µL of Protein G Sepharose beads (Roche, 11243233001) were placed in an EP tube and washed three times with Pierce RIPA lysis buffer. The protein that had been incubated with the antibody overnight was added to the Sepharose beads, followed by mixing and incubation at 4°C for 2 h to ensure sufficient binding between the antibody and the protein. The mixture was centrifuged at 4000 rpm for 1 min. The supernatant was aspirated off, followed by the addition of 600 µL of Pierce RIPA lysis buffer. The tube was gently inverted, followed by centrifugation to collect the supernatant. The above steps were repeated three times to wash away non-specifically bound proteins. Finally, 35 µL of 1×loading buffer was added, the mixture was mixed well, heated at 60°C for 20 min to denature the protein, and then stored at - 20°C. The protein was detected by Western blot and dot blot methods (DYKDDDDK-Tag (3B9) mAb, Abmart, M20008; Anti-Biotin, Vector, sp-3000).

### (2) Experimental results:

Co-immunoprecipitation assays demonstrated that QD202 can bind to the DIR protein (FIG. 14). This demonstrates that the ATP6V1B2 regulator according to the present application can bind to DIR. For example, the ATP6V1B2 regulators having the sequences according to the present application can bind to DIR.

### Example 5 The DIR regulators according to the present application and/or the ATP6V1B2 regulators according to the present application improve the cognitive and memory decline in DIR transgenic mice

### (1) Materials and methods:

Thirty homozygous DIR-Ki mice were divided into three groups: the control group (HO+PBS), the QD202 1 mg/kg group (HO+QD202 1 mpk), and the QD202 5 mg/kg group (HO+QD202 5 mpk). After 7 consecutive days of intravenous administration in the QD202-treated groups, the novel object recognition test was initiated.

### Experimental setup:

Square PVC plastic box (dimensions: 25 cm × 25 cm × 30 cm), two identical size 1 batteries, one plastic toy of the same height as the batteries, experimental observation box, camera, and video recording software

### Experimental method and steps:

On the day of the experiment, the mice were allowed to acclimatize to the experimental room for 30 min in advance. The test was divided into an acclimation period and a testing period.

Familiarization phase: Two identical batteries were placed 5 cm away from a box wall, with a 5 cm gap between the two batteries. The mice were placed in the center of the area and allowed to explore freely for 10 min. The testing period test was conducted 3-4 h after the familiarization phase ended.

Testing period: A toy with size and height similar to those of the battery was randomly used to replace one battery and placed in the same position as the original battery. The mice were placed in the center of the area and allowed to explore freely for 10 min. After the experiment, the mice were returned to their cages. The entire process was recorded using a camera. The experimental apparatus was cleaned with 75% alcohol during the experimental intervals. After the experiment, the time taken for the mice to explore novel and familiar objects in the video was counted manually, and the time recognition index of the novel objects was calculated. When a mouse's nose is within 2 cm of an object, smelling or touching the object is considered exploratory behavior; biting or climbing on the object is not considered exploratory behavior. The recognition index of mice for novel objects = (time spent exploring novel objects - time spent exploring familiar objects) / (time spent exploring novel objects + time spent exploring familiar objects). The higher the recognition index, the longer the animal takes to explore novel objects. Data from mice whose total exploration time was less than 15 s will be excluded. The experimenters and video analysts were kept double-blind.

### (2) Experimental results:

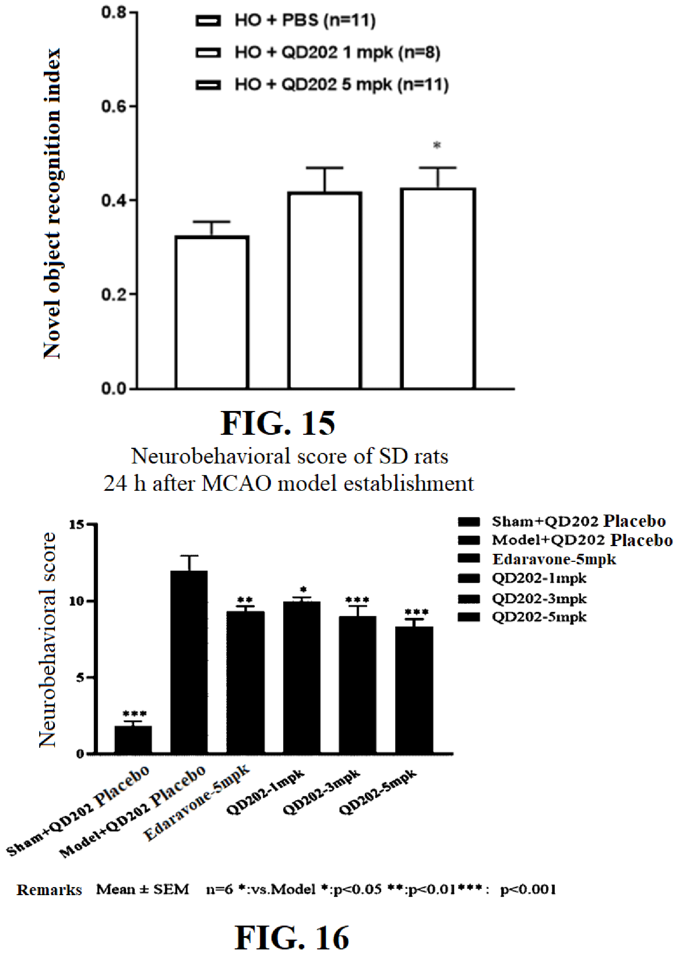

Experimental results showed that intravenous administration of QD202 at 1.0 mg/kg and 5.0 mg/kg for 7 consecutive days significantly improved the novel object recognition index in DIR-Ki mice (see FIG. 15). This demonstrates that the ATP6V1B2 regulator according to the present application can serve as a DIR regulator. For example, an ATP6V1B2 regulator having the sequence according to the present application can serve as a DIR regulator, thereby enabling its use in treating diseases associated with the DIR, the intron-retained splicing product of DNA damage-induced transcript 4-like transcripts, and/or functional fragments thereof.

### Example 6 Effects of the DIR regulator and/or ATP6V1B2 regulator according to the present application on postoperative function and cerebral infarction extent in rats after MCAO surgery

This example illustrates that the DIR regulator and/or ATP6V1B2 regulator according to the present application can improve the neurological and behavioral abilities of rats after MCAO surgery, whether administered intravenously or orally. For example, they can improve the neurological and behavioral scores of rats after MCAO surgery and reduce the extent of cerebral infarction. Middle cerebral artery occlusion (MCAO) is a commonly used stroke model in mice and rats. Edaravone is a drug that is widely used in the treatment of acute ischemic stroke. This example illustrates that the DIR regulator and/or ATP6V1B2 regulator according to the present application have therapeutic efficacy against stroke, with such efficacy being superior to that of edaravone.

Experimental protocol: Male SD rats (260-280 g) were randomly assigned to the following groups: Sham + QD202 placebo group (n=6), Model + QD202 placebo group (n=6), positive control group (Edaravone-5 mpk, n=6), QD202-1 mpk group (n=6), QD202-3 mpk group (n=6), QD202-5 mpk group (n=6). The sham group served as the sham-operated group, while rats in the model group were subjected to Middle Cerebral Artery Occlusion (MCAO) for model establishment. For the edaravone-treated group, administration was initiated 3 days prior to surgery, once daily; on the day of surgery, a total of 6 doses were given (30 min before surgery, 30 min after surgery, and 60 min after surgery). Rats in the QD202 groups received a single dose at 30 min after surgery. At 24 h postoperatively, neurological function was scored according to Table 3. Subsequently, the whole brain tissues of the rats were harvested for TTC staining to evaluate the infarct volume.

The detailed procedure for MCAO model establishment is as follows: (1) The animals were anesthetized via inhalation, followed by cervical hair removal and disinfection with povidone-iodine; (2) The animals were fixed in a supine position, with the neck area exposed; (3) A midline incision was made on the neck using surgical scissors, and blunt dissection was performed to expose the right common carotid artery, internal carotid artery, and external carotid artery, the common carotid artery was clamped with an arterial clip, a dead knot was tied on the external carotid artery close to the cranial side using 5-0 suture, and a slip knot was made on the external carotid artery near the bifurcation of the common carotid artery; (4) The internal carotid artery was clamped with an arterial clip, the external carotid artery was transected with microscissors between the two knots, a monofilament suture was inserted into the internal carotid artery through the transected end, and then the arterial clip on the internal carotid artery was released, the suture was advanced into the middle cerebral artery until the tip of the suture bent, the slip knot at the common carotid artery bifurcation was tightened, and the occlusion duration was set at 60 min; (5) After occlusion, the slip knot was slightly loosened, the monofilament suture was withdrawn, the slip knot on the external carotid artery was immediately tied off tightly, the arterial clip on the common carotid artery was removed, the cervical incision was sutured, and the surgical site was disinfected with povidone-iodine; (6) an appropriate amount of glucose solution was administered via injection postoperatively to maintain the basic viability of the animals.

**Table 3 Neurological function scoring**

| **Test items** | **Behavioral performance** | **Score** |
|---|---|---|
| | Irritability/piloerection | 1 |
| | Immobility/staring | 1 |
| General behavior | Epileptic seizure/myospasm/tremor | 1 |
| | Forelimb flexion | 1 |
| | Hindlimb flexion | 1 |
| | Head deviation from the vertical axis within 30 s >10° | 1 |
| | Inability to walk in a straight line | 1 |
| Motor | Circling around a fixed point | 2 |
| | Circling (>50%) | 3 |
| | Falling down | 4 |
| Sensory function | Absence of corneal reflex | 1 |
| | Absence of pinna reflex | 1 |
| | Absence of startle reflex | 1 |
| Proprioceptive sensory function | Clinging tightly to the edge of the balance beam | 1 |
| (On the balance beam) | Clinging tightly to the balance beam with one limb slipping off | 2 |
| | Clinging tightly to the balance beam with limbs hanging down or rotating (>60 s) | 3 |
| | Attempting to balance on the balance beam but falling off (5 s) | 4 |
| | Unable to maintain balance and falling off directly | 5 |
| Total score | | 18 |
| Remarks | 1-6 points: mild injury; 7-12 points: moderate injury; 13-18 points: severe injury. | |

Neurobehavioral scoring results: As shown in FIG. 16, compared with the Model+QD202 placebo group, the neurobehavioral score of the Sham+QD202 placebo group was lower than that of the Model+QD202 placebo group, which was statistically significant (p<0.001). Compared with the Model + QD202 placebo group, the neurobehavioral score of the positive control group (Edaravone-5 mpk) was significantly lower than that of the Model + QD202 placebo group with a statistically significant difference (p<0.01), the neurobehavioral score of the QD202-1 mpk group was lower than that of the Model + QD202 placebo group with a statistical difference (p<0.05), and the neurobehavioral scores of both the QD202-3 mpk group and the QD202-5 mpk group were lower than that of the Model + QD202 placebo group with extremely significant statistical differences (p<0.001).

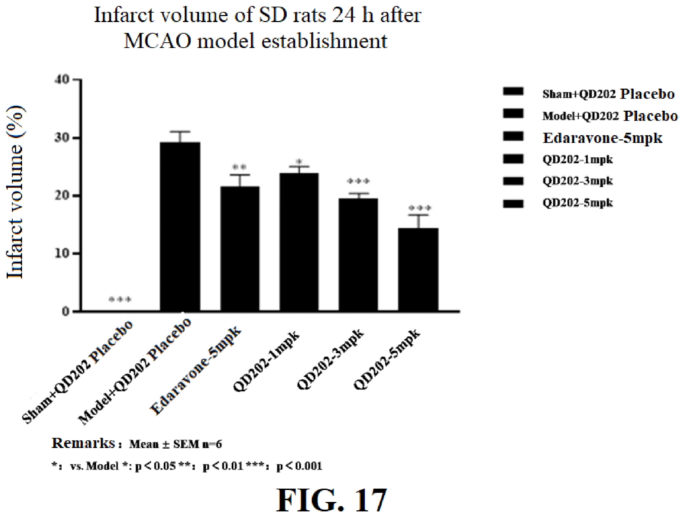

Results of TTC Staining: According to the results shown in FIG. 17, compared with the Model + QD202 placebo group, the Sham + QD202 placebo group showed no cerebral infarct volume, with an extremely significant statistical difference (p<0.001); compared with the Model + QD202 placebo group, the infarct volume of the positive control group (Edaravone-5 mpk) was significantly lower than that of the Model + QD202 placebo group, with a statistically significant difference (p<0.01); the infarct volume of the QD202-1 mpk group was lower than that of the Model + QD202 placebo group, with a statistical difference (p<0.05); the infarct volumes of both the QD202-3 mpk group and the QD202-5 mpk group were lower than that of the Model + QD202 placebo group, with extremely significant statistical differences (p<0.001).

### Example 7: The effect of the DIR regulator according to the present application and/or the ATP6V1B2 regulator according to the present application on scavenging ROS in an in vitro glucose-oxygen deprivation cell model.

This example illustrates that the DIR regulator and/or ATP6V1B2 regulator according to the present application exert free radical-scavenging and antioxidant effects, thereby conferring neuroprotective effects and therapeutic efficacy against stroke, with such effects being superior to those of edaravone.

Currently, based on the pathogenesis and progression of ischemic stroke, the most widely used in vitro model is the neural cell oxygen-glucose deprivation (OGD) model, which is primarily designed to simulate the development of ischemic injury in vivo. By subjecting cells to glucose-free and oxygen-depleted treatment, this model mimics the apoptosis induced by insufficient energy supply in brain tissue due to cerebral infarction.

Cell culture: Mouse hippocampal HT22 cells were cultured in DMEM and 10% serum at 37°C in a 5% CO2 incubator.

Oxygen-glucose deprivation (OGD) cell model: HT22 cells were seeded at a density of about 1 ×10⁵ into 96-well plates and cultured for 24 h in a 37°C incubator with 5% CO₂/95% air in DMEM and 10% serum. After placing HT22 cells in glucose-free medium, they were transferred to a sealed container containing a 5% CO₂/95% N₂ mixture and incubated at 37°C for 16 h. The glucose-free medium was then replaced with a normal medium containing glucose, and the mixture was incubated for 24 h in a 37°C incubator containing 5% CO₂/95% air for reoxygenation.

Experimental grouping: Normally cultured HT22 cells were used as the control group. HT22 cells subjected to OGD were reoxygenated in normal culture medium and treated separately with vehicle, QD202 (0.1 µM, 1 µM, 5 µM), and the ROS scavenger edaravone (200 µM). After 24 h of reoxygenation, quantitative detection of ROS was performed.

Quantitative detection of ROS: Reactive oxygen species were detected using the Beyotime reactive oxygen species detection kit and the fluorescent probe DCFH-DA. DCFH-DA was diluted 1:1000 with serum-free culture medium to a final concentration of 10 µM. The culture medium was removed and the diluted DCFH-DA was added. The cells were then incubated in a 37°C cell culture incubator for 20 min. The cells were washed three times with serum-free cell culture medium to thoroughly remove any DCFH-DA that had not entered the cells. Observation was performed under a fluorescence microscope, followed by quantification of the average fluorescence intensity using Image J software.

Data analysis: Three parallel experiments were performed. The average fluorescence intensity of the OGD plus vehicle group was set as the standard value of 100% for normalization of the fluorescence intensity of cells in each group. Data were expressed as mean ± standard deviation, and non-parametric tests were performed on the data of each group using One-Way ANOVA.

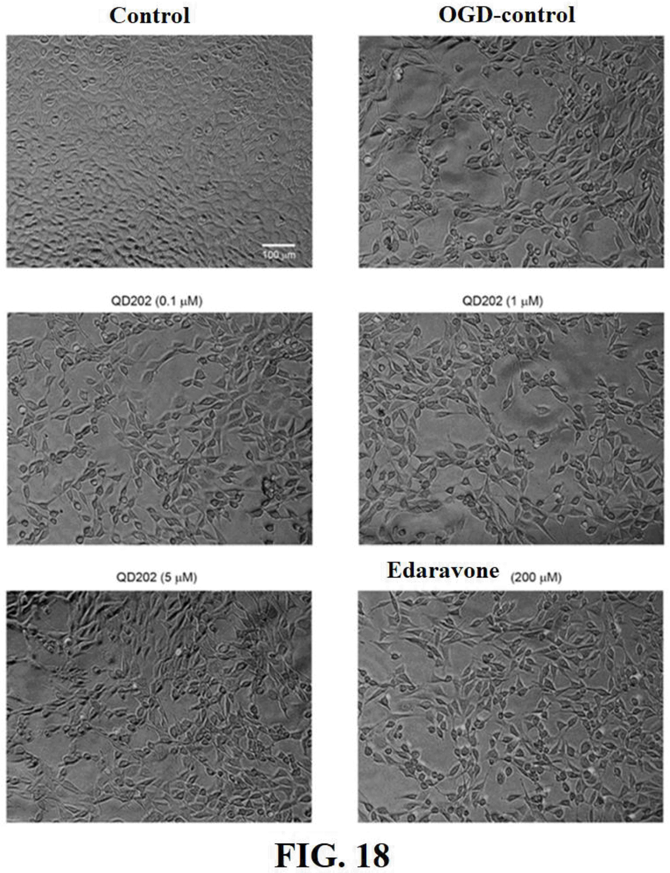

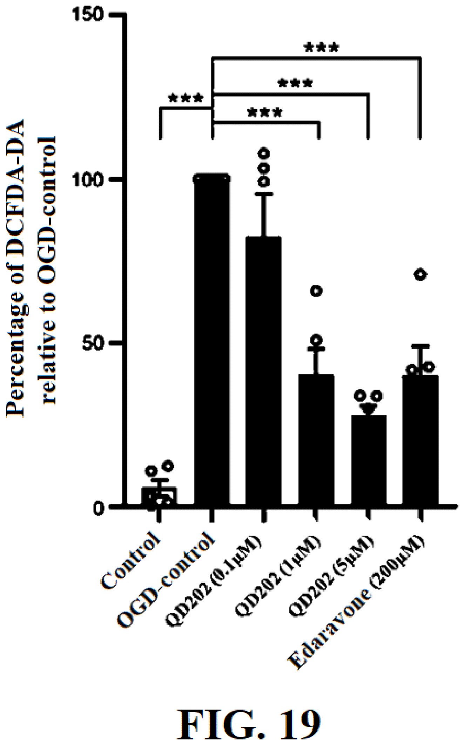

Results: Compared with normally cultured HT22 cells, the ROS content in the vehicle-treated group increased significantly after OGD treatment. In contrast, QD202 at concentrations of 1 µM and 5 µM both markedly inhibited the elevation of ROS levels in HT22 cells, with inhibition rates of 60.63±8.27% and 70.09±2.84%, respectively. Treatment with 0.1 µM QD202 slightly inhibited ROS production, with an inhibition rate of 17.55±12.95%. In addition, edaravone (200 µM), the positive control drug, also significantly inhibited the OGD-induced increase in ROS, with an inhibition rate of 63.79±10.9% (see FIG. 18 and 19).

## Claims

1. Use of a proton pump regulator in the preparation of a reagent for preventing and/or treating a disease, wherein the proton pump regulator regulates ATP6V1B2 and/or a functionally active fragment thereof, and the disease comprises a stroke.

2. Use of a DIR regulator in the preparation of a reagent for preventing and/or treating a disease, wherein the DIR regulator regulates the intron-retained splicing product DIR of DNA damage-inducible transcript 4-like transcripts and/or functional fragments thereof, and the disease comprises a stroke.

3. The use according to any one of claims 1-2, wherein the stroke includes ischemic stroke and/or hemorrhagic stroke.

4. The use according to any one of claims 1-3, wherein the ischemic stroke includes cerebral infarction.

5. The use according to claim 4, wherein the cerebral infarction includes lacunar infarction, ischemic cerebral infarction and/or hemorrhagic infarction.

6. The use according to any one of claims 3-5, wherein the ischemic stroke is caused by factors including: thrombus, embolism and/or hypotension.

7. The use according to claim 6, wherein the thrombus is caused by factors including: atherosclerosis, aneurysm, vascular malformation, arteritis and/or vasospasm.

8. The use according to any one of claims 3-7, wherein the ischemic stroke includes large artery atherosclerosis stroke, cardiogenic embolic stroke, small artery occlusion stroke, stroke of other determined etiology and/or stroke of undetermined etiology.

9. The use according to any one of claims 3-8, wherein the hemorrhagic stroke includes brain parenchymal hemorrhage, intraventricular hemorrhage and/or subarachnoid hemorrhage.

10. The use according to any one of claims 3-9, wherein the hemorrhagic stroke includes primary cerebral hemorrhage and/or secondary cerebral hemorrhage.

11. The use according to any one of claims 3-10, wherein the hemorrhagic stroke includes aneurysmal subarachnoid hemorrhage.

12. The use according to any one of claims 3-11, wherein the hemorrhagic stroke is caused by factors including: vascular malformation, aneurysm, blood disease, cerebral amyloid angiopathy, moyamoya disease, cerebral arteritis, anticoagulation or thrombolytic therapy and/or tumor stroke.

13. The use according to any one of claims 1-12, wherein the stroke includes stroke-induced impairments.

14. The use according to claim 13, wherein the impairment includes impairments observed through imaging.

15. The use according to claim 14, wherein the impairment observed through imaging includes intracerebral hematoma, intraventricular hemorrhage and/or subarachnoid hemorrhage.

16. The use according to any one of claims 14-15, wherein the impairment observed through imaging includes edema, hematoma and/or mass effect.

17. The use according to any one of claims 14-16, wherein the impairment observed through imaging includes dense artery sign, insular ribbon sign, blurred or decreased density of the basal ganglia, occlusion of small penetrating cerebral arteries, edema and/or mass effect.

18. The use according to claim 13, wherein the impairment includes impairment of learning ability, and the learning ability comprises cognitive ability, motor ability, memory ability, and/or spatial exploration ability.

19. The use according to claim 18, wherein the learning ability is measured by implementing a test selected from the group consisting of: novel object recognition test and water maze test.

20. The use according to claim 19, wherein the novel object recognition test is used for evaluating cognitive ability, motor ability and/or spatial exploration ability.

21. The use according to claim 19, wherein the water maze test is used for evaluating memory ability, motor ability and/or spatial exploration ability.

22. The use according to any one of claims 1-21, wherein the ATP6V1B2 and/or functionally active fragment thereof is each derived from mammals.

23. The use according to any one of claims 1-22, wherein the ATP6V1B2 and/or functionally active fragment thereof is each derived from humans or mice.

24. The use according to any one of claims 1-23, wherein the ATP6V1B2 comprises the amino acid sequence set forth in SEQ ID NO: 8 or 16.

25. The use according to any one of claims 1-24, wherein the functionally active fragment of the ATP6V1B2 is capable of specifically binding to the amino acid sequence set forth in SEQ ID NO: 5.

26. The use according to any one of claims 1-25, wherein the functionally active fragment of the ATP6V1B2 is capable of specifically binding to the amino acid sequence set forth in SEQ ID NO: 1.

27. The use according to any one of claims 1-26, wherein the functionally active fragment of the ATP6V1B2 comprises at least a portion of the amino acid sequence from position 288 to position 512 of human ATP6V1B2 protein.

28. The use according to any one of claims 1-27, wherein the functionally active fragment of the ATP6V1B2 comprises at least a portion of the amino acid sequence from position 288 to position 512 of mouse ATP6V1B2 protein.

29. The use according to any one of claims 1-28, wherein the functionally active fragment of the ATP6V1B2 comprises the amino acid sequence set forth in any one of SEQ ID NOs: 10-11.

30. The use according to any one of claims 1-29, wherein the ATP6V1B2 comprises the amino acid sequence encoded by the nucleotide sequence set forth in SEQ ID NO: 9 or 17.

31. The use according to any one of claims 1-30, wherein the proton pump regulator is capable of regulating the activity and/or function of the proton pump.

32. The use according to any one of claims 1-31, wherein the proton pump regulator is capable of improving the expression level and/or activity of proton pump-associated proteins in a subject.

33. The use according to any one of claims 1-32, wherein the proton pump regulator is capable of regulating the expression level and/or activity of the ATP6V1B2.

34. The use according to any one of claims 1-33, wherein the proton pump regulator is capable of improving the expression level and/or activity of the ATP6V1B2 in a subject, and wherein the improvement comprises, compared to the original expression level and/or activity of the ATP6V1B2 in the subject, improving the expression level and/or activity of the ATP6V1B2 by at least about 10%.

35. The use according to any one of claims 31-34, wherein the expression level of the ATP6V1B2 comprises the expression level of the ATP6V1B2 gene, the transcription level of ATP6V1B2 gene, and/or the expression level of ATP6V1B2 protein.

36. The use according to any one of claims 1-35, wherein the proton pump regulator is capable of increasing the release of neurotransmitters at neuronal synapses, and the increase includes an increase of at least about 10% compared to the original level of neurotransmitter release at neuronal synapses in the subject.

37. The use according to any one of claims 1-36, wherein the proton pump regulator increases the release frequency of excitatory postsynaptic currents, and the increase includes an increase of at least about 10% compared to the original level of release frequency of excitatory postsynaptic currents in the subject.

38. The use according to any one of claims 1-37, wherein the DIR regulator causes a reduction of the expression level and/or biological activity of the DIR and/or functional fragment thereof in the subject.

39. The use according to claim 38, wherein the reduction comprises a decrease of at least about 10% in the expression level and/or biological activity of the DIR and/or functional fragment thereof compared to the original expression level and/or biological activity of the DIR and/or functional fragment thereof in the subject.

40. The use according to any one of claims 38-39, wherein the expression level includes the expression level of a gene encoding the DIR/or functional fragment thereof, the transcription level of the gene encoding the DIR/or functional fragment thereof, and/or the expression level of the DIR/or functional fragment thereof.

41. The use according to any one of claims 1-40, wherein the functional fragment of the DIR retains at least a portion of the biological activity of the DIR.

42. The use according to claim 41, wherein the biological activity includes influencing the excitability of neurons and/or inhibiting the activity of neurons.

43. The use according to any one of claims 41-42, wherein the biological activity includes the ability to reduce the frequency of excitatory postsynaptic currents (EPSCs), and/or the ability to reduce the amplitude of EPSCs.

44. The use according to claim 43, wherein the reduction comprises, by administering the DIR and/or functional fragment thereof and/or a nucleic acid encoding the DIR and/or functional fragment thereof, reducing the frequency of excitatory postsynaptic currents (EPSCs) in the subject, and/or reducing the amplitude of EPSCs in the subject, compared to the original biological activity of the DIR and/or functional fragment thereof in the subject.

45. The use according to any one of claims 41-44, wherein the biological activity includes influencing cognitive abilities.

46. The use according to any one of claims 41-45, wherein the biological activity includes participation in signaling pathways associated with Aβ deposition, and/or participation in signaling pathways associated with Tau tangle formation.

47. The use according to any one of claims 41-46, wherein the biological activity includes inducing Aβ deposition and/or amyloid plaque formation through gelsolin.

48. The use according to claim 47, wherein the DIR/or functional fragment thereof induces Aβ deposition and/or amyloid plaque formation by binding to gelsolin.

49. The use according to any one of claims 1-48, wherein the expression levels of the DIR and/or functional fragment thereof are positively correlated with the expression levels of Aβ.

50. The use according to any one of claims 38-49, wherein the reduction comprises, by administering the DIR and/or functional fragment thereof and/or a nucleic acid encoding the DIR and/or functional fragment thereof, reducing the cognitive ability of the subject, compared to the original biological activity of the DIR and/or functional fragment thereof in the subject.

51. The use according to any one of claims 1-50, wherein the DIR/or functional fragment thereof is derived from mammals.

52. The use according to any one of claims 1-51, wherein the DIR/or functional fragment thereof is derived from primates.

53. The use according to any one of claims 1-52, wherein the DIR/or functional fragment thereof is derived from humans.

54. The use according to any one of claims 1-53, wherein the DIR comprises the amino acid sequence set forth in SEQ ID NO: 56.

55. The use according to any one of claims 1-54, wherein the functional fragment of the DIR comprises an amino acid sequence encoded by the retained intron in *DDIT4L.*

56. The use according to any one of claims 1-55, wherein the functional fragment of the DIR comprises the amino acid sequence set forth in any one of SEQ ID NOs: 59-60.

57. The use according to any one of claims 1-56, wherein the proton pump regulator and/or the DIR regulator include proteins and/or polypeptides.

58. The use according to claim 57, wherein the proton pump regulator and/or the DIR regulator comprise the amino acid sequence set forth in SEQ ID NO: 33 and/or variants thereof, where X represents any amino acid.

59. The use according to any one of claims 1-58, wherein the proton pump regulator and/or the DIR regulator comprise the amino acid sequence set forth in any one of SEQ ID NOs: 30-32 and/or variants thereof, where X represents any amino acid.

60. The use according to any one of claims 1-59, wherein the proton pump regulator and/or the DIR regulator comprise the amino acid sequence set forth in any one of SEQ ID NOs: 27-29 and/or variants thereof, where X represents any amino acid.

61. The use according to any one of claims 1-60, wherein the proton pump regulator and/or the DIR regulator comprise the amino acid sequences set forth in SEQ ID NOs: 19-26 and/or variants thereof.

62. The use according to any one of claims 1-61, wherein the proton pump regulator and/or the DIR regulator include a multimer.

63. The use according to claim 62, wherein the multimer includes a homodimer.

64. The use according to any one of claims 1-63, wherein the cysteine in the amino acid sequence of the proton pump regulator and/or the DIR regulator is not subject to modification for sulfhydryl blocking.

65. The use according to any one of claims 1-64, wherein the serine in the amino acid sequence of the proton pump regulator and/or the DIR regulator is not subject to modification for phosphorylation.

66. The use according to any one of claims 1-65, wherein the proton pump regulator and/or the DIR regulator include fusion proteins and/or fusion polypeptides.

67. The use according to claim 66, wherein the fusion protein and/or fusion polypeptide comprise molecules capable of being transported across the blood-brain barrier to the brain.

68. The use according to claim 67, wherein the molecule capable of being transported across the blood-brain barrier to the brain comprises a polypeptide.

69. The use according to any one of claims 67-68, wherein the molecule capable of being transported across the blood-brain barrier to the brain comprises a cell-penetrating peptide.

70. The use according to claim 69, wherein the cell-penetrating peptide comprises the amino acid sequence set forth in SEQ ID NO: 34.

71. The use according to any one of claims 1-70, wherein the proton pump regulator and/or the DIR regulator comprise the amino acid sequence set forth in SEQ ID NO: 48, where X represents any amino acid.

72. The use according to any one of claims 1-71, wherein the proton pump regulator and/or the DIR regulator comprise the amino acid sequence set forth in any one of SEQ ID NOs: 45-47, where X represents any amino acid.

73. The use according to any one of claims 1-72, wherein the proton pump regulator and/or the DIR regulator comprise the amino acid sequence set forth in any one of SEQ ID NOs: 42-44 and/or variants thereof, where X represents any amino acid.

74. The use according to any one of claims 1-73, wherein the proton pump regulator and/or the DIR regulator comprise the amino acid sequence set forth in any one of SEQ ID NOs: 35-41 and/or variants thereof.

75. The use according to any one of claims 1-74, wherein the subject includes mammals.

76. The use according to any one of claims 1-75, wherein the subject includes humans.

77. The use according to any one of claims 1-76, wherein the reagent is formulated to be suitable for oral administration and/or injection administration.

78. Use of a proton pump regulator in the preparation of a reagent for preventing and/or treating a disease, wherein the proton pump regulator regulates ATP6V1B2 and/or a functionally active fragment thereof, and the disease includes a disease associated with the intron-retained splicing product DIR of DNA damage-inducible transcript 4-like transcripts and/or a functional fragment thereof.

79. The use according to claim 78, wherein the ATP6V1B2 and/or functionally active fragment thereof is each from mammals.

80. The use according to any one of claims 78-79, wherein the ATP6V1B2 and/or functionally active fragment thereof is each from humans or mice.

81. The use according to any one of claims 78-80, wherein the ATP6V1B2 comprises the amino acid sequence set forth in SEQ ID NO: 8 or 16.

82. The use according to any one of claims 78-81, wherein the functionally active fragment of the ATP6V1B2 is capable of specifically binding to the amino acid sequence set forth in SEQ ID NO: 5.

83. The use according to any one of claims 78-82, wherein the functionally active fragment of the ATP6V1 B2 is capable of specifically binding to the amino acid sequence set forth in SEQ ID NO: 1.

84. The use according to any one of claims 78-83, wherein the functionally active fragment of the ATP6V1B2 comprises at least a portion of the amino acid sequence from position 288 to position 512 of human ATP6V1B2 protein.

85. The use according to any one of claims 78-84, wherein the functionally active fragment of the ATP6V1B2 comprises at least a portion of the amino acid sequence from position 288 to position 512 of mouse ATP6V1B2 protein.

86. The use according to any one of claims 78-85, wherein the functionally active fragment of the ATP6V1B2 comprises the amino acid sequence set forth in any one of SEQ ID NOs: 10-11.

87. The use according to any one of claims 78-86, wherein the ATP6V1B2 comprises the amino acid sequence encoded by the nucleotide sequence set forth in SEQ ID NO: 9 or 17.

88. The use according to any one of claims 78-87, wherein the proton pump regulator is capable of regulating the activity and/or function of the proton pump.

89. The use according to any one of claims 78-88, wherein the proton pump regulator is capable of improving the expression level and/or activity of proton pump-associated proteins in a subject.

90. The use according to any one of claims 78-89, wherein the proton pump regulator is capable of regulating the expression level and/or activity of the ATP6V1B2.

91. The use according to any one of claims 78-90, wherein the proton pump regulator is capable of improving the expression level and/or activity of the ATP6V1B2 in a subject, and wherein the improvement comprises, compared to the original expression level and/or activity of the ATP6V1B2 in the subject, improving the expression level and/or activity of the ATP6V1B2 by at least about 10%.

92. The use according to any one of claims 78-91, wherein the expression level of the ATP6V1B2 comprises the expression level of the ATP6V1B2 gene, the transcription level of ATP6V1B2 gene, and/or the expression level of the ATP6V1B2 protein.

93. The use according to any one of claims 78-92, wherein the proton pump regulator is capable of increasing the release of neurotransmitters at neuronal synapses, and the increase includes an increase of at least about 10% compared to the original level of neurotransmitter release at neuronal synapses in the subject.

94. The use according to any one of claims 78-93, wherein the proton pump regulator increases the release frequency of excitatory postsynaptic currents, and the increase includes an increase of at least about 10% compared to the original level of release frequency of excitatory postsynaptic currents in the subject.

95. The use according to any one of claims 78-94, wherein the proton pump regulator causes a reduction of the expression level and/or biological activity of the DIR and/or functional fragment thereof in the subject.

96. The use according to claim 95, wherein the reduction comprises a decrease of at least about 10% in the expression level and/or biological activity of the DIR and/or functional fragment thereof compared to the original expression level and/or biological activity of the DIR and/or functional fragment thereof in the subject.

97. The use according to any one of claims 95-96, wherein the expression level includes the expression level of a gene encoding the DIR/or functional fragment thereof, the transcription level of the gene encoding the DIR/or functional fragment thereof, and/or the expression level of the DIR/or functional fragment thereof.

98. The use according to any one of claims 95-97, wherein the functional fragment of the DIR retains at least a portion of the biological activity of the DIR.

99. The use according to claim 98, wherein the biological activity includes influencing the excitability of neurons and/or inhibiting the activity of neurons.

100. The use according to any one of claims 98-99, wherein the biological activity includes the ability to reduce the frequency of excitatory postsynaptic currents (EPSCs), and/or the ability to reduce the amplitude of EPSCs.

101. The use according to claim 100, wherein the reduction comprises, by administering the DIR and/or functional fragment thereof and/or a nucleic acid encoding the DIR and/or functional fragment thereof, reducing the frequency of excitatory postsynaptic currents (EPSCs) in the subject, and/or reducing the amplitude of EPSCs in the subject, compared to the original biological activity of the DIR and/or functional fragment thereof in the subject.

102. The use according to any one of claims 95-101, wherein the biological activity includes influencing cognitive abilities.

103. The use according to any one of claims 95-102, wherein the biological activity includes participation in signaling pathways associated with Aβ deposition, and/or participation in signaling pathways associated with Tau tangle formation.

104. The use according to any one of claims 95-103, wherein the biological activity includes inducing Aβ deposition and/or amyloid plaque formation through gelsolin.

105. The use according to claim 104, wherein the DIR/or functional fragment thereof induces Aβ deposition and/or amyloid plaque formation by binding to gelsolin.

106. The use according to any one of claims 95-105, wherein the expression levels of the DIR and/or functional fragment thereof are positively correlated with the expression levels of Aβ.

107. The use according to any one of claims 95-106, wherein the reduction comprises, by administering the DIR and/or functional fragment thereof and/or a nucleic acid encoding the DIR and/or functional fragment thereof, reducing the cognitive ability of the subject, compared to the original biological activity of the DIR and/or functional fragment thereof in the subject.

108. The use according to any one of claims 78-107, wherein the DIR/or functional fragment thereof is derived from mammals.

109. The use according to any one of claims 78-108, wherein the DIR/or functional fragment thereof is derived from primates.

110. The use according to any one of claims 78-109, wherein the DIR/or functional fragment thereof is derived from humans.

111. The use according to any one of claims 78-110, wherein the DIR comprises the amino acid sequence set forth in SEQ ID NO: 56.

112. The use according to any one of claims 78-111, wherein the functional fragment of the DIR comprises an amino acid sequence encoded by the retained intron in *DDIT4L.*

113. The use according to any one of claims 78-112 wherein the functional fragment of the DIR comprises the amino acid sequence set forth in any one of SEQ ID NOs: 59-60.

114. The use according to any one of claims 78-113, wherein the proton pump regulator includes proteins and/or polypeptides.

115. The use according to claim 114, wherein the proton pump regulator comprises the amino acid sequence set forth in SEQ ID NO: 33 and/or variants thereof, where X represents any amino acid.

116. The use according to any one of claims 78-115, wherein the proton pump regulator comprises the amino acid sequence set forth in any one of SEQ ID NOs: 30-32 and/or variants thereof, where X represents any amino acid.

117. The use according to any one of claims 78-116, wherein the proton pump regulator comprises the amino acid sequence set forth in any one of SEQ ID NOs: 27-29 and/or variants thereof, where X represents any amino acid.

118. The use according to any one of claims 78-117, wherein the proton pump regulator comprises the amino acid sequences set forth in SEQ ID NOs: 19-26 and/or variants thereof.

119. The use according to any one of claims 78-118, wherein the proton pump regulator includes multimers.

120. The use according to claim 119, wherein the multimer includes a homodimer.

121. The use according to any one of claims 78-120, wherein the cysteine in the amino acid sequence of the proton pump regulator is not subject to modification for sulfhydryl blocking.

122. The use according to any one of claims 78-121, wherein the serine in the amino acid sequence of the proton pump regulator is not subject to modification for phosphorylation.

123. The use according to any one of claims 78-122, wherein the proton pump regulator includes fusion proteins and/or fusion polypeptides.

124. The use according to claim 123, wherein the fusion protein and/or fusion polypeptide comprise molecules capable of being transported across the blood-brain barrier to the brain.

125. The use according to claim 124, wherein the molecule capable of being transported across the blood-brain barrier to the brain comprises a polypeptide.

126. The use according to any one of claims 124-125, wherein the molecule capable of being transported across the blood-brain barrier to the brain comprises a cell-penetrating peptide.

127. The use according to claim 126, wherein the cell-penetrating peptide comprises the amino acid sequence set forth in SEQ ID NO: 34.

128. The use according to any one of claims 78-127, wherein the proton pump regulator comprises the amino acid sequence set forth in SEQ ID NO: 48, where X represents any amino acid.

129. The use according to any one of claims 78-128, wherein the proton pump regulator comprises the amino acid sequence set forth in any one of SEQ ID NOs: 45-47, where X represents any amino acid.

130. The use according to any one of claims 78-129, wherein the proton pump regulator comprises the amino acid sequence set forth in any one of SEQ ID NOs: 42-44 and/or variants thereof, where X represents any amino acid.

131. The use according to any one of claims 78-130, wherein the proton pump regulator comprises the amino acid sequence set forth in any one of SEQ ID NOs: 35-41 and/or variants thereof.

132. The use according to any one of claims 78-131, wherein the subject includes mammals.

133. The use according to any one of claims 78-132, wherein the subject includes humans.

134. The use according to any one of claims 78-133, wherein the reagent is formulated to be suitable for oral administration and/or injection administration.

135. The use according to any one of claims 78-134, wherein the disease associated with the intron-retained splicing product DIR of DNA damage-inducible transcript 4-like transcripts and/or functional fragments thereof includes cognitive impairment, neurodegenerative diseases and/or stroke.

136. The use according to claim 135, wherein the cognitive impairment includes cognitive impairment caused by normal aging, Lewy body dementia (LBD), frontotemporal dementia and/or vascular dementia.

137. The use according to any one of claims 135-136, wherein the inducing disease of the cognitive impairment includes Alzheimer's disease, multi-infarct type, Parkinson's disease, AIDS and/or Creutzfeldt-Jakob disease (CJD).

138. The use according to any one of claims 135-137, wherein the cognitive impairment includes mild cognitive impairment (MCI), moderate-stage cognitive impairment and advanced-stage cognitive impairment.

139. The use according to any one of claims 135-138, wherein the cognitive impairment includes multi-domain amnestic MCI (aMCI-m).

140. The use according to any one of claims 135-139, wherein the neurodegenerative disease includes acute neurodegenerative disease and chronic neurodegenerative disease.

141. The use according to any one of claims 135-140, wherein the neurodegenerative disease includes neurodegenerative disease caused by neuronal death and glial cell homeostasis, neurodegenerative disease caused by aging, neurodegenerative disease caused by influence on the CNS cell function, neurodegenerative disease caused by abnormal communication between cells, and/or neurodegenerative disease caused by impaired cell motility.

142. The use according to any one of claims 135-140, wherein the neurodegenerative disease includes Alzheimer's disease, Parkinson's disease, multiple sclerosis (MS), amyotrophic lateral sclerosis (ALS), and/or Huntington's disease (HD).

143. The use according to any one of claims 135-142, wherein the neurodegenerative disease includes presenile dementia.

144. The use according to any one of claims 135-143, wherein the neurodegenerative disease includes the early stage, middle stage and/or late stage of presenile dementia.

145. The use according to any one of claims 135-144, wherein the stroke includes ischemic stroke and/or hemorrhagic stroke.

146. The use according to claim 145, wherein the ischemic stroke includes cerebral infarction.

147. The use according to claim 146, wherein the cerebral infarction includes lacunar infarction, ischemic cerebral infarction and/or hemorrhagic infarction.

148. The use according to any one of claims 145-147, wherein the ischemic stroke is caused by factors including: thrombus, embolism and/or hypotension.

149. The use according to claim 148, wherein the thrombus is caused by factors including: atherosclerosis, aneurysm, vascular malformation, arteritis and/or vasospasm.

150. The use according to any one of claims 145-149, wherein the ischemic stroke includes large artery atherosclerosis stroke, cardiogenic embolic stroke, small artery occlusion stroke, stroke of other determined etiology and/or stroke of undetermined etiology.

151. The use according to any one of claims 145-151, wherein the hemorrhagic stroke includes brain parenchymal hemorrhage, intraventricular hemorrhage and/or subarachnoid hemorrhage.

152. The use according to any one of claims 145-151, wherein the hemorrhagic stroke includes primary cerebral hemorrhage and/or secondary cerebral hemorrhage.

153. The use according to any one of claims 145-152, wherein the hemorrhagic stroke includes aneurysmal subarachnoid hemorrhage.

154. The use according to any one of claims 145-153, wherein the hemorrhagic stroke is caused by factors including: vascular malformation, aneurysm, blood disease, cerebral amyloid angiopathy, moyamoya disease, cerebral arteritis, anticoagulation or thrombolytic therapy and/or tumor stroke.

155. The use according to any one of claims 135-154, wherein the stroke includes stroke-induced impairments.

156. The use according to claim 155, wherein the impairment includes impairments observed through imaging.

157. The use according to claim 156, wherein the impairment observed through imaging includes intracerebral hematoma, intraventricular hemorrhage and/or subarachnoid hemorrhage.

158. The use according to any one of claims 156-157, wherein the impairment observed through imaging includes edema, hematoma and/or mass effect.

159. The use according to any one of claims 156-158, wherein the impairment observed through imaging includes dense artery sign, insular ribbon sign, blurred or decreased density of the basal ganglia, occlusion of small penetrating cerebral arteries, edema and/or mass effect.

160. The use according to claim 156, wherein the impairment includes impairment of learning ability, and the learning ability comprises cognitive ability, motor ability, memory ability, and/or spatial exploration ability.

161. The use according to claim 160, wherein the learning ability is measured by implementing a test selected from the group consisting of: novel object recognition test and water maze test.

162. The use according to claim 161, wherein the novel object recognition test is used for evaluating cognitive ability, motor ability and/or spatial exploration ability.

163. The use according to any one of claims 161-162, wherein the water maze test is used for evaluating memory ability, motor ability and/or spatial exploration ability.
